# EUROPÄISCHE PATENTSCHRIFT

(11) **EP 3 911 716 B1**
(45) Veröffentlichungstag und Bekanntmachung des Hinweises auf die Patenterteilung: **21.09.2022**
(21) Anmeldenummer: 20700398.9
(22) Anmeldetag: 15.01.2020
(51) Int. Cl.: C09K 11/06, H01L 51/50, C07C 13/567, C07D 307/91, C07D 317/70, C07D 493/04

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 17.01.2019 EP 19152285
(43) Veröffentlichungstag der Anmeldung: 24.11.2021
(73) Patentinhaber: Merck Patent GmbH, 64293 Darmstadt (DE)
(72) Erfinder: KOENEN, Nils, 64347 GRIESHEIM (DE); HAYER, Anna, 64293 DARMSTADT (DE); MAIER-FLAIG, Florian, 69469 WEINHEIM (DE); PFISTER, Jochen, 64342 SEEHEIM-JUGENHEIM (DE); HEIL, Holger, 60316 FRANKFURT AM MAIN (DE)
(86) Internationale Anmeldenummer: PCT/EP2020/050845
(87) Internationale Veröffentlichungsnummer: WO 2020/148303

(56) Entgegenhaltungen:
- CN-A- 105 254 561
- CN-A- 107 353 281
- CN-A- 108 203 417
- US-A1- 2018 354 931

## Beschreibung

Die vorliegende Erfindung betrifft Materialien für die Verwendung in elektronischen Vorrichtungen, insbesondere in organischen Elektrolumineszenzvorrichtungen.

In organischen Elektrolumineszenzvorrichtungen (OLEDs) werden als emittierende Materialien häufig phosphoreszierende metallorganische Komplexe eingesetzt. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu Verbesserungen der OLED-Eigenschaften führen. So gibt es insbesondere bei der Lebensdauer und der Effizienz löslich oder zumindest teilweise löslich prozessierter organischer Elektrolumineszenzvorrichtungen noch Verbesserungsbedarf. Weiterhin ist es erforderlich, dass die Verbindungen für die Verarbeitung aus Lösung eine ausreichend hohe Löslichkeit in gängigen organischen Lösemitteln aufweisen. Um die Vorrichtung nach Aufbringen einer Schicht aus Lösung ausheizen zu können und auch für die Anwendung bei erhöhter Temperatur ist eine hohe Glasübergangstemperatur essentiell. Es besteht daher weiterhin Bedarf an verbesserten Materialien.

Als Matrixmaterialien für phosphoreszierende Emitter werden üblicherweise Verbindungen mit loch- und/oder elektronentransportierenden Eigenschaften eingesetzt. Es kann vorteilhaft sein, zusätzlich zu diesen Materialien weitere Matrixmaterialien einzusetzen, welche weder lochnoch elektronentransportierende Eigenschaften aufweisen und somit nicht bzw. nicht in wesentlichem Umfang am Ladungstransport innerhalb der OLED teilnehmen (WO 2010/108579). Aus WO 2009/124627 und WO 2016/184540 sind 9,9-Diphenylfluorenderivate bekannt, welche an den Phenylgruppen mit weiteren Arylgruppen substituiert sind. Fluorenderivate, welche in der 4-Position des Fluorens substituiert sind, sind nicht offenbart.

Überraschend wurde gefunden, dass 9,9-Diphenylfluorenderivate, welche an mindestens einer und vorzugsweise an beiden Phenylgruppen jeweils in 3'-Position substituiert sind und welche gleichzeitig am Fluorengrundkörper in 4-Position einen aromatischen Substituenten aufweisen, sich sehr gut für die Verwendung in organischen Elektrolumineszenzvorrichtungen eignen und dort zu deutlichen Verbesserungen gegenüber dem Stand der Technik führen. Diese Verbindungen und deren Verwendung in elektronischen Vorrichtungen sind daher der Gegenstand der vorliegenden Erfindung. Diese Verbindungen eignen sich insbesondere als Wide Bandgap Materialien für die Verwendung in der emittierenden Schicht einer phosphoreszierenden OLED.

Die erfindungsgemäßen Materialien weisen eine höhere Glasübergangstemperatur auf als entsprechende Materialien, welche ansonsten die gleiche Struktur aufweisen, jedoch keine aromatische Gruppe in 4-Position des Fluorengrundkörpers gebunden haben. Dadurch kann die Schicht nach Herstellung aus Lösung bei einer höheren Temperatur ausgeheizt werden. Weiterhin weisen die Verbindungen eine verbesserte Löslichkeit auf. Im Vergleich zu OLEDs enthaltend Verbindungen, welche eine entsprechende aromatische Gruppe in der 2-Position statt in der 4-Position des Fluorens gebunden enthält, weisen OLEDs mit den erfindungsgemäßen Verbindungen eine verbesserte Lebensdauer auf.

Der Übersichtlichkeit halber wird im Folgenden die Nummerierung des 9,9-Diphenylfluorens dargestellt: 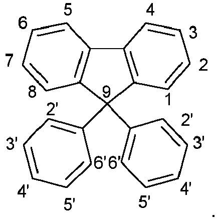 Gegenstand der Erfindung sind Verbindungen der folgenden Formel (1), 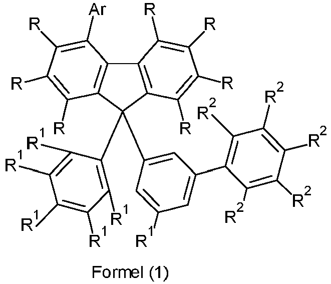 wobei für die verwendeten Symbole gilt:
- Ar: ist ein aromatisches Ringsystem mit 6 bis 30 aromatischen Ringatomen, welches mit einem oder mehreren Resten R³ substituiert sein kann, oder eine Dibenzofuran- oder Dibenzothiophengruppe, welche jeweils mit einem oder mehreren Resten R³ substituiert sein kann, oder eine Kombination aus einem aromatischen Ringsystem mit 6 bis 18 aromatischen Ringatomen und einer Dibenzofuran- oder Dibenzothiophengruppe, wobei diese Gruppen jeweils durch einen oder mehrere Reste R³ substituiert sein können; dabei kann Ar mit dem benachbarten Substituenten R ein Ringsystem bilden;
- R: ist bei jedem Auftreten gleich oder verschieden H, D, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenylgruppe mit 2 bis 20 C-Atomen, wobei die Alkyl-, Alkoxy- bzw. Alkenylgruppe jeweils mit einem oder mehreren Resten R³ substituiert sein kann, wobei ein oder mehrere H-Atome durch D ersetzt sein können, oder ein aromatisches Ringsystem mit 6 bis 30 aromatischen Ringatomen, das durch einen oder mehrere Reste R³ substituiert sein kann, oder eine Dibenzofuran- oder Dibenzothiophengruppe, die jeweils durch einen oder mehrere Reste R³ substituiert sein kann; dabei können zwei oder mehrere benachbarte Substituenten R auch miteinander ein mono- oder polycyclisches, aliphatisches Ringsystem bilden; weiterhin kann R mit einer benachbarten Gruppe Ar ein Ringsystem bilden;
- R¹, R²: ist bei jedem Auftreten gleich oder verschieden H, D, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenylgruppe mit 2 bis 20 C-Atomen, wobei die Alkyl-, Alkoxy- bzw. Alkenylgruppe jeweils mit einem oder mehreren Resten R³ substituiert sein kann, wobei ein oder mehrere H-Atome durch D ersetzt sein können, oder ein aromatisches Ringsystem mit 6 bis 30 aromatischen Ringatomen, das durch einen oder mehrere Reste R³ substituiert sein kann, oder eine Dibenzofuran- oder Dibenzothiophengruppe, die jeweils durch einen oder mehrere Reste R³ substiuiert sein kann; dabei können mehrere benachbarte Substituenten R¹ miteinander ein Ringsystem bilden; weiterhin können mehrere benachbarte Substituenten R² miteinander ein Ringsystem bilden;
- R³: ist bei jedem Auftreten gleich oder verschieden H, D, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die Alkyl- bzw. Alkoxygruppe jeweils mit einem oder mehreren Resten R⁴ substituiert sein kann, wobei ein oder mehrere H-Atome durch D ersetzt sein können, oder ein aromatisches Ringsystem mit 6 bis 30 aromatischen Ringatomen, das durch einen oder mehrere Reste R⁴ substituiert sein kann, oder eine Dibenzofuranoder Dibenzothiophengruppe, die jeweils durch einen oder mehrere Reste R⁴ substituiert sein kann; dabei können zwei oder mehrere benachbarte Substituenten R³ auch miteinander ein Ringsystem bilden;
- R⁴: ist bei jedem Auftreten gleich oder verschieden H, D oder ein aliphatischer und/oder aromatischer Kohlenwasserstoffrest mit 1 bis 20 C-Atomen.

Eine Arylgruppe im Sinne dieser Erfindung enthält 6 bis 30 C-Atome. Dabei wird unter einer Arylgruppe entweder ein einfacher aromatischer Cyclus, also Benzol, oder eine kondensierte Arylgruppe, beispielsweise Naphthalin, Anthracen, Pyren, etc., verstanden. Ein aromatisches Ringsystem im Sinne dieser Erfindung enthält 6 bis 30 C-Atome im Ringsystem. Unter einem aromatischen Ringsystem im Sinne dieser Erfindung soll ein System verstanden werden, das nicht notwendigerweise nur Arylgruppen enthält, sondern in dem auch mehrere Arylgruppen durch eine kurze, nicht-aromatische Einheit (bevorzugt weniger als 10 % der von H verschiedenen Atome), wie z. B. ein sp³-hybridisiertes C-Atom, unterbrochen sein können. So sollen beispielsweise auch Systeme wie 9,9'-Spirobifluoren oder 9,9-Diarylfluoren, etc. als aromatische Ringsysteme im Sinne dieser Erfindung verstanden werden. Ebenso werden unter einem aromatischen Ringsystem Systeme verstanden, in denen mehrere Arylgruppen durch Einfachbindungen miteinander verknüpft sind, beispielsweise Biphenyl oder Terphenyl.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl und 2-Ethylhexyl verstanden. Unter einer Alkenylgruppe im Sinne dieser Erfindung werden insbesondere Ethenyl, Propenyl, Butenyl, Pentenyl, Cyclopentenyl, Hexenyl, Cyclohexenyl, Heptenyl, Cycloheptenyl, Octenyl und Cyclooctenyl verstanden. Unter einer C₁- bis C₂₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen Ringsystem mit 5 bis 30 aromatischen Ringatomen, welches über beliebige Positionen am Aromaten verknüpft sein kann, werden insbesondere Gruppen verstanden, die abgeleitet sind von Benzol, Naphthalin, Anthracen, Phenanthren, Benzanthracen, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen oder Spiroisotruxen.

Wenn mehrere benachbarte Substituenten miteinander ein Ringsystem bilden, kann dieses mono- oder polycyclisch sein, und es kann aliphatisch oder aromatisch sein. Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

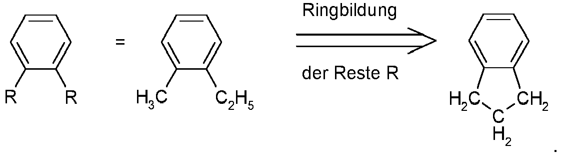

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

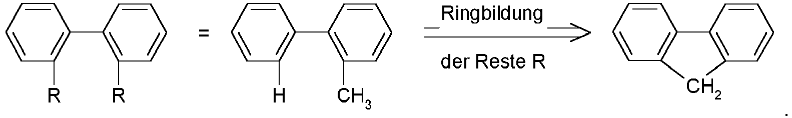

In einer bevorzugten Ausführungsform der Erfindung ist die Verbindung der Formel (1) ausgewählt aus den Verbindungen der folgenden Formeln (2a), (2b) und (2c), 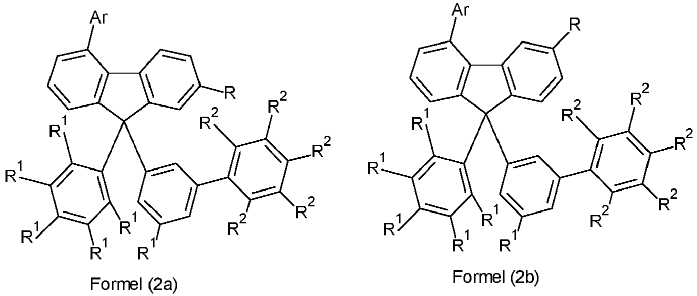 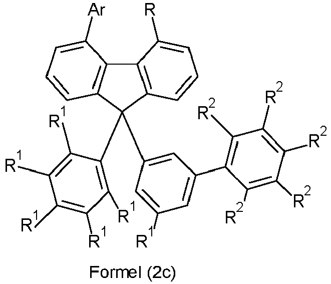 wobei die verwendeten Symbole die oben genannten Bedeutungen aufweisen.

In einer weiteren bevorzugten Ausführungsform der Erfindung ist die Verbindung der Formel (1) ausgewählt aus den Verbindungen der folgenden Formeln (3a) und (3b), 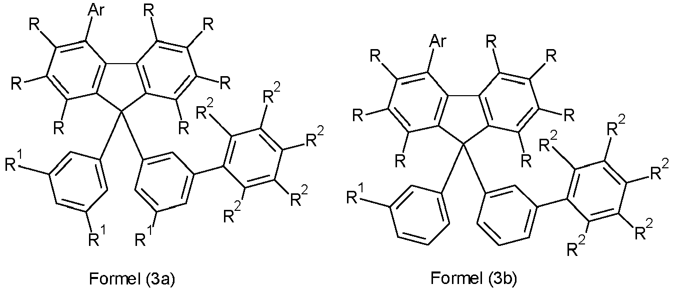 wobei die verwendeten Symbole die oben genannten Bedeutungen aufweisen.

Besonders bevorzugt ist die Verbindung der Formel (1) ausgewählt aus den Verbindungen der folgenden Formeln (4a) bis (4f), 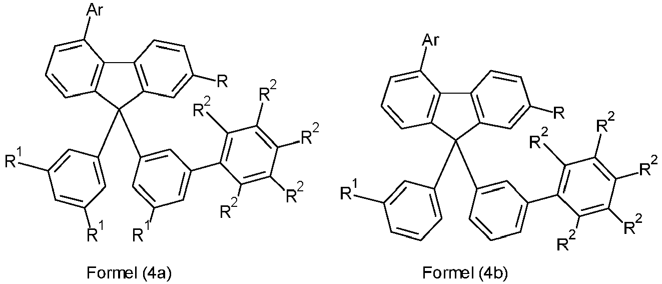 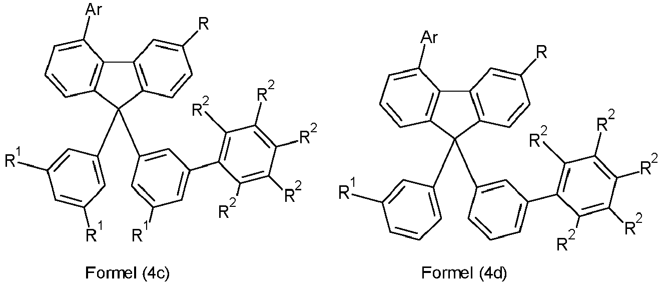 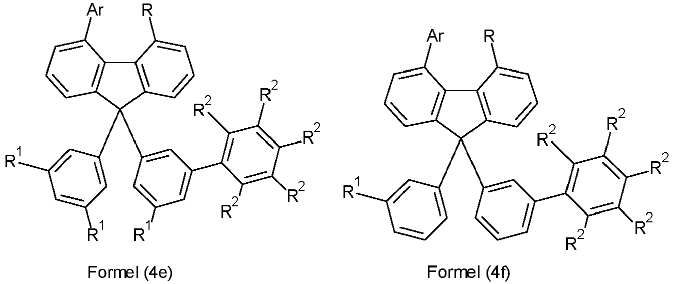 wobei die verwendeten Symbole die oben genannten Bedeutungen aufweisen.

In einer weiteren bevorzugten Ausführungsform der Erfindung ist die Verbindung der Formel (1) ausgewählt aus den Verbindungen der folgenden Formel (5), 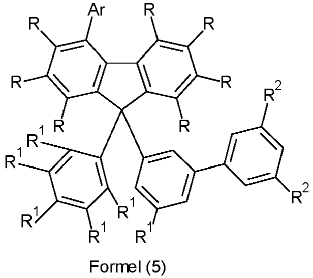 wobei die verwendeten Symbole die oben genannten Bedeutungen aufweisen.

Besonders bevorzugt sind die Verbindungen der folgenden Formeln (6a) bis (6f), 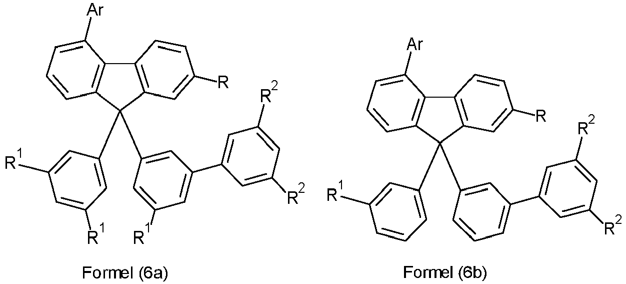 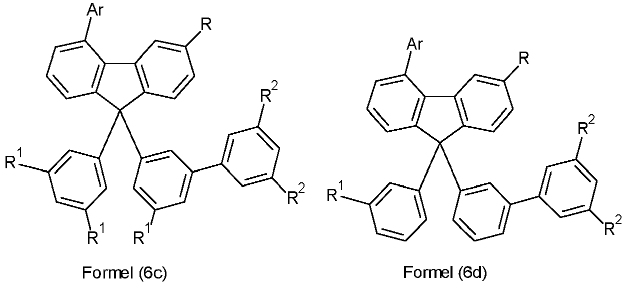 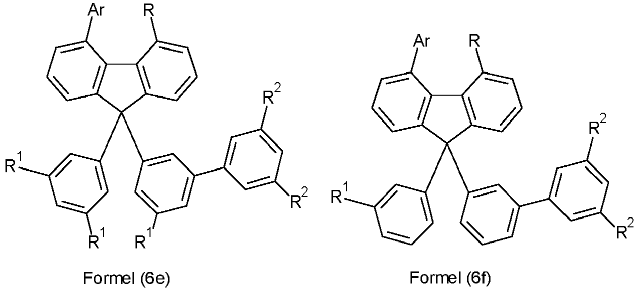 wobei die verwendeten Symbole die oben genannten Bedeutungen aufweisen.

Im Folgenden werden bevorzugte Ausführungsformen der Gruppen Ar, R, R¹, R², R³ und R⁴ beschrieben. In einer besonders bevorzugten Ausführungsform der Erfindung treten die nachfolgend genannten Bevorzugungen für Ar, R, R¹, R², R³ und R⁴ gleichzeitig auf und gelten für die Strukturen der Formel (1), sowie für alle oben aufgeführten bevorzugten Ausführungsformen.

In einer bevorzugten Ausführungsform der Erfindung ist Ar ein aromatisches Ringsystem mit 6 bis 24 aromatischen Ringatomen, welches mit einem oder mehreren, bevorzugt nicht-aromatischen Resten R³ substituiert sein kann, oder eine Dibenzofuran- oder Dibenzothiophengruppe, welche jeweils mit einem oder mehreren, bevorzugt nicht-aromatischen Resten R³ substiuiert sein kann. In einer besonders bevorzugten Ausführungsform der Erfindung ist Ar ein aromatisches Ringsystem mit 6 bis 18 aromatischen Ringatomen, insbesondere mit 6 bis 12 aromatischen Ringatomen, welches jeweils mit einem oder mehreren, bevorzugt nicht-aromatischen Resten R³ substituiert sein kann.

Geeignete Gruppen Ar sind ausgewählt aus der Gruppe bestehend aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Dibenzofuran, welches über die 1-, 2-, 3oder 4-Position verknüpft sein kann, Dibenzothiophen, welches über die 1-, 2-, 3- oder 4-Position verknüpft sein kann, Phenanthren, Triphenylen oder einer Kombination dieser Gruppen, welche jeweils mit einem oder mehreren, bevorzugt nicht-aromatischen Resten R³ substituiert sein können.

Dabei ist Ar bevorzugt gewählt aus den Gruppen der folgenden Formeln Ar-1 bis Ar-26, 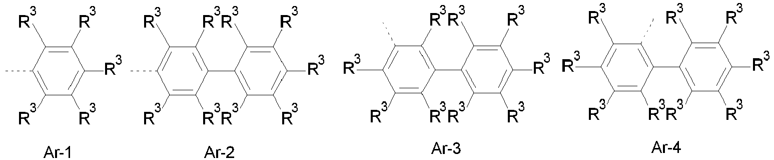 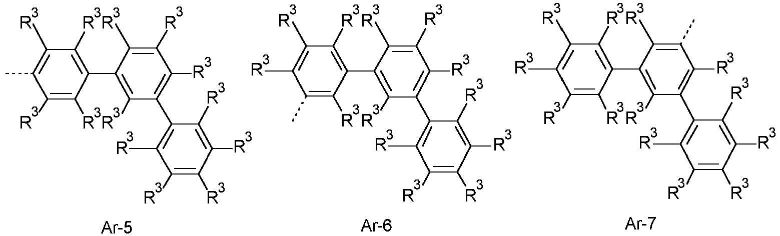 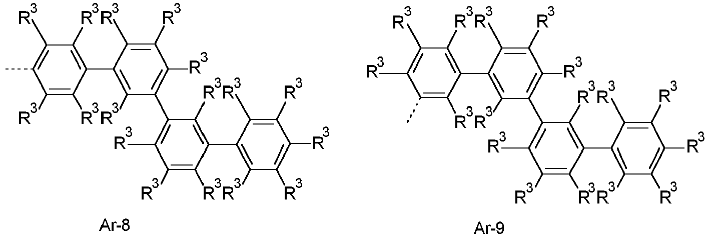 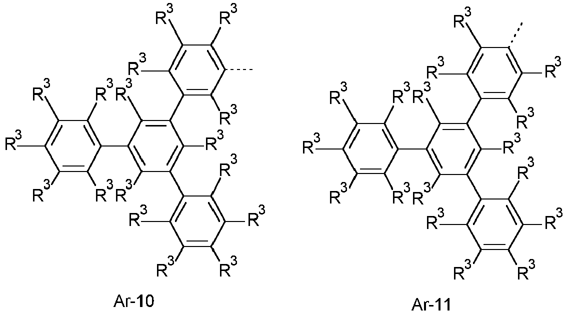 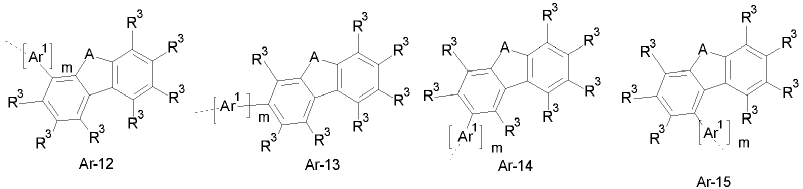 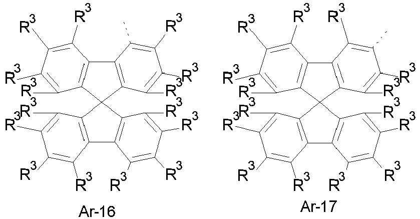 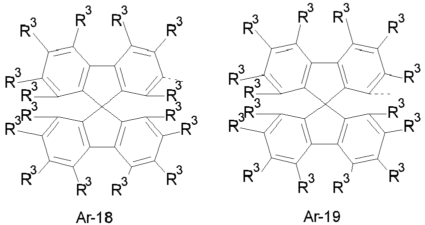 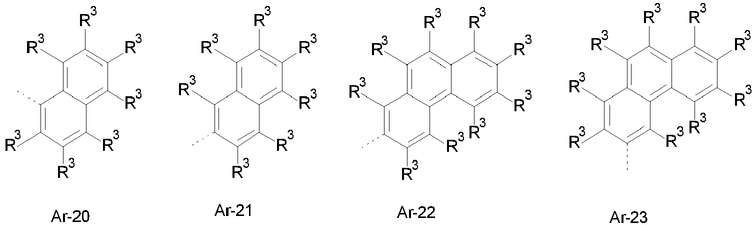 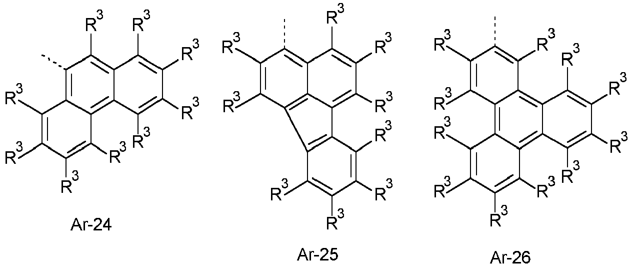 wobei R³ die oben genannten Bedeutungen aufweist, die gestrichelte Bindung die Bindung an den Fluorengrundkörper darstellt und weiterhin gilt:
- Ar¹: ist bei jedem Auftreten gleich oder verschieden ein bivalentes aromatisches Ringsystem mit 6 bis 12 aromatischen Ringatomen, welches jeweils mit einem oder mehreren Resten R³ substituiert sein kann;
- A: ist bei jedem Auftreten gleich oder verschieden C(R³)₂, O oder S;
- m: ist 0 oder 1, wobei m = 0 bedeutet, dass die Gruppe Ar¹ nicht vorhanden ist und dass die entsprechende aromatische bzw. heteroaromatische Gruppe direkt an den Fluorengrundkörper gebunden ist.

In einer weiteren Ausführungsform der Erfindung steht Ar für eine Phenylgruppe, die Gruppe R, die der Gruppe Ar benachbart ist, steht für eine Gruppe CH(R³)₂, und Ar und R bilden miteinander ein Ringsystem, so dass eine Verbindung der folgenden Formel (7a) oder (7b) entsteht, 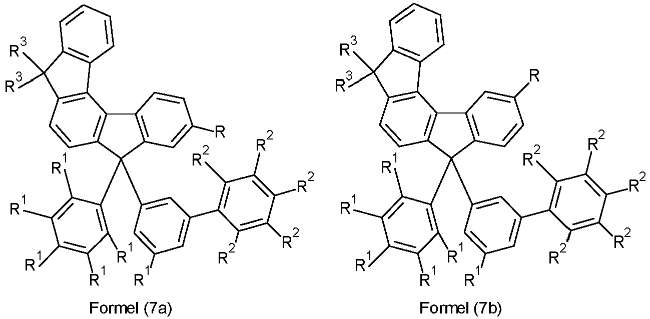 wobei die verwendeten Symbole die oben genannten Bedeutungen aufweisen.

In einer weiteren bevorzugten Ausführungsform der Erfindung ist R gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R³ substituiert sein kann, oder einem aromatischen Ringsystem mit 6 bis 18 aromatischen Ringatomen, bevorzugt mit 6 bis 12 aromatischen Ringatomen, das durch einen oder mehrere Substituenten R³ substituiert sein kann. Besonders bevorzugt ist R gleich oder verschieden bei jedem Auftreten ausgewählt aus der Gruppe bestehend aus H, D, einer geradkettigen Alkylgruppe mit 1 bis 4 C-Atomen oder einer verzweigten Alkylgruppe mit 3 oder 4 C-Atomen, wobei die Alkylgruppe jeweils mit einem oder mehreren Resten R³ substituiert sein kann, bevorzugt jedoch unsubstituiert ist. Ganz besonders bevorzugt ist R gleich oder verschieden bei jedem Auftreten H oder D, insbesondere H.

Besonders bevorzugt sind somit die Verbindungen der folgenden Formeln (2d), (6g) bzw. (6h), 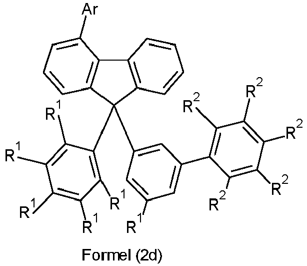 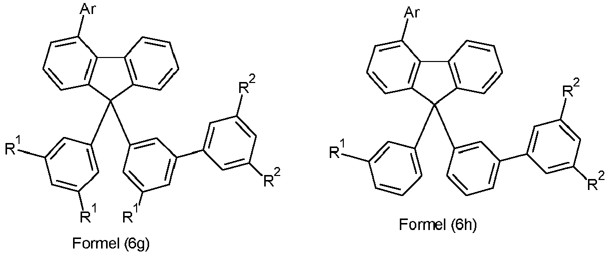 wobei die verwendeten Symbole die oben genannten Bedeutungen aufweisen.

In einer weiteren bevorzugten Ausführungsform der Erfindung ist R¹ gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R³ substituiert sein kann, bevorzugt jedoch unsubstituiert ist, oder einem aromatischen Ringsystem mit 6 bis 24 aromatischen Ringatomen, das durch einen oder mehrere Reste R³ substituiert sein kann, oder Dibenzofuran oder Dibenzothiophen, das jeweils durch einen oder mehrere Reste R³ substiuiert sein kann. Besonders bevorzugt ist R¹ gleich oder verschieden bei jedem Auftreten ausgewählt aus der Gruppe bestehend aus H, D, einer geradkettigen Alkylgruppe mit 1 bis 4 C-Atomen oder einer verzweigten Alkylgruppe mit 3 oder 4 C-Atomen, oder einem aromatischen Ringsystem mit 6 bis 18 aromatischen Ringatomen, das durch einen oder mehrere, bevorzugt nicht-aromatische Reste R³ substituiert sein kann, bevorzugt jedoch unsubstituiert ist.

In einer weiteren bevorzugten Ausführungsform der Erfindung ist R² gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R³ substituiert sein kann, bevorzugt jedoch unsubstituiert ist, oder einem aromatischen Ringsystem mit 6 bis 24 aromatischen Ringatomen, das durch einen oder mehrere Reste R³ substituiert sein kann, oder Dibenzofuran oder Dibenzothiophen, das jeweils durch einen oder mehrere Reste R³ substiuiert sein kann. Besonders bevorzugt ist R² gleich oder verschieden bei jedem Auftreten ausgewählt aus der Gruppe bestehend aus H, D, einer geradkettigen Alkylgruppe mit 1 bis 4 C-Atomen oder einer verzweigten Alkylgruppe mit 3 oder 4 C-Atomen, oder einem aromatischen Ringsystem mit 6 bis 18 aromatischen Ringatomen, das durch einen oder mehrere, bevorzugt nicht-aromatische Reste R³ substituiert sein kann, bevorzugt jedoch unsubstituiert ist.

Wenn R¹ bzw. R² für ein aromatisches Ringsystem bzw. Dibenzofuran oder Dibenzothiophen bzw. eine Kombination dieser Gruppen steht, sind bevorzugte Gruppen R¹ bzw. R² ausgewählt aus der Gruppe bestehend aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3oder 4-Position verknüpft sein kann, Naphthalin, welches über die 1- oder 2-Position verknüpft sein kann, Dibenzofuran, welches über die 1-, 2-, 3oder 4-Position verknüpft sein kann, Dibenzothiophen, welches über die 1-, 2-, 3- oder 4-Position verknüpft sein kann, Phenanthren, Triphenylen oder einer Kombination dieser Gruppen, welche jeweils mit einem oder mehreren Resten R³, bevorzugt nicht-aromatischen Resten R³, substituiert sein können.

Aromatische bzw. heteroaromatische Gruppen R¹ bzw. R² sind bevorzugt bei jedem Auftreten gleich oder verschieden gewählt aus den Gruppen der folgenden Formeln R-1 bis R-26, 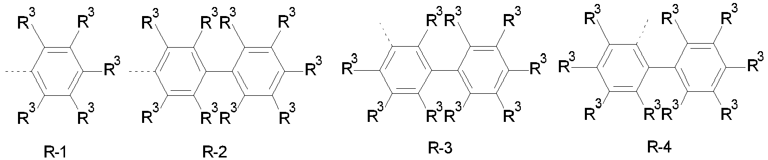 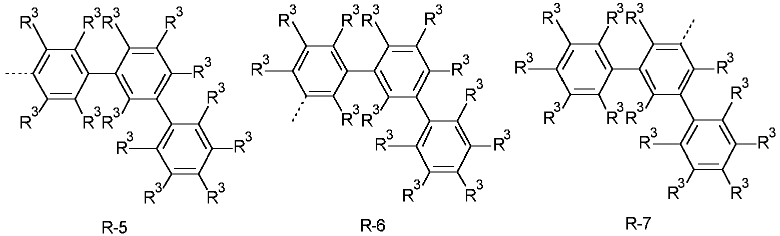 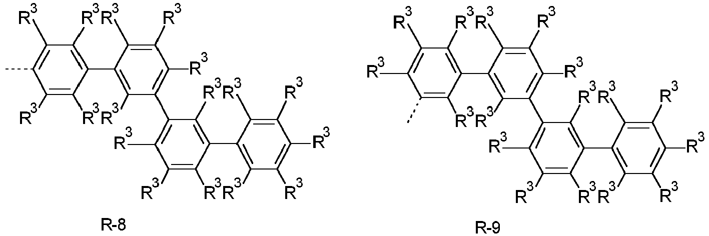 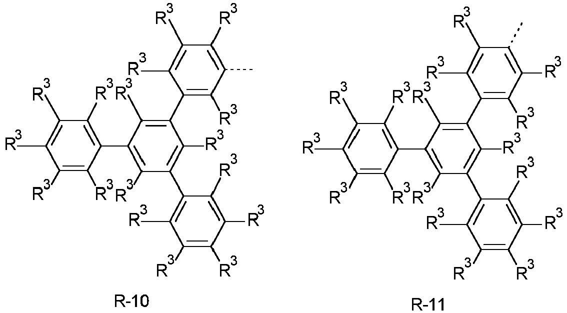 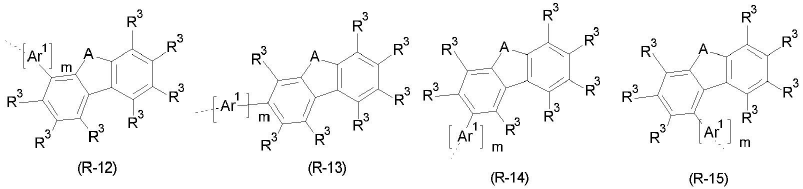 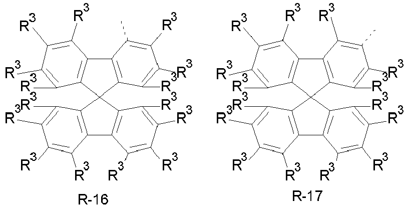 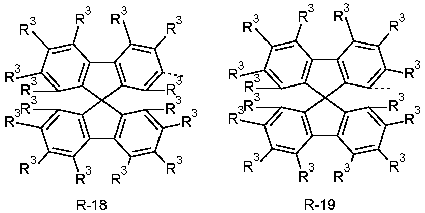 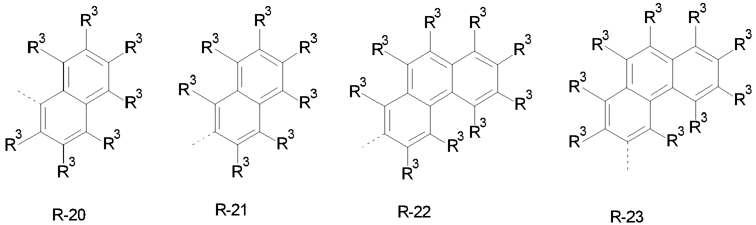 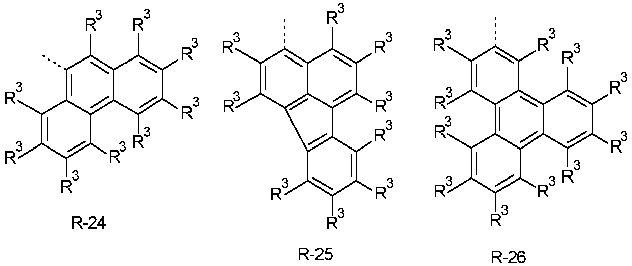 wobei R³, Ar¹, A und m die oben genannten Bedeutungen aufweist, die gestrichelte Bindung die Bindung an die Phenylgruppe darstellt und m = 0 bedeutet, dass die Gruppe Ar¹ nicht vorhanden ist und dass die entsprechende aromatische bzw. heteroaromatische Gruppe direkt an die Phenylgruppe gebunden ist.

In einer weiteren bevorzugten Ausführungsform der Erfindung ist R³ gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R⁴ substituiert sein kann, bevorzugt jedoch unsubstituiert ist, oder einem aromatischen Ringsystem mit 6 bis 24 aromatischen Ringatomen, das durch einen oder mehrere Reste R⁴ substituiert sein kann, bevorzugt aber unsubstituiert ist. Besonders bevorzugt ist R³ gleich oder verschieden bei jedem Auftreten ausgewählt aus der Gruppe bestehend aus H, D, einer geradkettigen Alkylgruppe mit 1 bis 4 C-Atomen oder einer verzweigten Alkylgruppe mit 3 oder 4 C-Atomen oder einem aromatischen Ringsystem mit 6 bis 18 aromatischen Ringatomen, bevorzugt mit 6 bis 12 aromatischen Ringatomen, das jeweils durch einen oder mehrere Reste R⁴ substituiert sein kann, bevorzugt aber unsubstituiert ist.

R⁴ ist bevorzugt bei jedem Auftreten gleich oder verschieden H, D, ein aliphatischer Kohlenwasserstoffrest mit 1 bis 4 C-Atomen oder ein aromatischer Kohlenwasserstoffrest mit 6 bis 12 C-Atomen.

Eine besonders bevorzugte Ausführungsform der Erfindung sind die Verbindungen der folgenden Formeln (8a) und (8b), 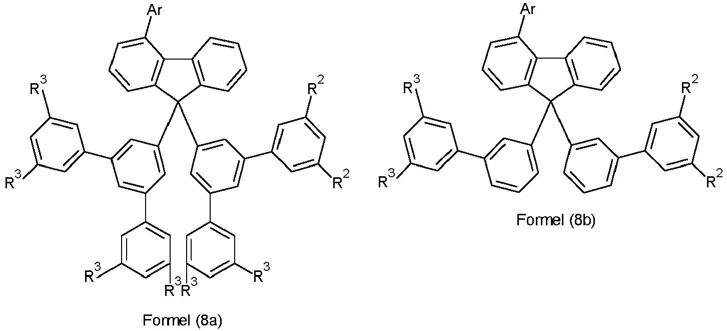 wobei die verwendeten Symbole die oben genannten Bedeutungen aufweisen.

Ganz besonders bevorzugt sind die Verbindungen der folgenden Formeln (9a) und (9b), 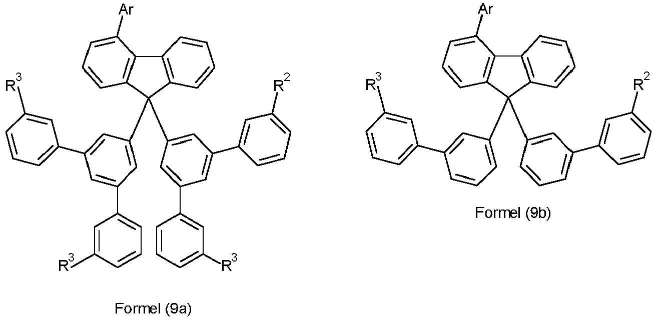 wobei die verwendeten Symbole die oben genannten Bedeutungen aufweisen.

Dabei stehen R² und R³ in den Formeln (8a), (8b), (9a) und (9b) bevorzugt für ein aromatisches Ringsystem mit 6 bis 18 aromatischen Ringatomen, besonders bevorzugt mit 6 bis 12 aromatischen Ringatomen, wobei R² mit einem oder mehreren Substituenten R³ und R³ mit einem oder mehreren Substituenten R⁴ substituiert sein können, bevorzugt aber unsubstituiert sind.

Gemäß einer bevorzugten Ausgestaltung weisen die Verbindungen der Formel (1) bzw. der bevorzugten Ausführungsformen ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf. Weiterhin bevorzugt weisen die Verbindungen der Formel (1) bzw. der bevorzugten Ausführungsformen ein Molekulargewicht von größer oder gleich 750 g/mol.

Weiterhin zeichnen sich bevorzugte erfindungsgemäße Verbindungen dadurch aus, dass diese eine hohe Löslichkeit in gängigen organischen Lösemitteln aufweisen, bevorzugt eine Löslichkeit von ≥ 10 mg/ml in Toluol.

Bevorzugt stellt die Verbindung der Formel (1) bzw. der bevorzugten Ausführungsformen einen Kohlenwasserstoff dar, enthält also keine Heteroatome.

Gemäß einer besonderen Ausführungsform weist eine Verbindung der Formel (1) bzw. der bevorzugten Ausführungsformen eine Glasübergangstemperatur von mindestens 110 °C, besonders bevorzugt von mindestens 120 °C, ganz besonders bevorzugt von mindestens 140 °C und insbesondere bevorzugt von mindestens 160 °C auf, bestimmt nach DIN EN ISO 11357-1 und DIN EN ISO 11357-2.

Besonders bevorzugt stellt die Verbindung der Formel (1) bzw. der bevorzugten Ausführungsformen ein Wide Bandgap Material dar. Dabei ist es bevorzugt, wenn die Verbindung eine Bandlücke (bandgap) von 2.5 eV oder mehr, bevorzugt 3.0 eV oder mehr, ganz bevorzugt von 3.5 eV oder mehr aufweist. Als ein "Wide Bandgap" Material im Sinne der vorliegenden Erfindung wird ein Material mit einer Bandlücke von 3.0 eV oder mehr bezeichnet. Die Bandlücke kann durch die Energieniveaus des höchsten besetzten Molekülorbitals (highest occupied molecular orbital, HOMO) und des niedrigsten unbesetzten Molekülorbitals (lowest unoccupied molecular orbital, LUMO) berechnet werden. Dabei wird die Geometrieoptimierung der Grundzustandsgeometrie der isolierten Gasphasenmoleküle mit der Methode B3PW91/6-31G(d) durchgeführt. Die nachfolgenden TD-DFT-Rechnungen der vertikalen Singulett- und Triplettanregungsenergien (Absorptionsenergien) werden unter Verwendung derselben Methode (d.h. B3PW91/6-31G(d)) durchgeführt. Dafür wird das Programmpaket Gaussian (Ausgabe E.01) verwendet.

Die erfindungsgemäßen Verbindungen können nach dem Fachmann allgemein bekannten Syntheseschritten dargestellt werden, wie in Schema 1 bis 3 dargestellt. Dabei wird in einem ersten Schritt ein aromatischer ortho-Bromphenylbenzoesäurealkylester mit einer ortho-Arylsubstituierten Phenylboronsäure bzw. einem entsprechenden Boronsäureester gekuppelt (Schema 1). Dabei können die Phenylgruppen auch jeweils durch Substituenten R substituiert sein, und statt Brom kann im Edukt auch eine andere Abgangsgruppe verwendet werden. Das entstandene Biphenylderivat kann mit einer meta-Brom-substituierten Phenyllithiumverbindung zu einem 9,9-Diphenylfluoren umgesetzt werden, welches an den Phenylgruppen durch Brom substituiert ist (Schema 2). Statt einer Bromgruppe kann die Phenyllithium auch zwei oder mehr Bromgruppen oder auch andere Abgangsgruppen tragen. Dieses Fluorenderivat wird in einem letzten Schritt in einer Suzuki-Kupplung mit einem Phenylboronsäurederivat, welches auch weiter substituiert sein kann, oder in einer anderen Kupplungreaktion zu dem Produkt umgesetzt (Schema 3).

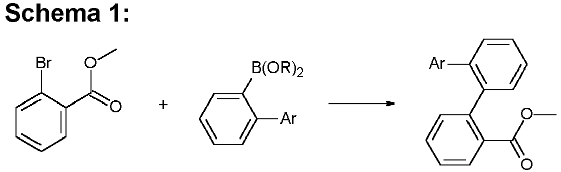

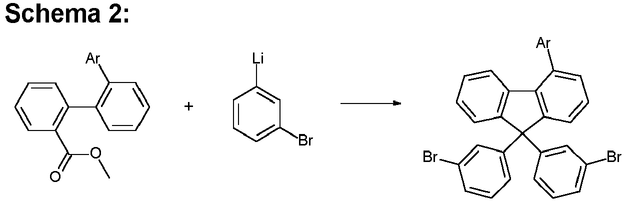

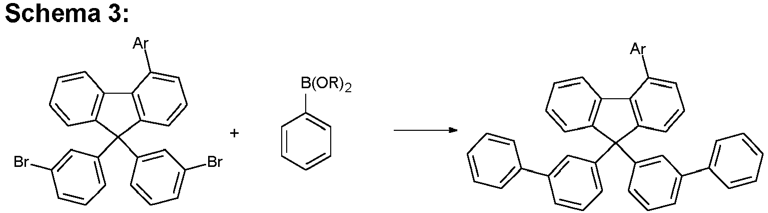

Nochmals ein weiterer Gegenstand der vorliegenden Erfindung ist eine Zusammensetzung enthaltend eine erfindungsgemäße Verbindung und wenigstens ein weiteres funktionelles Material, welches sich von den erfindungsgemäßen Verbindungen unterscheidet. Funktionelle Materialen sind generell die organischen oder anorganischen Materialien, welche zwischen Anode und Kathode eingebracht sind. Vorzugsweise ist das funktionelle Material ausgewählt aus der Gruppe bestehend aus fluoreszierenden Emittern, phosphoreszierenden Emittern, Hostmaterialien (Matrixmaterialien), Elektronentransportmaterialien, Elektroneninjektionsmaterialien, Lochleitermaterialien, Lochinjektionsmaterialien, Elektronenblockiermaterialien und Lochblockiermaterialien, insbesondere phosphoreszierenden Emittern und weiteren Matrixmaterialien.

Ein weiterer Gegenstand der vorliegenden Erfindung ist eine Zusammensetzung, enthaltend wenigstens eine Verbindung gemäß Formel (1) bzw. den bevorzugten Ausführungsformen sowie mindestens einen Emitter sowie mindestens ein Matrixmaterial ausgewählt aus der Gruppe bestehend aus Elektronentransportmaterialien, Lochtransportmaterialien und bipolaren Materialien. Emitter umfassen fluoreszierende Emitter, phosphoreszierende Emitter und Emitter, die TADF (thermally activated delayed fluorescence) zeigen. Bevorzugt sind phosphoreszierende Emitter. Bevorzugt handelt es sich bei dem Matrixmaterial um ein Elektronetransportmaterial oder ein bipolares Material, insbesondere ein Elektronentransportmaterial.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung enthaltend mindestens eine Verbindung gemäß Formel (1) oder gemäß den bevorzugten Ausführungsformen und ein oder mehrere Lösemittel, insbesondere organische Lösemittel, beispielsweise die oben aufgeführten Lösemittel. Dabei handelt es sich bevorzugt um Lösungen, Suspensionen oder Miniemulsionen, insbesondere Lösungen. Wie solche Lösungen hergestellt werden können, ist dem Fachmann bekannt und beispielsweise in der WO 2002/072714, der WO 2003/019694 und der darin zitierten Literatur beschrieben.

Die Verbindungen gemäß Formel (1) oder gemäß den bevorzugten Ausführungsformen eignen sich für den Einsatz in elektronischen Vorrichtungen, insbesondere in organischen Elektrolumineszenzvorrichtungen (OLEDs).

Ein weiterer Gegenstand der Erfindung ist daher die Verwendung von Verbindungen gemäß Formel (1) bzw. den bevorzugten Ausführungsformen in einer elektronischen Vorrichtung, insbesondere in einer organischen Elektrolumineszenzvorrichtung.

Ein weiterer Gegenstand der Erfindung ist eine elektronische Vorrichtung, enthaltend mindestens eine Verbindung gemäß Formel (1) bzw. den bevorzugten Ausführungsformen, wobei die elektronische Vorrichtung bevorzugt ausgewählt ist aus der Gruppe bestehend aus organischen Elektrolumineszenzvorrichtungen (OLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

Nochmals ein weiterer Gegenstand der Erfindung sind organische Elektrolumineszenzvorrichtungen, enthaltend Anode, Kathode und mindestens eine emittierende Schicht, dadurch gekennzeichnet, dass mindestens eine organische Schicht, bevorzugt eine emittierende Schicht, mindestens eine Verbindung gemäß Formel (1) oder den bevorzugten Ausführungsformen enthält.

Ferner ist ein weiterer Gegenstand der Erfindung eine organische Elektrolumineszenzvorrichtung, enthaltend eine emittierende Schicht, die mindestens eine Verbindung gemäß Formel (1) bzw. gemäß den bevorzugten Ausführungsformen und mindestens einen phosphoreszierenden Emitter und mindestens ein Matrixmaterial umfasst, welches sich von den erfindungsgemäßen Verbindungen unterscheidet. Vorzugsweise kann das weitere Matrixmaterial ein loch- und/oder ein elektronenleitendes Matrixmaterial oder ein bipolares Matrixmaterial, welches sowohl lochleitende als auch elektronenleitende Eigenschaften aufweist, sein. Besonders bevorzugt weist die erfindungsgemäße Verbindung die Eigenschaften eines Wide Bandgap Materials auf, und das weitere Matrixmaterial ist elektronenleitend.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Exzitonenblockierschichten, Ladungserzeugungsschichten und/oder organischen oder anorganischen p/n-Übergängen. Weiterhin können die Schichten, insbesondere die Ladungstransportschichten, auch dotiert sein. Die Dotierung der Schichten kann für einen verbesserten Ladungstransport vorteilhaft sein. Es sei aber darauf hingewiesen, dass nicht notwendigerweise jede dieser Schichten vorhanden sein muss und die Wahl der Schichten immer von den verwendeten Verbindungen abhängt.

Als Materialien für lochtransportierende Schichten von OLEDs können insbesondere Indenofluorenamin-Derivate, Aminderivate, Hexaazatriphenylenderivate, Aminderivate mit kondensierten Aromaten, Monobenzoindenofluorenamine, Dibenzoindenofluorenamine, Spirobifluoren-Amine, Fluoren-Amine, Spiro-Dibenzopyran-Amine, Dihydroacridin-Derivate, Spirodibenzofurane und Spirodibenzothiophene, Phenanthren-Diarylamine, Spiro-Tribenzotropolone, Spirobifluorene mit meta-Phenyldiamingruppen, Spiro-Bisacridine, Xanthen-Diarylamine, und 9,10-Dihydroanthracen-Spiroverbindungen mit Diarylaminogruppen eingesetzt werden.

Weiterhin sind die folgenden Verbindungen HT-1 bis HT-72 geeignet zur Verwendung in einer Schicht mit lochtransportierender Funktion, insbesondere in einer Lochinjektionsschicht, einer Lochtransportschicht und/oder einer Elektronenblockierschicht, oder zur Verwendung in einer emittierenden Schicht als Matrixmaterial, insbesondere als Matrixmaterial in einer emittierenden Schicht enthaltend einen oder mehrere phosphoreszierende Emitter:

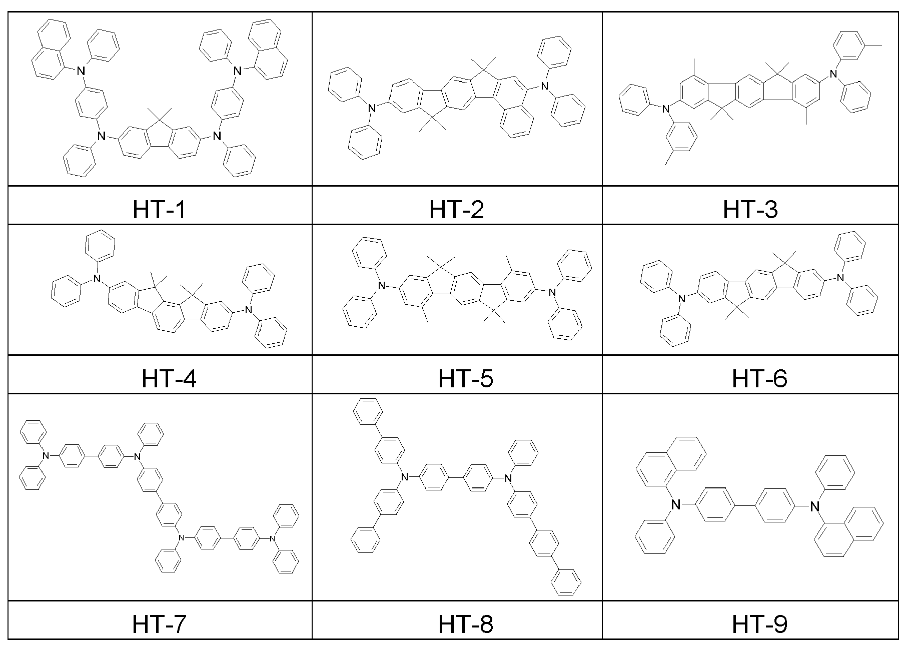

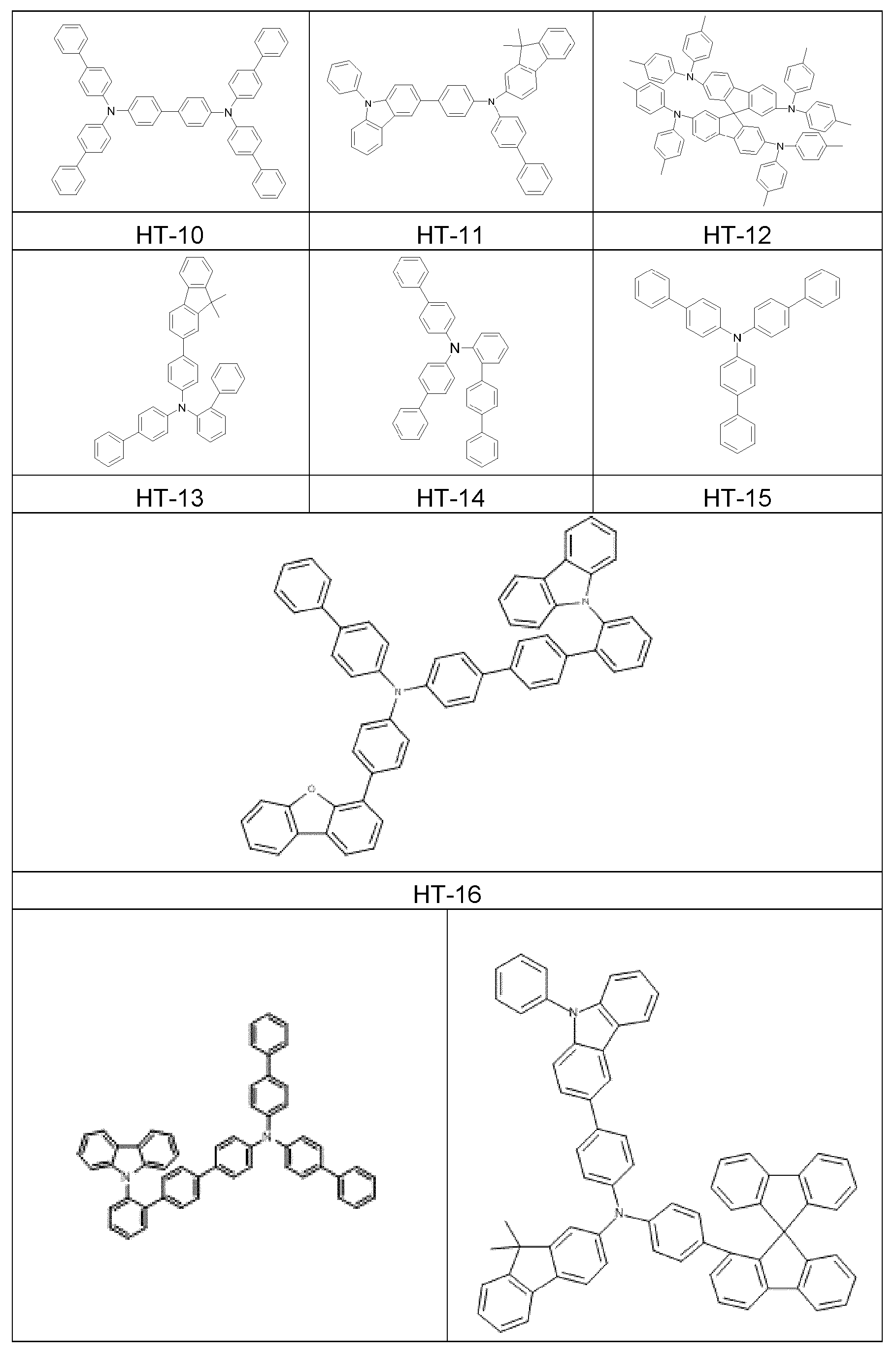

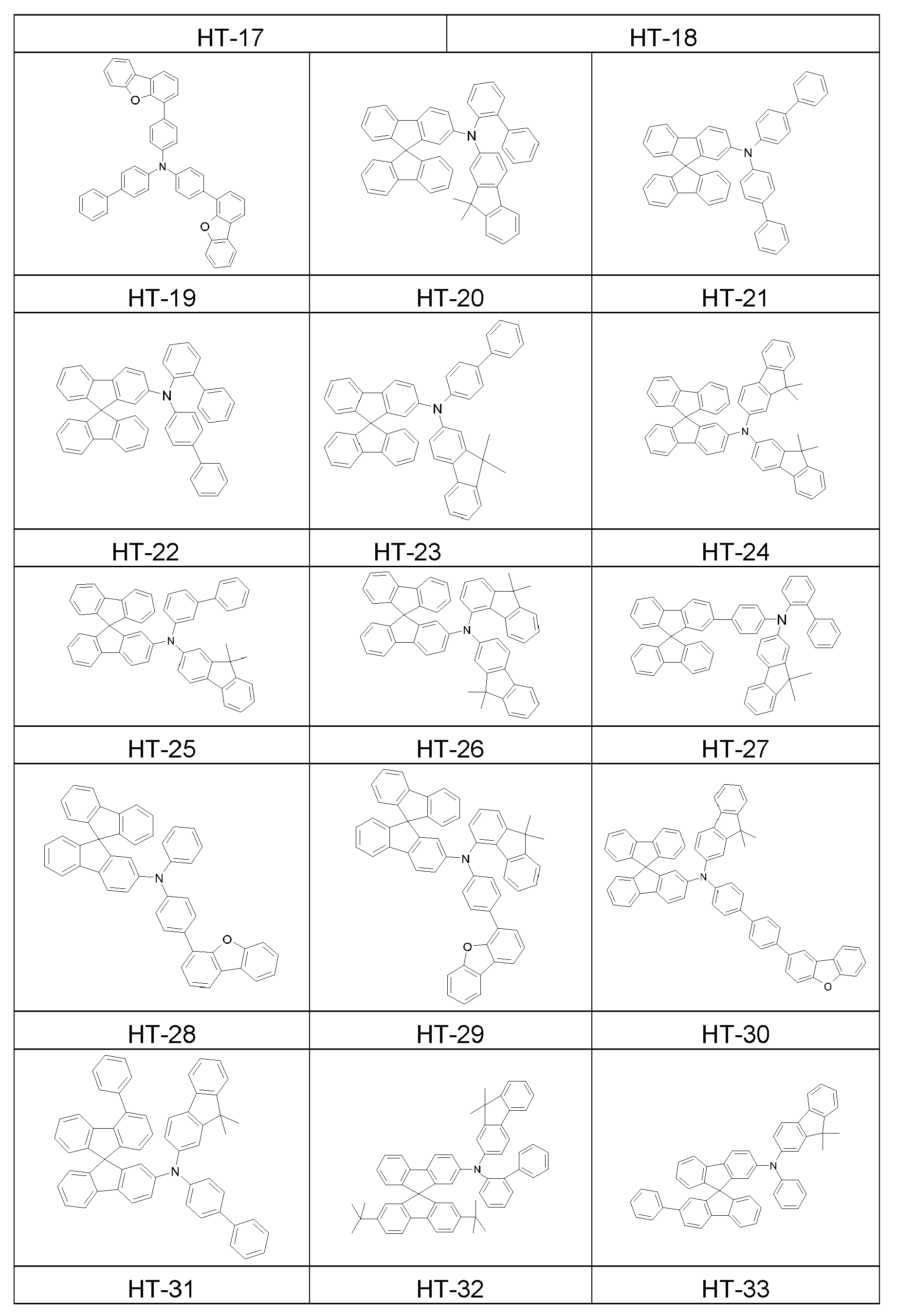

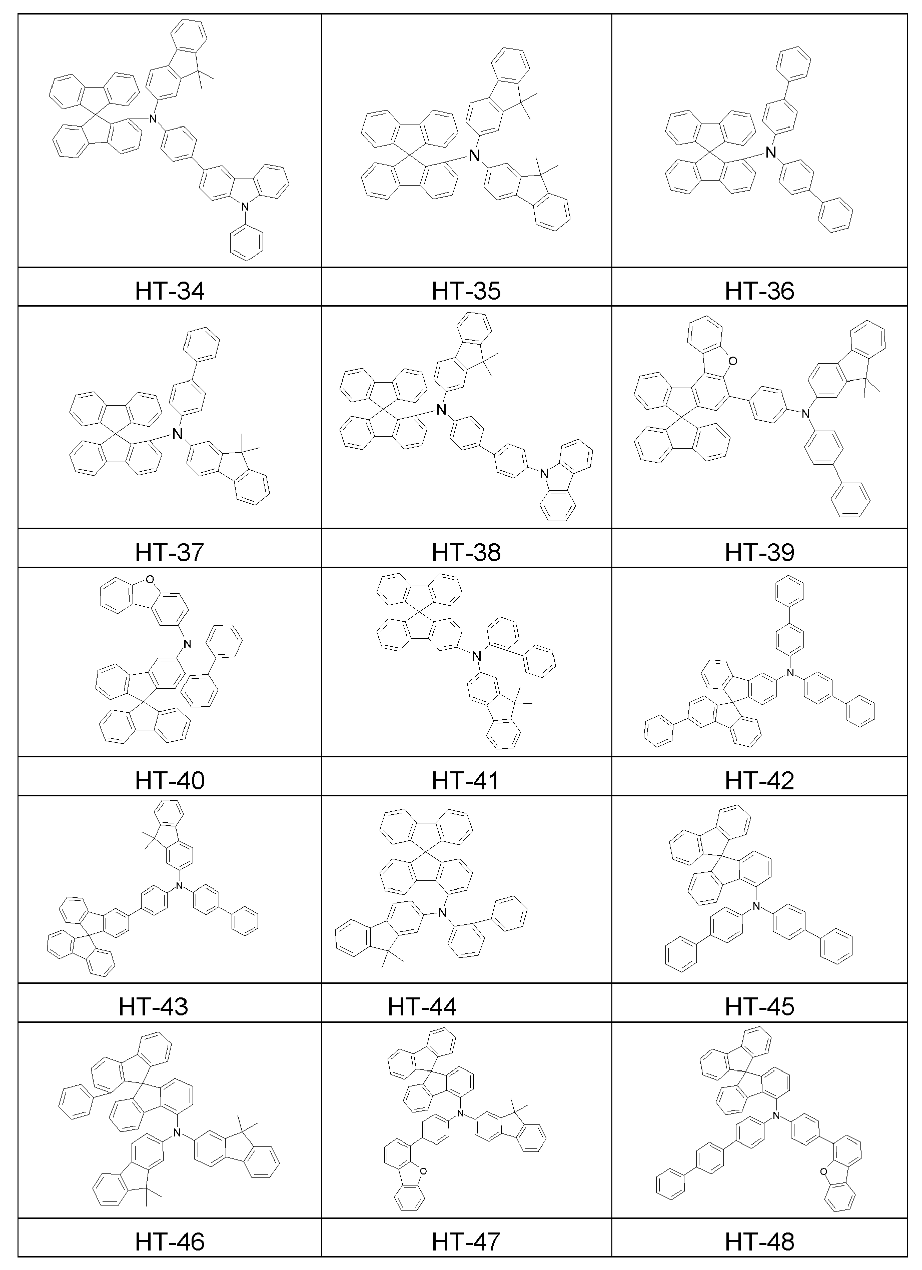

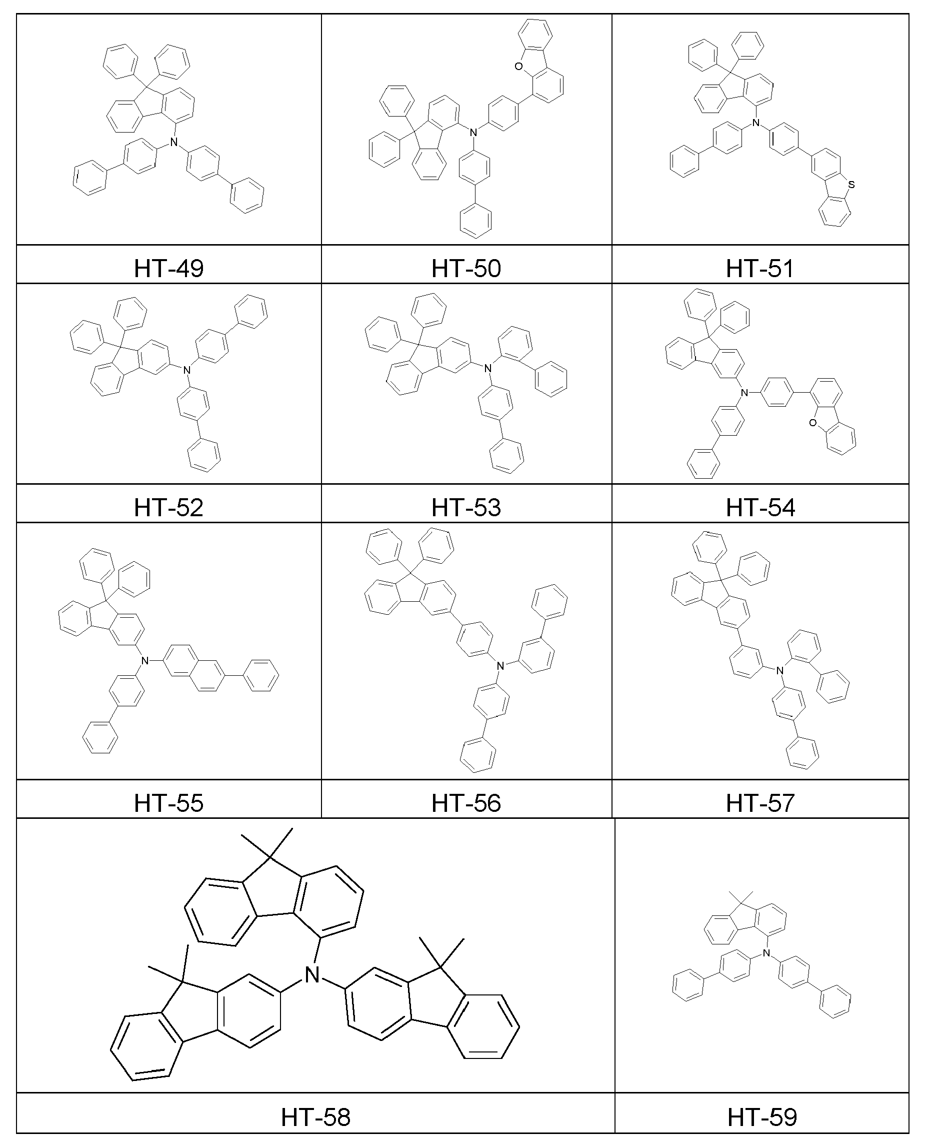

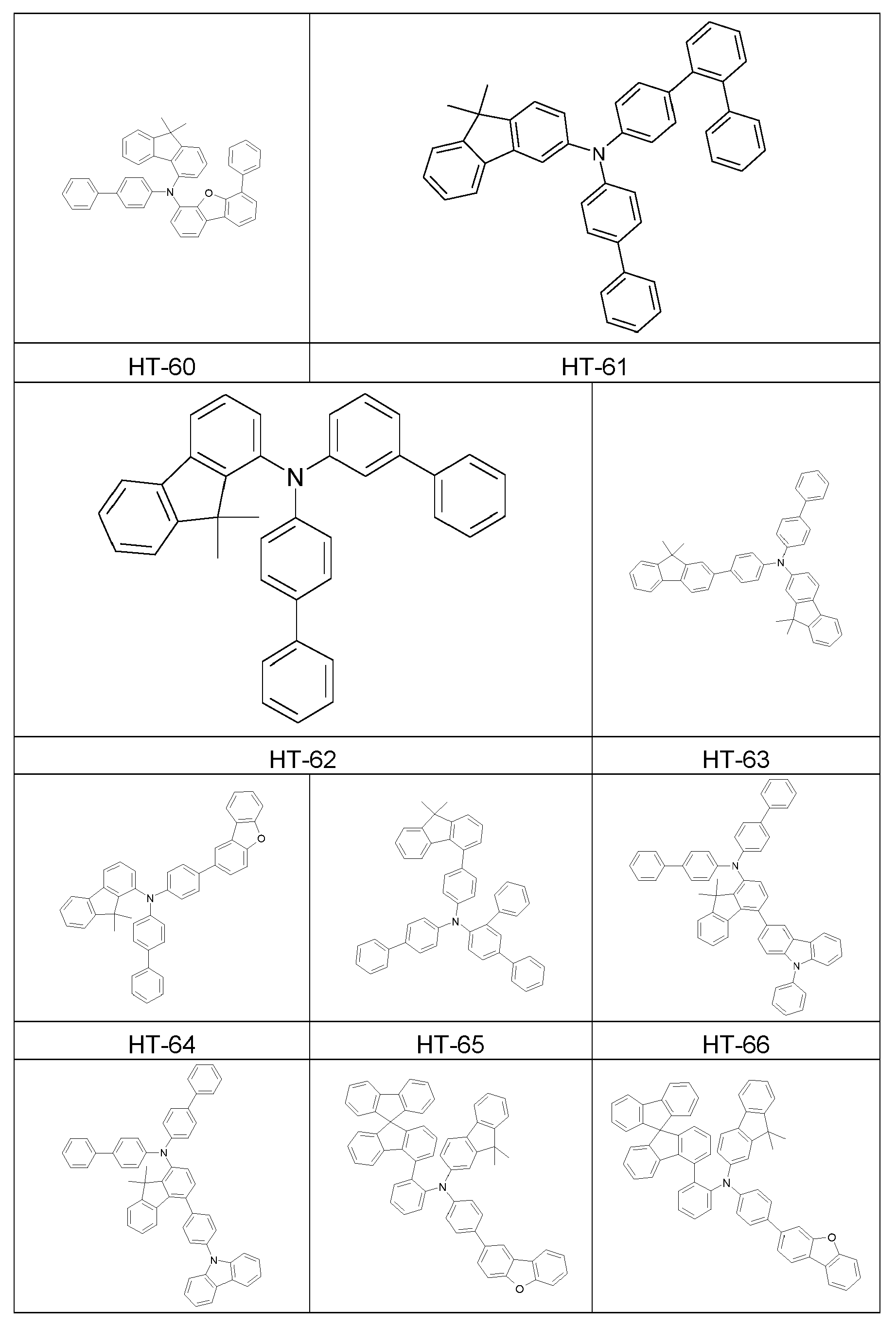

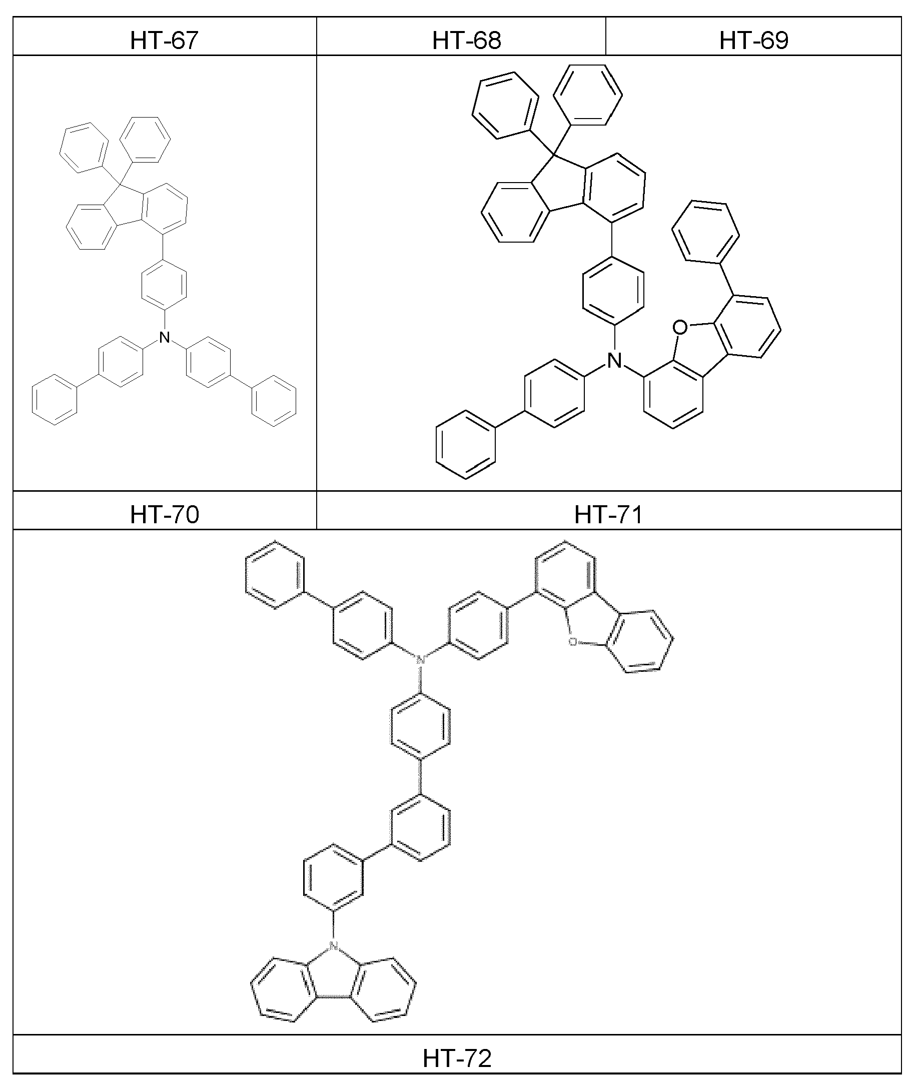

Die Verbindungen HT-1 bis HT-72 sind allgemein gut geeignet für die oben genannten Verwendungen in OLEDs jeglicher Bauart und Zusammensetzung, nicht nur in OLEDs gemäß der vorliegenden Anmeldung, die eine Verbindung gemäß Formel (1) enthalten. Die Verbindungen HT-1 bis HT-72 führen in OLEDs zu guten Leistungsdaten, insbesondere guter Lebensdauer und guter Effizienz.

Verfahren zur Herstellung der Verbindungen HT-1 bis HT-72 sind im Stand der Technik bekannt. Beispielsweise sind Verfahren zur Herstellung der Verbindungen HT-16, HT-17 und HT-72 in WO2014/079527, dort auf S. 32-33 und in den Ausführungsbeispielen, offenbart. Verfahren zur Herstellung der Verbindung HT-18 sind in WO 2013/120577 und WO2017/144150 in der Beschreibung und den Ausführungsbeispielen offenbart. Verfahren zur Herstellung der Verbindungen HT-20 bis HT-32 sind in WO2012/034627, dort auf S. 39-40 und in den Ausführungsbeispielen offenbart.

In einer Ausführungsform der Erfindung enthält die organische Elektrolumineszenzvorrichtung mehrere emittierende Schichten, wobei mindestens eine organische Schicht mindestens eine Verbindung gemäß Formel (1) bzw. den bevorzugten Verbindungen enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (1) bzw. gemäß den bevorzugten Ausführungsformen enthält und wobei die drei Schichten blaue, grüne und orange oder rote Emission zeigen.

In einer bevorzugten Ausführungsform der Erfindung werden die Verbindungen gemäß Formel (1) oder gemäß den bevorzugten Ausführungsformen als Matrixmaterial für phosphoreszierende Verbindungen in einer emittierenden Schicht eingesetzt. Dabei wird bevorzugt als Matrix eine Mischung eingesetzt, wobei mindestens eine Komponente dieser Mischung eine Verbindung gemäß Formel (1) oder den bevorzugten Ausführungsformen ist. Bevorzugt ist die andere Komponente dieser Mischung eine Lochtransportverbindung und/oder eine Elektronentransportverbindung und/oder eine bipolare Verbindung, insbesondere eine Elektronentransportverbindung. Bevorzugte Elektronentransportverbindungen sind Triazinderivate, Chinazolinderivate, Pyrimidinderivate und Lactame, wie sie nachfolgend genauer ausgeführt werden.

Der Anteil des erfindungsgemäßen Matrixmaterials gemäß Formel (1) liegt bevorzugt im Bereich von 5 bis 95 Gew.-%, besonders bevorzugt im Bereich von 20 bis 85 Gew.-% und ganz besonders bevorzugt im Bereich von 50 bis 75 Gew.-%, bezogen auf das Matrixmaterial. Entsprechend beträgt der Anteil der als elektronen- und/oder lochleitenden Matrixmaterial eingesetzten Verbindung 95 bis 5 Gew.-%, besonders bevorzugt 80 bis 15 Gew.-% und ganz besonders bevorzugt 50 bis 25 Gew.-%, bezogen auf das Matrixmaterial.

Bevorzugte Triazin-, Chinazolin- bzw. Pyrimidinderivate, welche als Mischung zusammen mit den erfindungsgemäßen Verbindungen eingesetzt werden können, sind die Verbindungen der folgenden Formeln (10), (11) und (12), 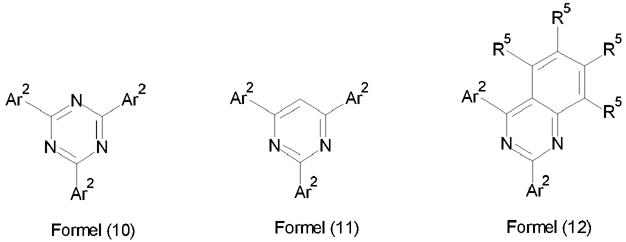 wobei R³ die oben genannten Bedeutungen aufweist und für die weiteren verwendeten Symbole gilt:
- Ar²: ist bei jedem Auftreten gleich oder verschieden ein aromatisches oder heteroaromatisches Ringsystem mit 5 bis 40 aromatischen Ringatomen, welches durch einen oder mehrere Reste R⁵ substituiert sein kann;
- R⁵: ist bei jedem Auftreten gleich oder verschieden H, D, F, Cl, Br, I, CN, NO₂, N(R³)₂, OR³, SR³, COOR³, C(=O)N(R³)₂, Si(R³)₃, B(OR³)₂, C(=O)R³, P(=O)(R³)₂, S(=O)R³, S(=O)₂R³, OSO₂R³, eine geradkettige Alkyl- oder Alkoxygruppe mit 1 bis 20 C-Atomen oder eine Alkenyloder Alkinylgruppe mit 2 bis 20 C-Atomen oder eine verzweigte oder cyclische Alkyl- oder Alkoxygruppe mit 3 bis 20 C-Atomen, wobei die Alkyl-, Alkoxy-, Alkenyl- oder Alkinylgruppe jeweils mit einem oder mehreren Resten R³ substituiert sein kann, wobei eine oder mehrere nicht benachbarte CH₂-Gruppen durch Si(R³)₂, C=O, NR³, O, S oder CONR³ ersetzt sein können, oder ein aromatisches oder heteroaromatisches Ringsystem mit 5 bis 30 aromatischen Ringatomen, das jeweils durch einen oder mehrere Reste R³ substituiert sein kann; dabei können zwei Reste R⁵ auch miteinander ein aliphatisches, heteroaliphatisches, aromatisches oder heteroaromatisches Ringsystem bilden.

Besonders bevorzugt sind die Triazinderivate der Formel (10) und die Chinazolinderivate der Formel (12), insbesondere die Triazinderivate der Formel (10).

In einer bevorzugten Ausführungsform der Erfindung ist Ar² bei jedem Auftreten gleich oder verschieden ein aromatisches oder heteroaromatisches Ringsystem mit 6 bis 30 aromatischen Ringatomen, insbesondere mit 6 bis 24 aromatischen Ringatomen, das durch einen oder mehrere Reste R⁵ substituiert sein kann.

Geeignete aromatische bzw. heteroaromatische Ringsysteme Ar² sind bei jedem Auftreten gleich oder verschieden ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R⁵, bevorzugt nicht-aromatischen Resten R⁵, substituiert sein können. Wenn Ar² für eine Heteroarylgruppe, insbesondere für Triazin, Pyrimidin, Chinazolin oder Carbazol steht, können auch aromatische oder heteroaromatische Reste R⁵ an dieser Heteroarylgruppe bevorzugt sein.

Dabei ist Ar² bevorzugt bei jedem Auftreten gleich oder verschieden gewählt aus den Gruppen der folgenden Formeln Ar²-1 bis Ar²-76, 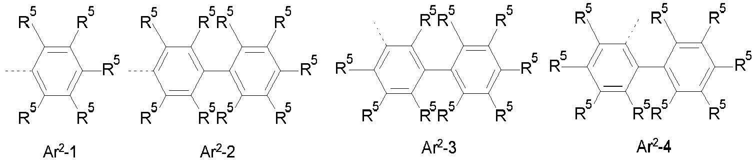 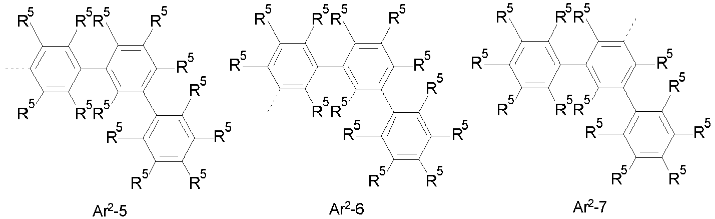 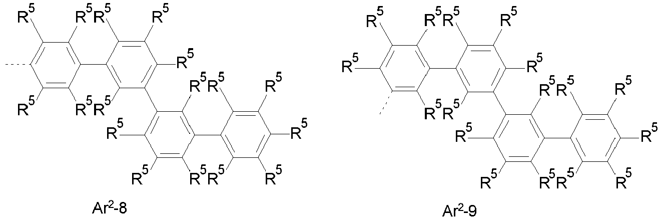 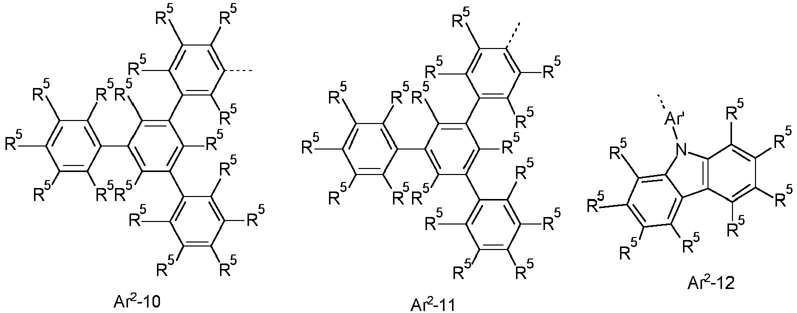 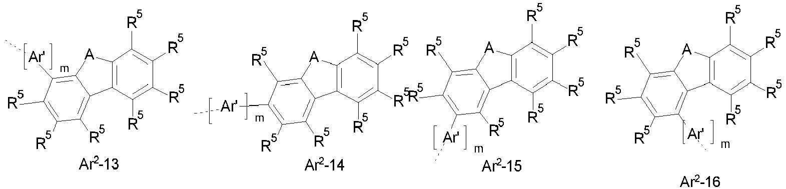 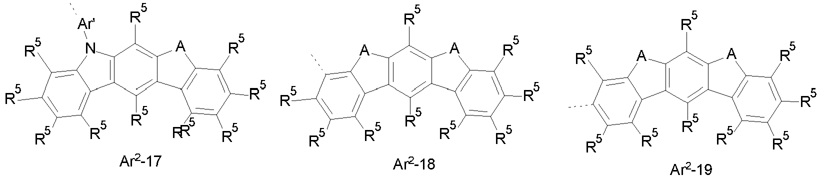 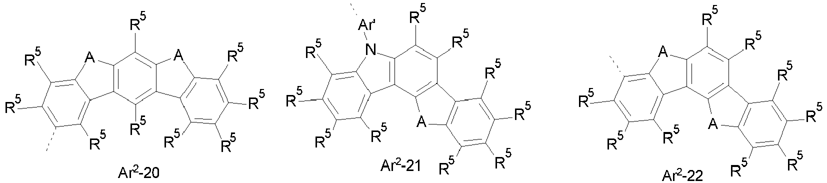 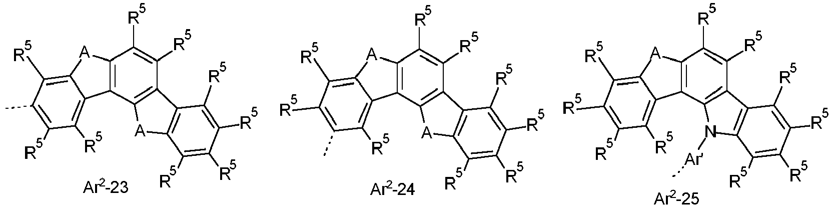 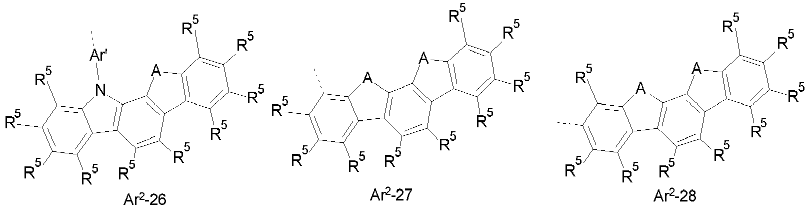 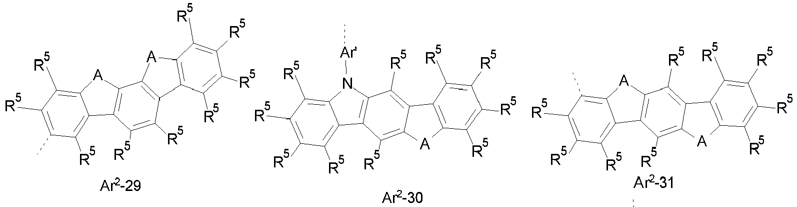 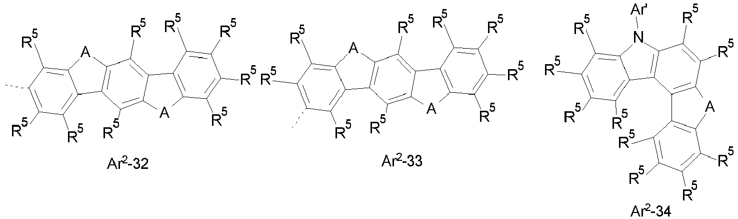 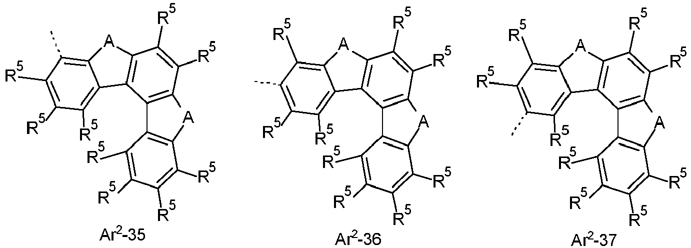 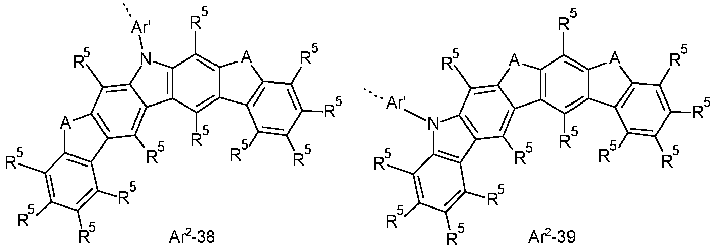 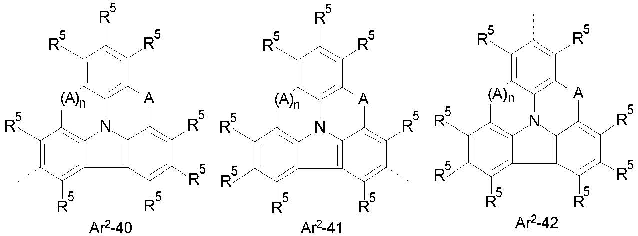 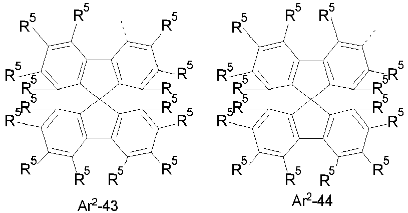 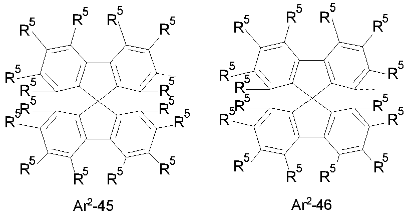 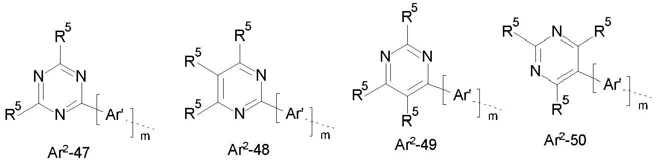 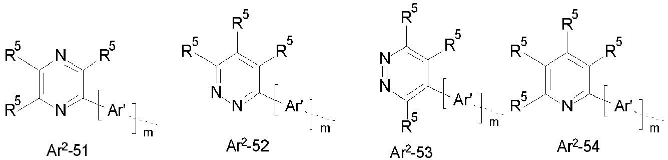 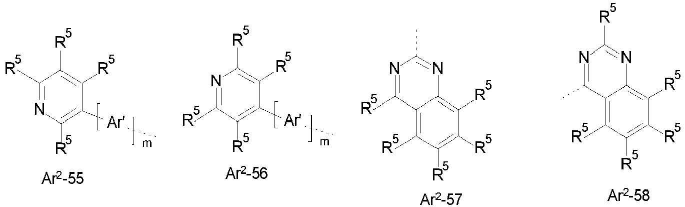 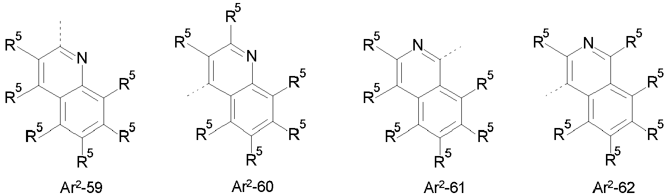 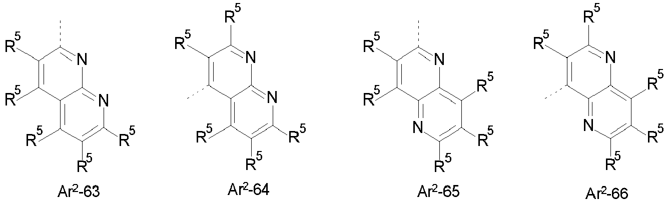 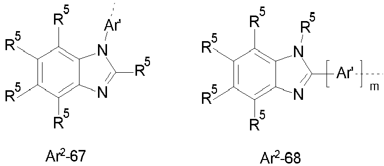 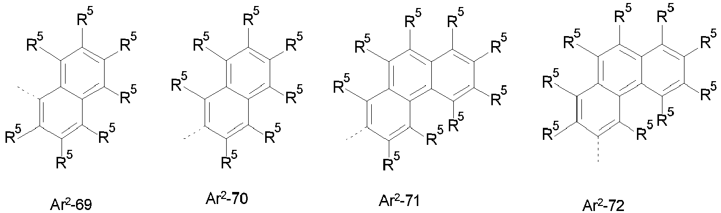 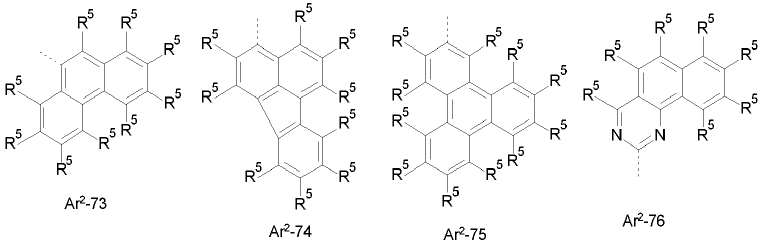 wobei R⁵ die oben genannten Bedeutungen aufweist, die gestrichelte Bindung die Bindung an das Triazin bzw. Pyrimidin bzw. Chinazolin darstellt und weiterhin gilt:
- Ar': ist bei jedem Auftreten gleich oder verschieden ein bivalentes aromatisches oder heteroaromatisches Ringsystem mit 6 bis 18 aromatischen Ringatomen, bevorzugt mit 6 bis 13 aromatischen Ringatomen, welches jeweils mit einem oder mehreren Resten R⁵ substituiert sein kann;
- A: ist bei jedem Auftreten gleich oder verschieden C(R⁵)₂, NR⁵, O oder S;
- n: ist 0 oder 1, wobei n = 0 bedeutet, dass an dieser Position keine Gruppe A gebunden ist und an die entsprechenden Kohlenstoffatome statt dessen Reste R⁵ gebunden sind;
- m: ist 0 oder 1, wobei m = 0 bedeutet, dass die Gruppe Ar' nicht vorhanden ist und dass die entsprechende aromatische bzw. heteroaromatische Gruppe direkt an das Triazin bzw. Pyrimidin bzw. Chinazolin gebunden ist.

Beispiele für geeignete Triazinverbindungen, welche als Matrixmaterialien zusammen mit den erfindungsgemäßen Verbindungen eingesetzt werden können, sind die in der folgenden Tabelle abgebildeten Verbindungen.

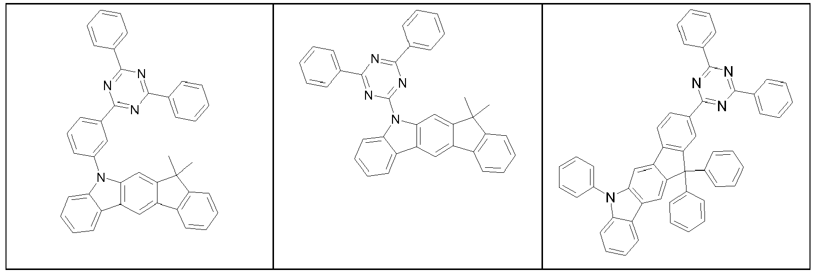

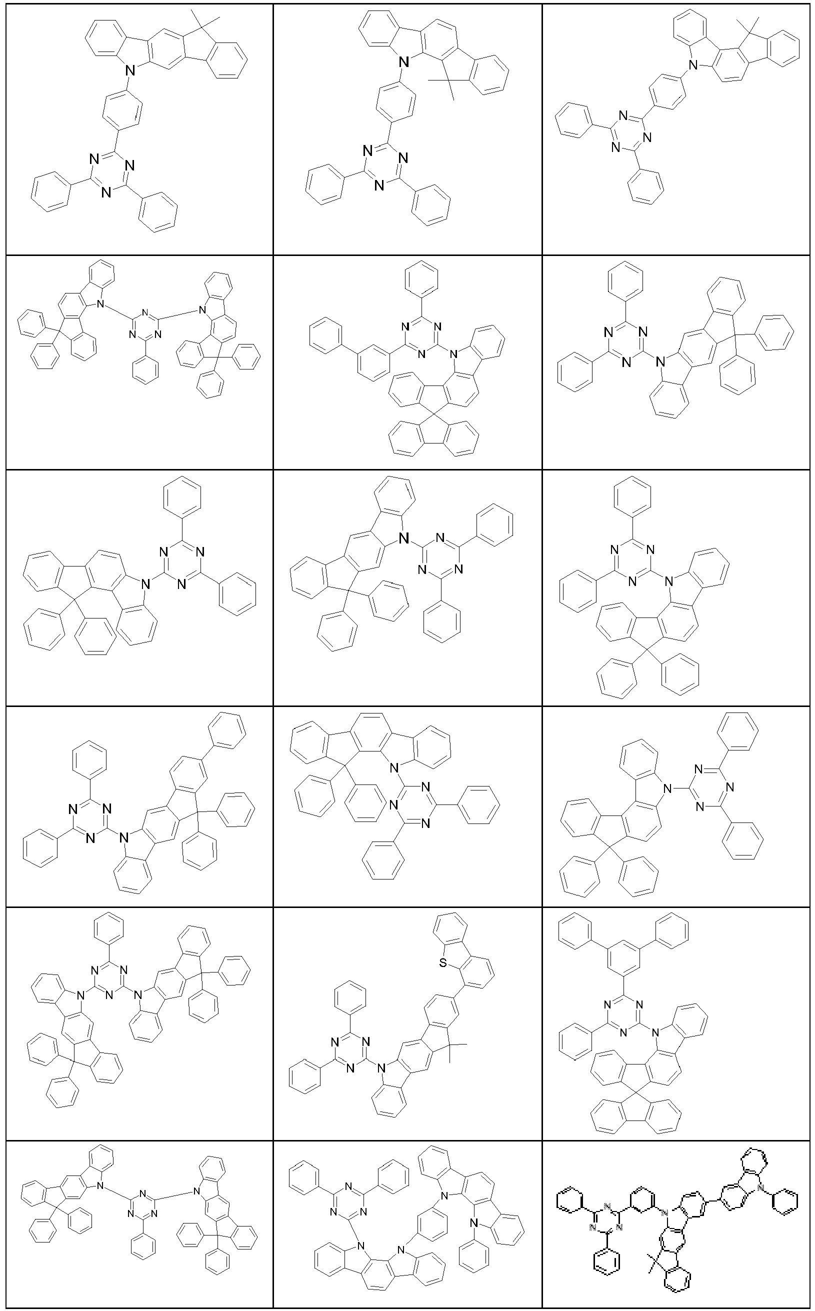

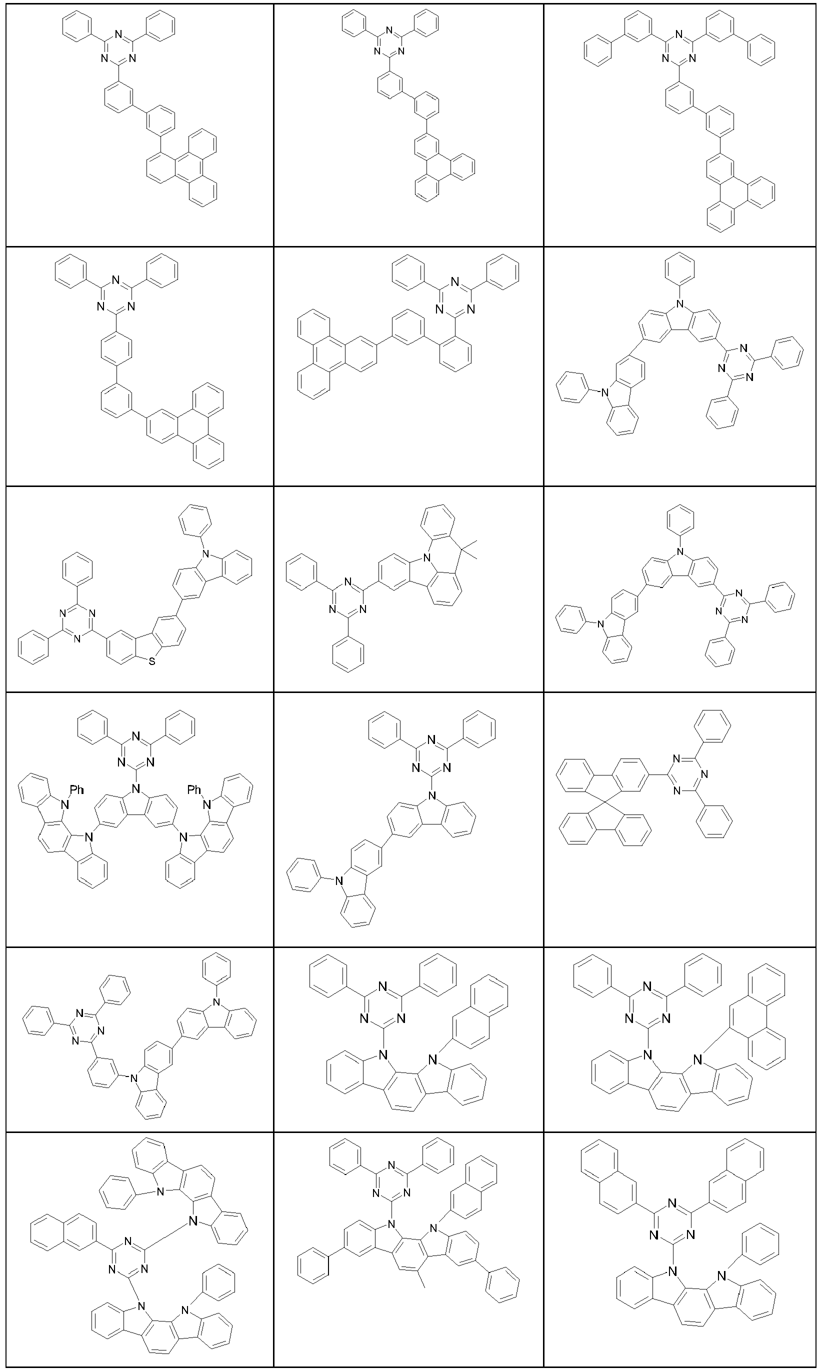

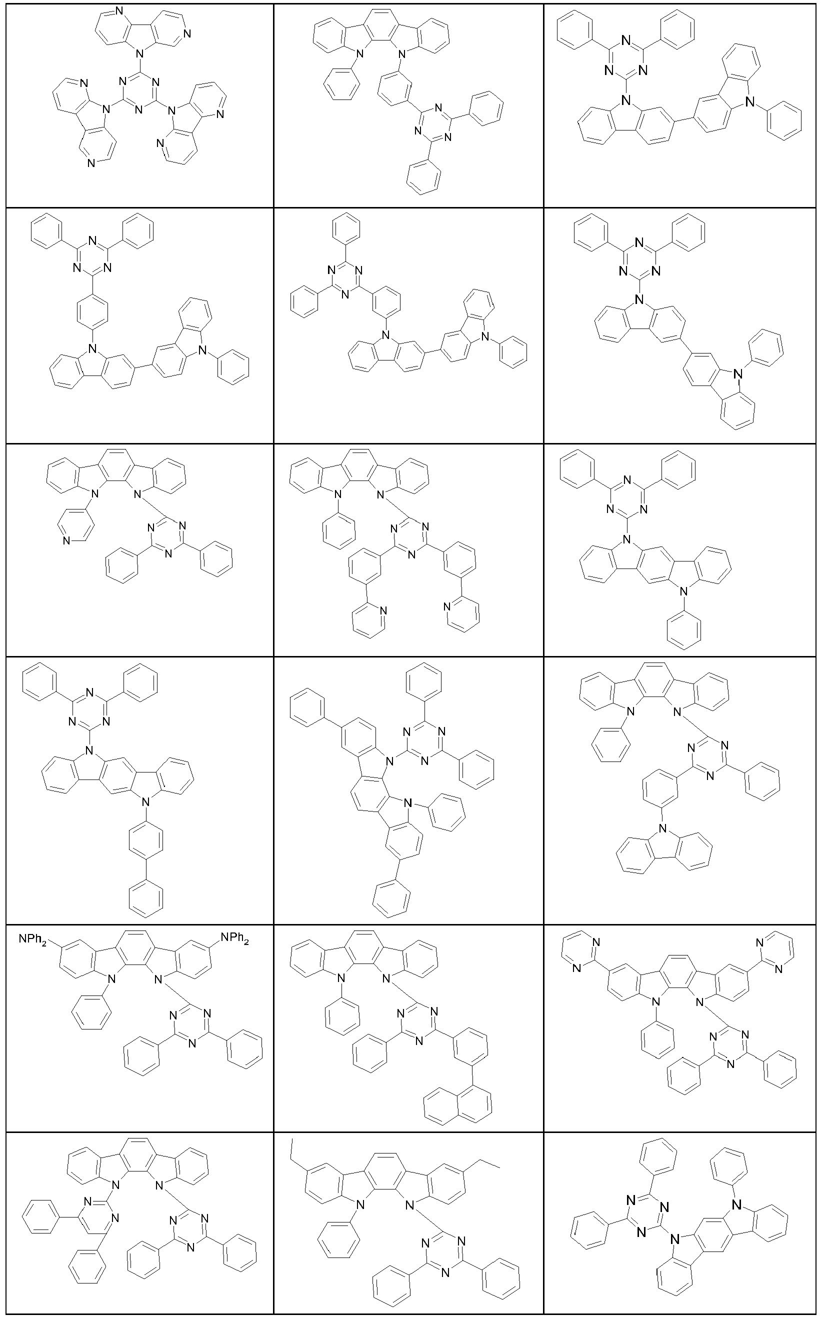

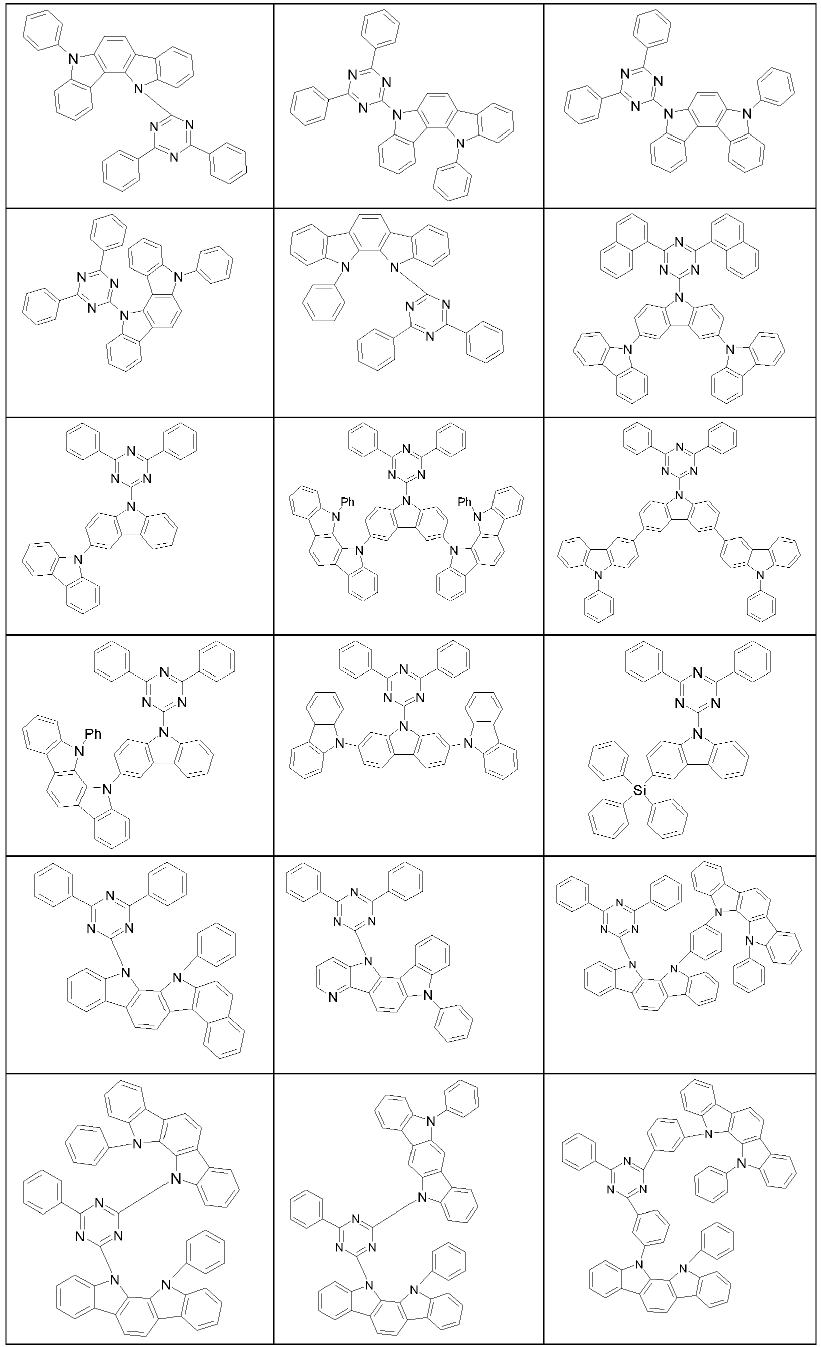

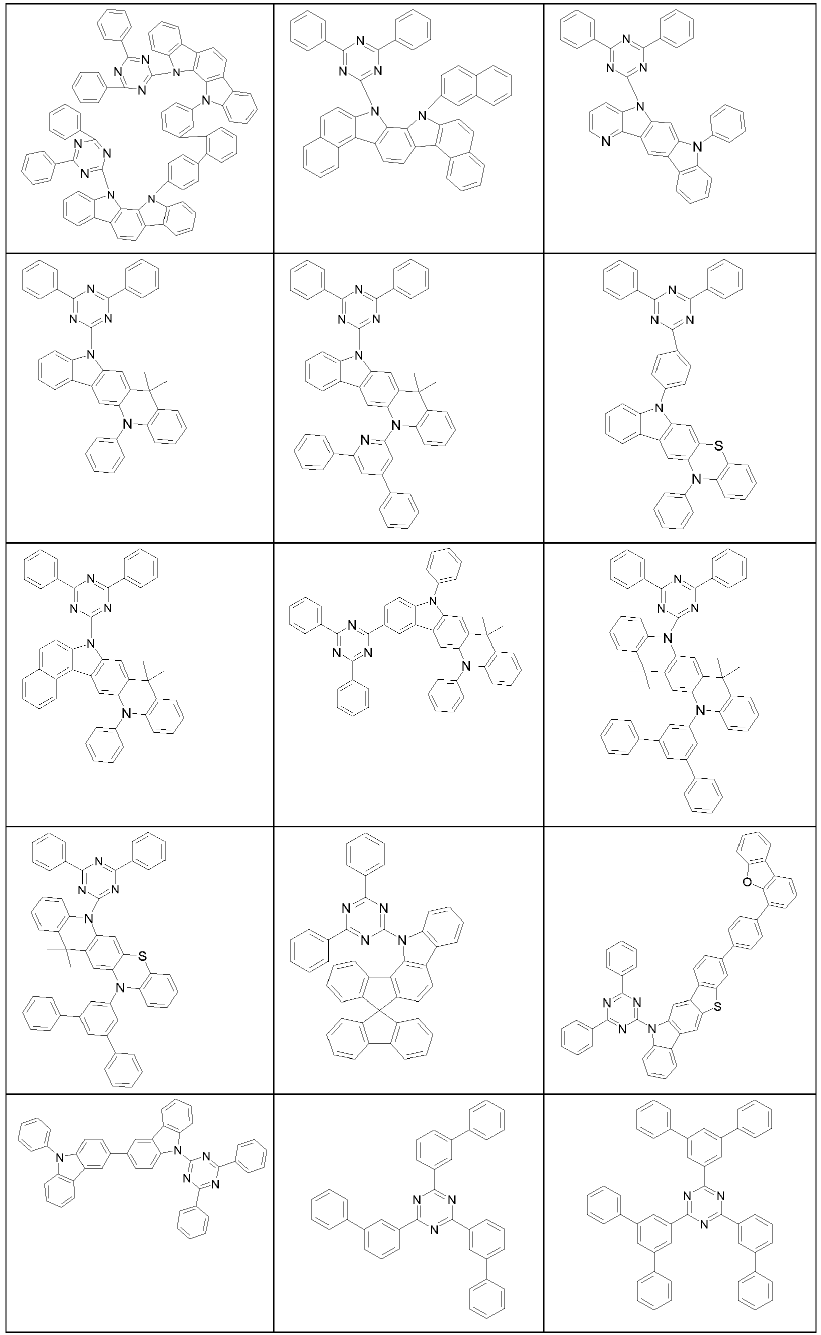

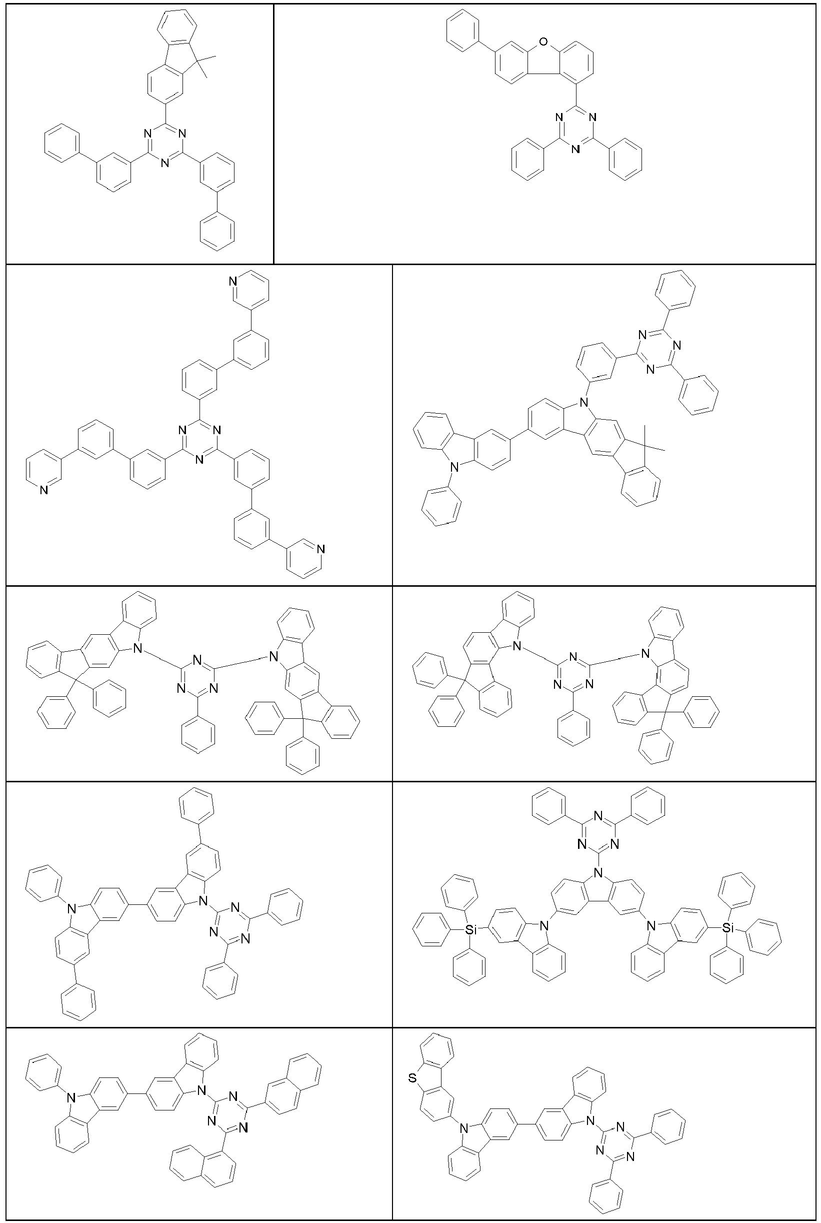

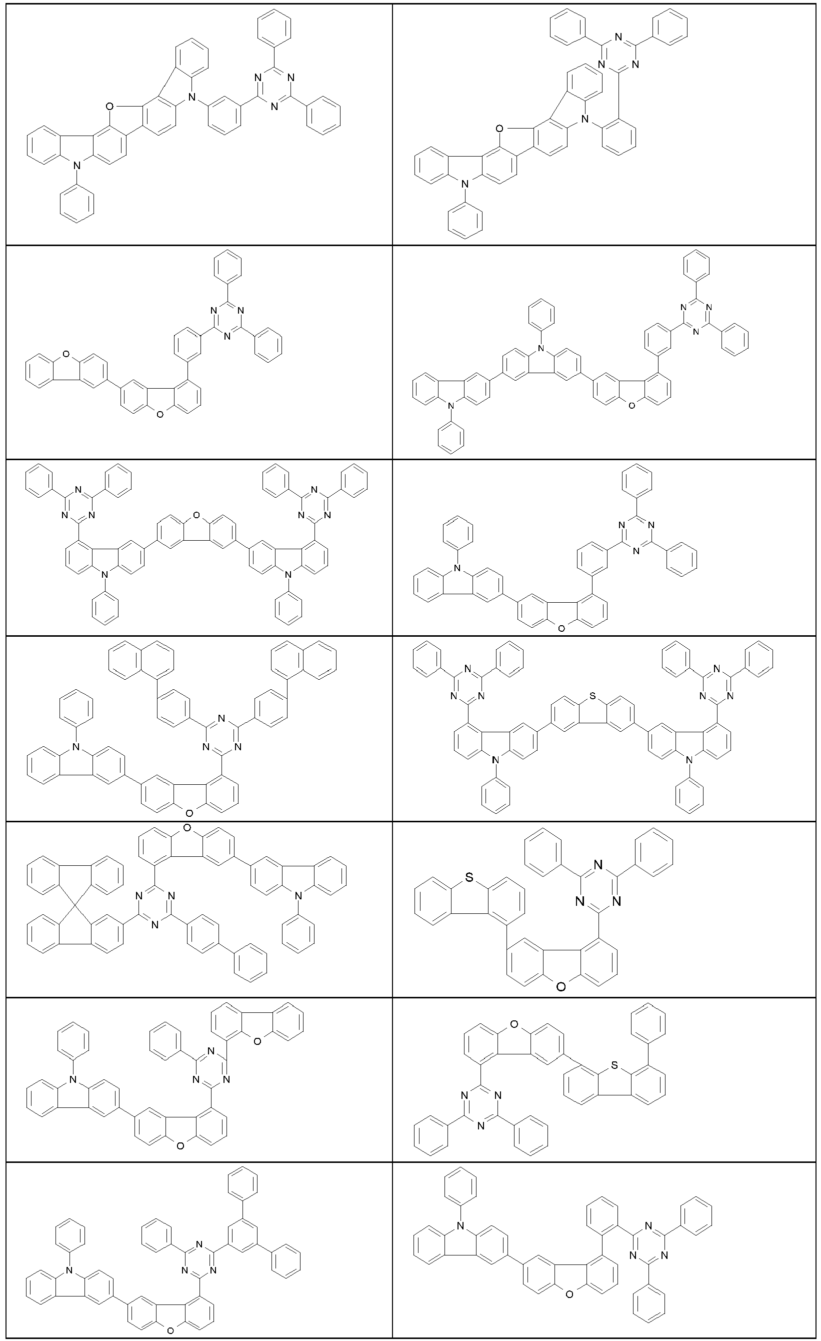

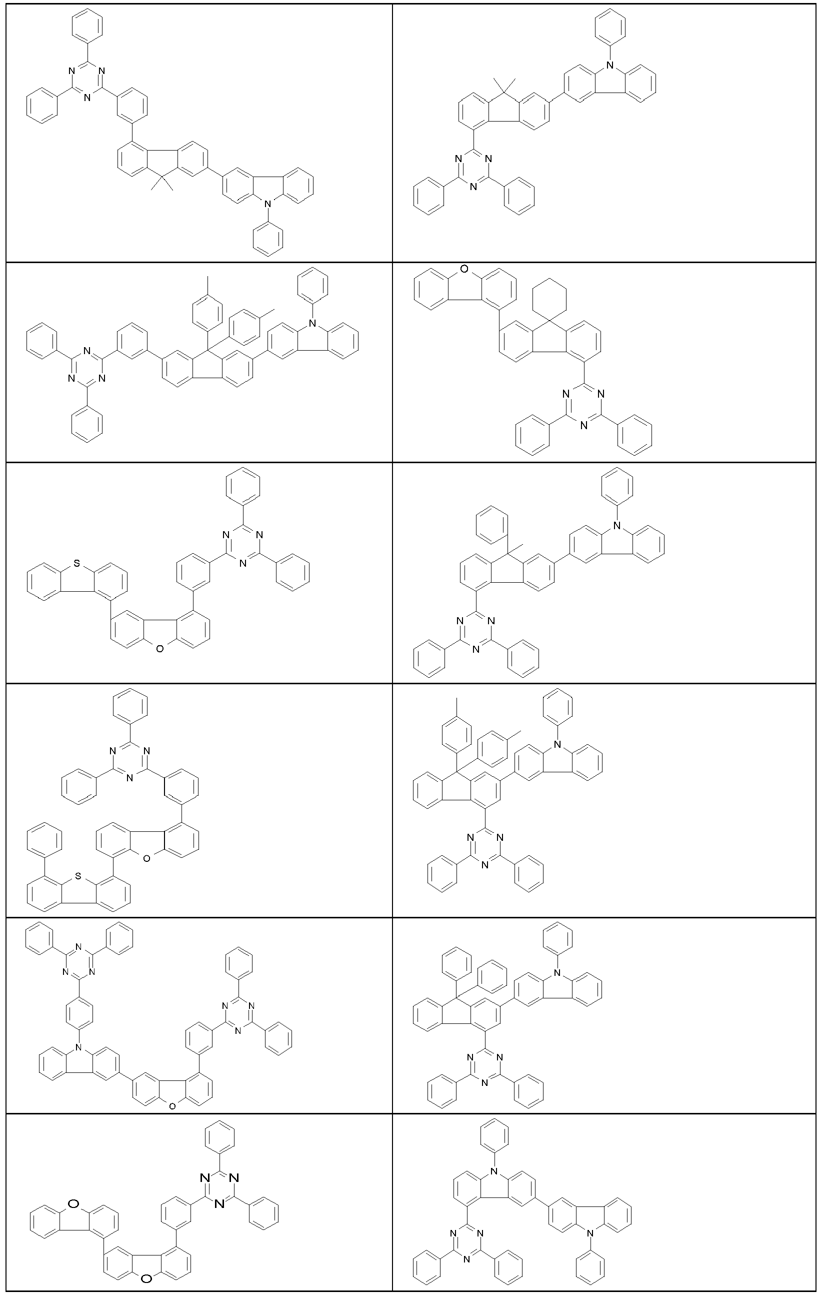

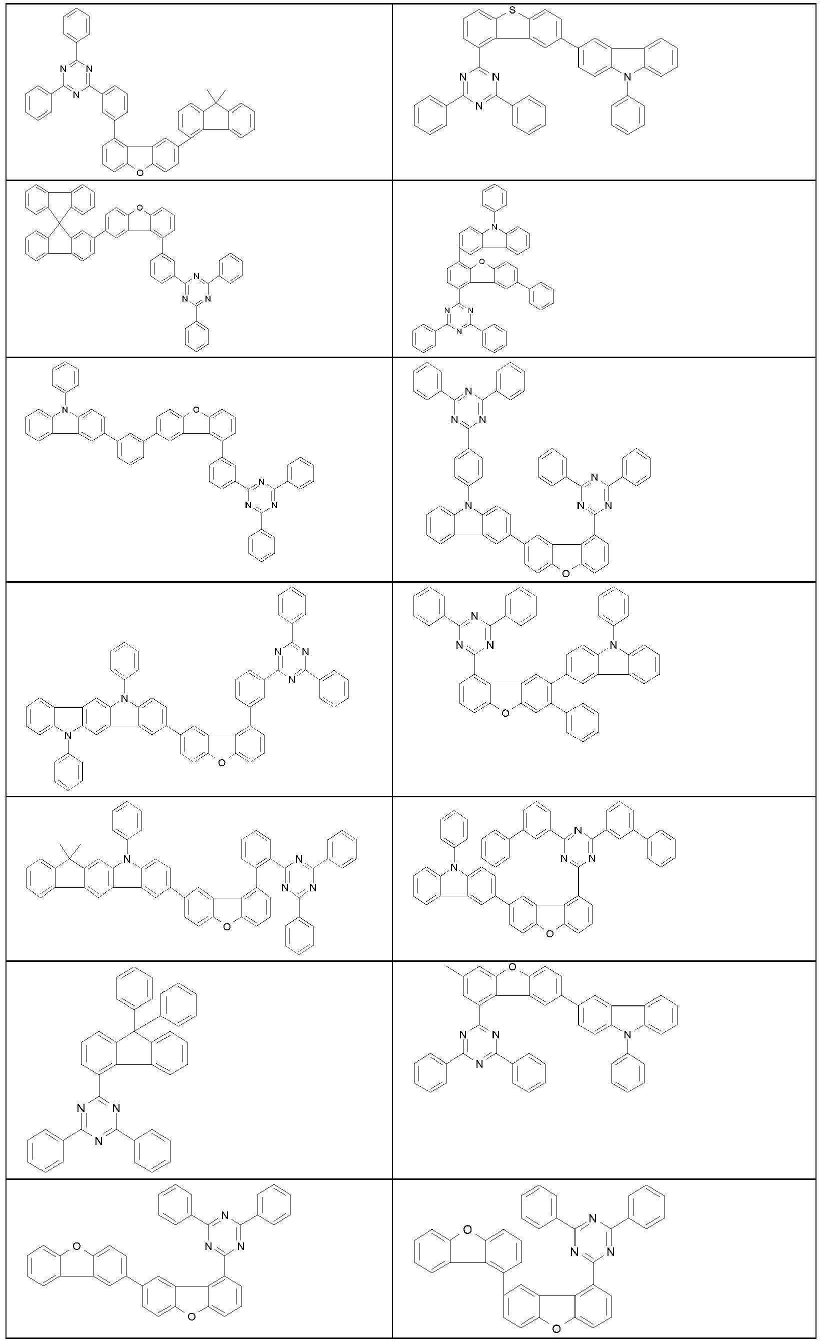

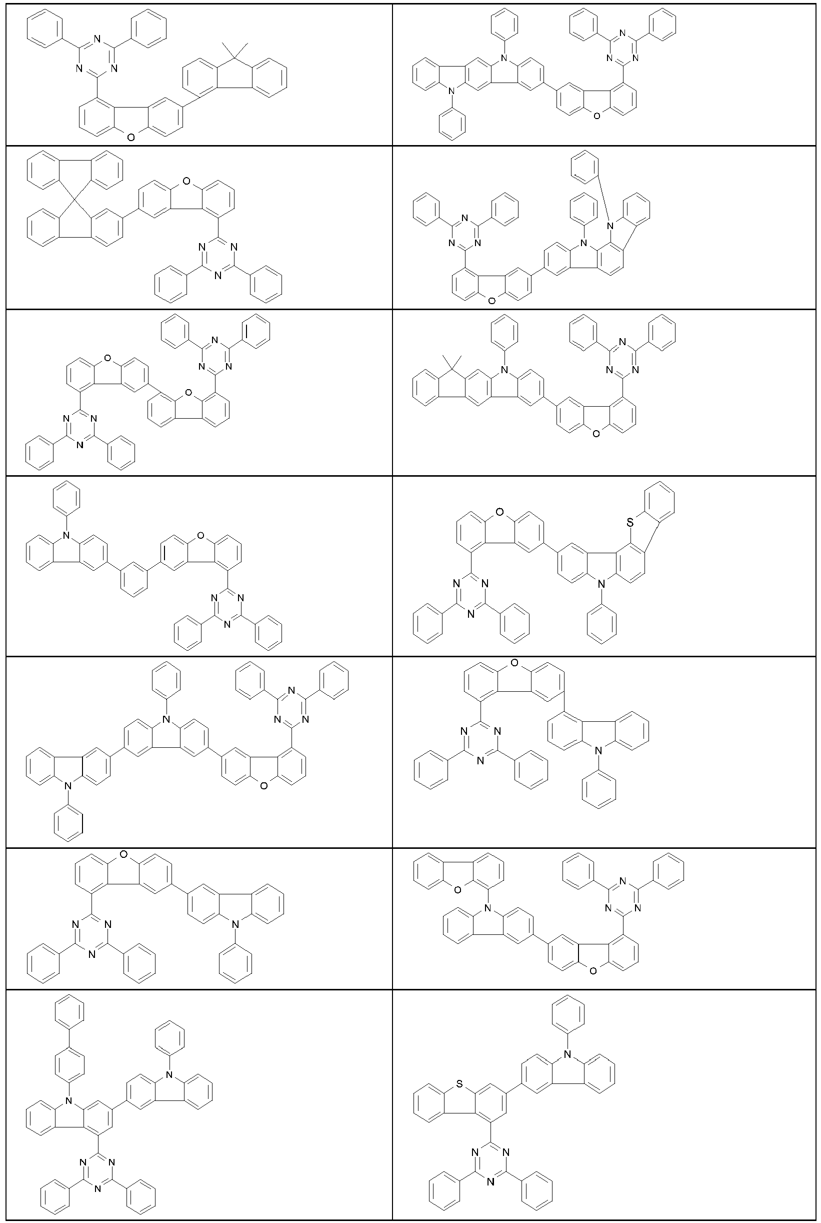

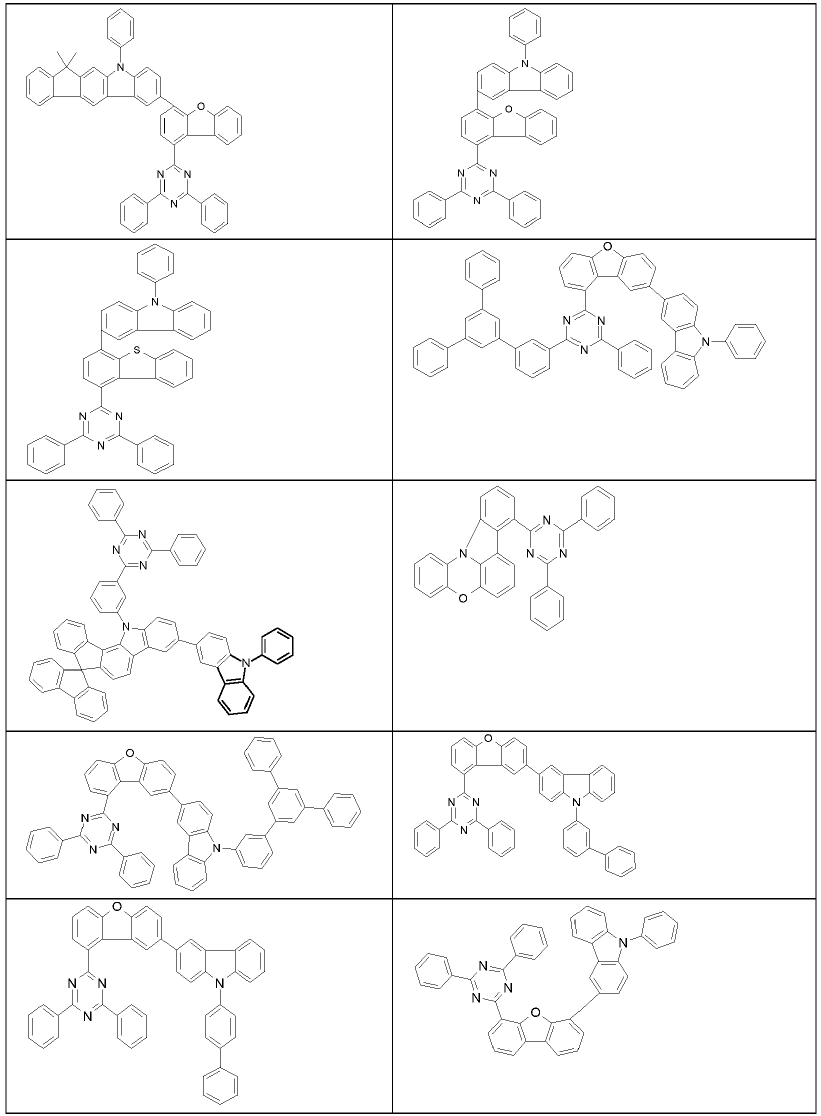

Beispiele für geeignete Chinazolinverbindungen sind die in der folgenden Tabelle abgebildeten Verbindungen:

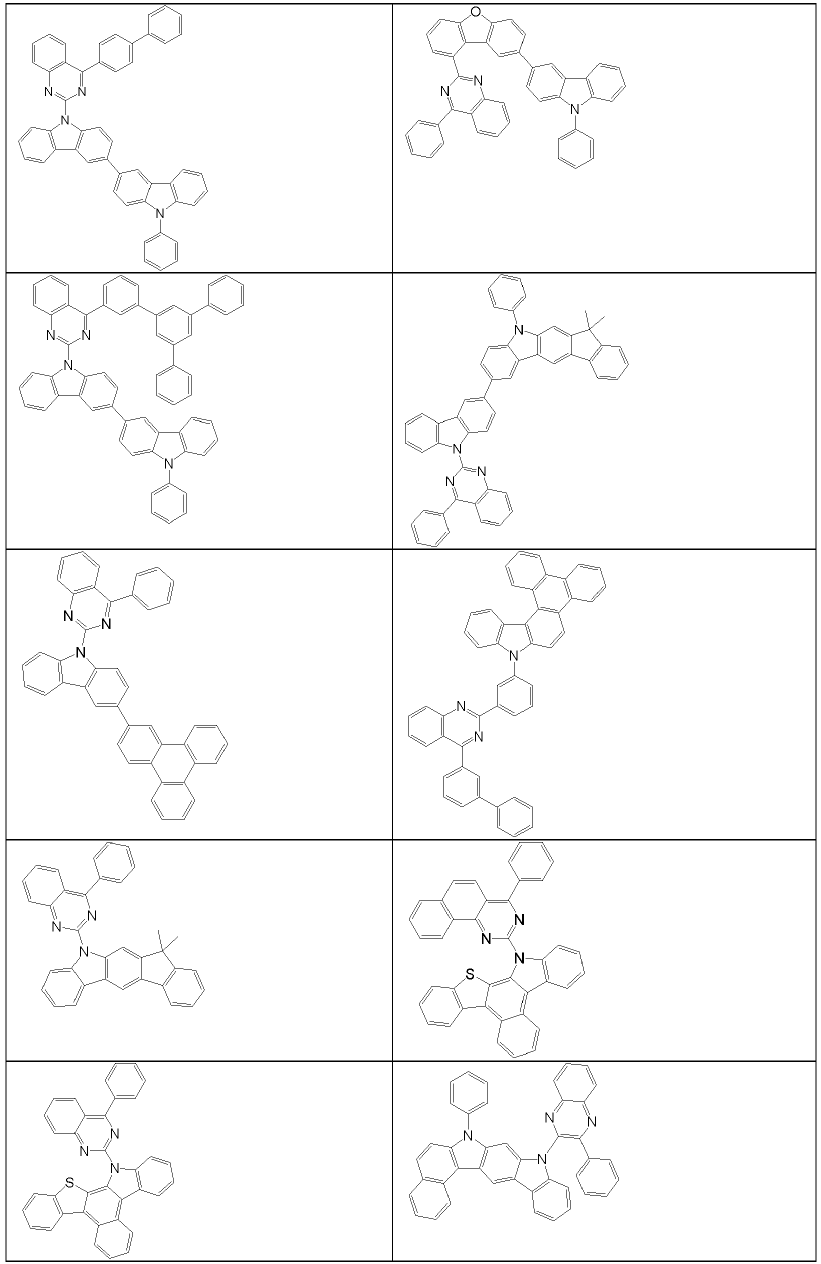

Wenn die Verbindung gemäß Formel (1) oder gemäß den bevorzugten Ausführungsformen als Matrixmaterial für eine emittierende Verbindung in einer emittierenden Schicht eingesetzt wird, kann sie in Kombination mit einem oder mehreren phosphoreszierenden Materialien (Triplettemitter) eingesetzt werden, wobei rot, orange, gelb oder grün phosphoreszierende Materialien bevorzugt sind. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Erfindung werden alle insbesondere lumineszierenden Iridium-, Platin- und Kupferverbindungen als phosphoreszierende Materialien bezeichnet. Die Mischung aus der Verbindung gemäß Formel (1) oder gemäß den bevorzugten Ausführungsformen und der emittierenden Verbindung enthält dann zwischen 99 und 1 Gew.-%, bevorzugt zwischen 98 und 10 Gew.-%, besonders bevorzugt zwischen 97 und 60 Gew.-% und ganz besonders bevorzugt zwischen 95 und 70 Gew.-% der Verbindung gemäß Formel (1) oder gemäß den bevorzugten Ausführungsformen bezogen auf die Gesamtmischung aus Emitter und Matrixmaterial. Entsprechend enthält die Mischung zwischen 1 und 99 Gew.-%, bevorzugt zwischen 2 und 90 Gew.-%, besonders bevorzugt zwischen 3 und 40 Gew.-% und ganz besonders bevorzugt zwischen 5 und 30 Gew.-% des Emitters bezogen auf die Gesamtmischung aus Emitter und Matrixmaterial. Bei Verwendung einer Mischung aus mehreren Emittern anstatt nur eines Emitters gelten diese Bevorzugungen entsprechend für den Gesamtanteil an Emittern in der emittierenden Schicht.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Es kann auch bevorzugt sein, wenn die Elektrolumineszenzvorrichtung zwei oder mehrere phosphoreszierende Dotanden enthält, wobei der kürzerwellig emittierende Dotand dann als Co-Matrix für den längerwellig emittierenden Dotanden eingesetzt wird. In dieser Ausführungsform ist es insbesondere bevorzugt, wenn einer der Dotanden rot emittiert und der andere Dotand gelb oder grün emittiert.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186 und WO 2018/041769, WO 2019/020538, WO 2018/178001, WO 2019/115423 und WO 2019/158453 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

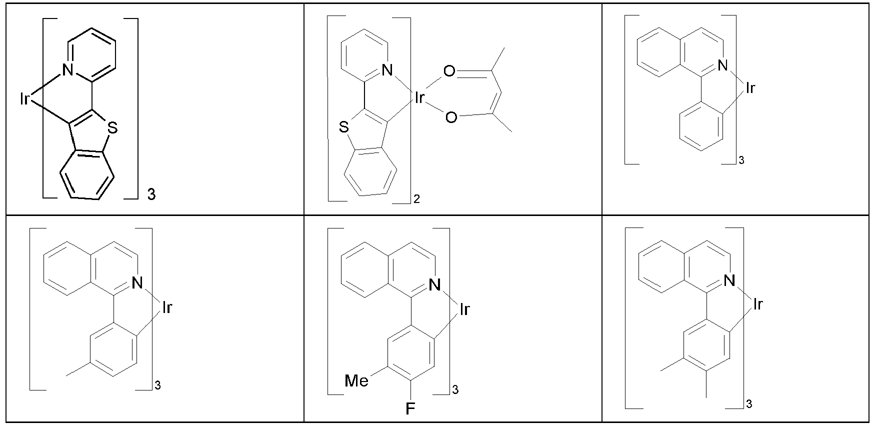

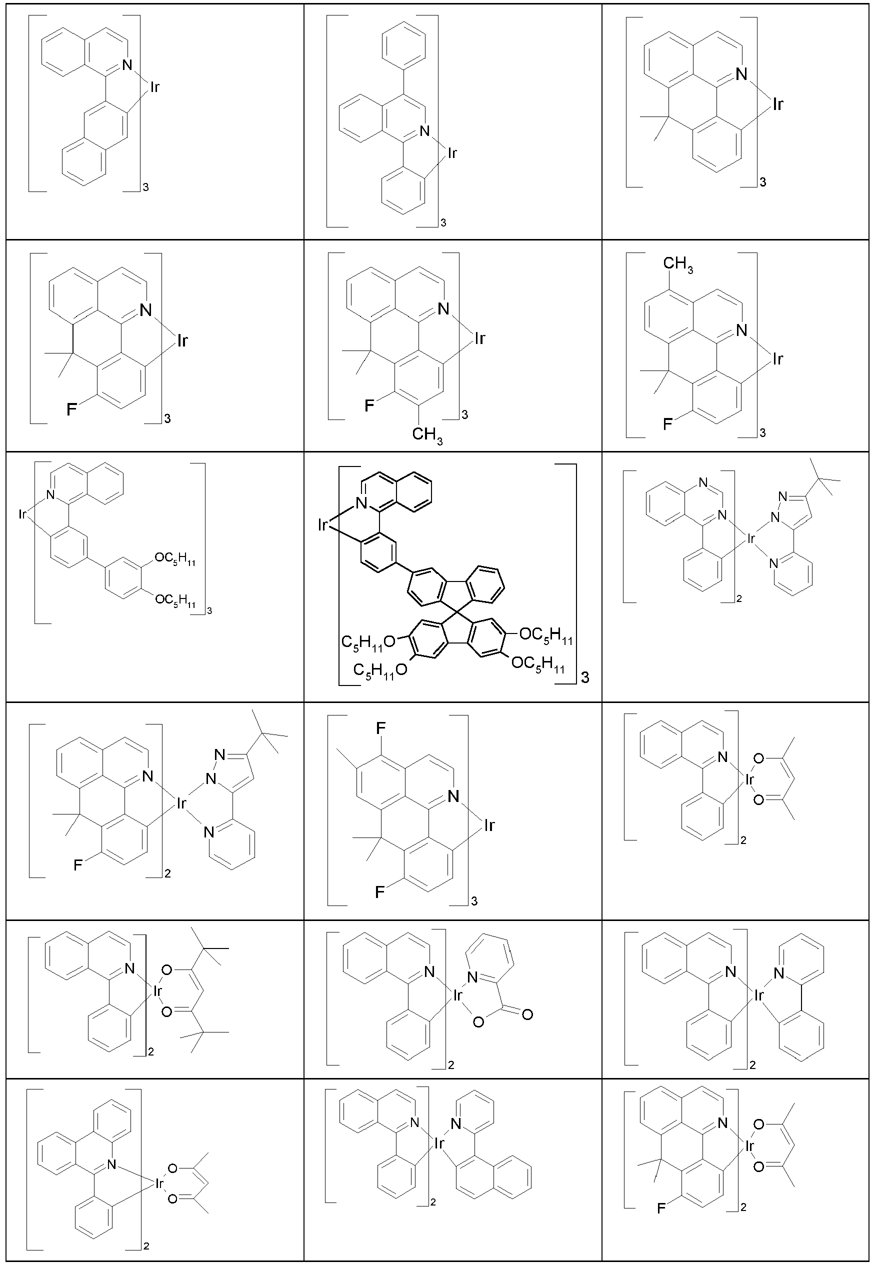

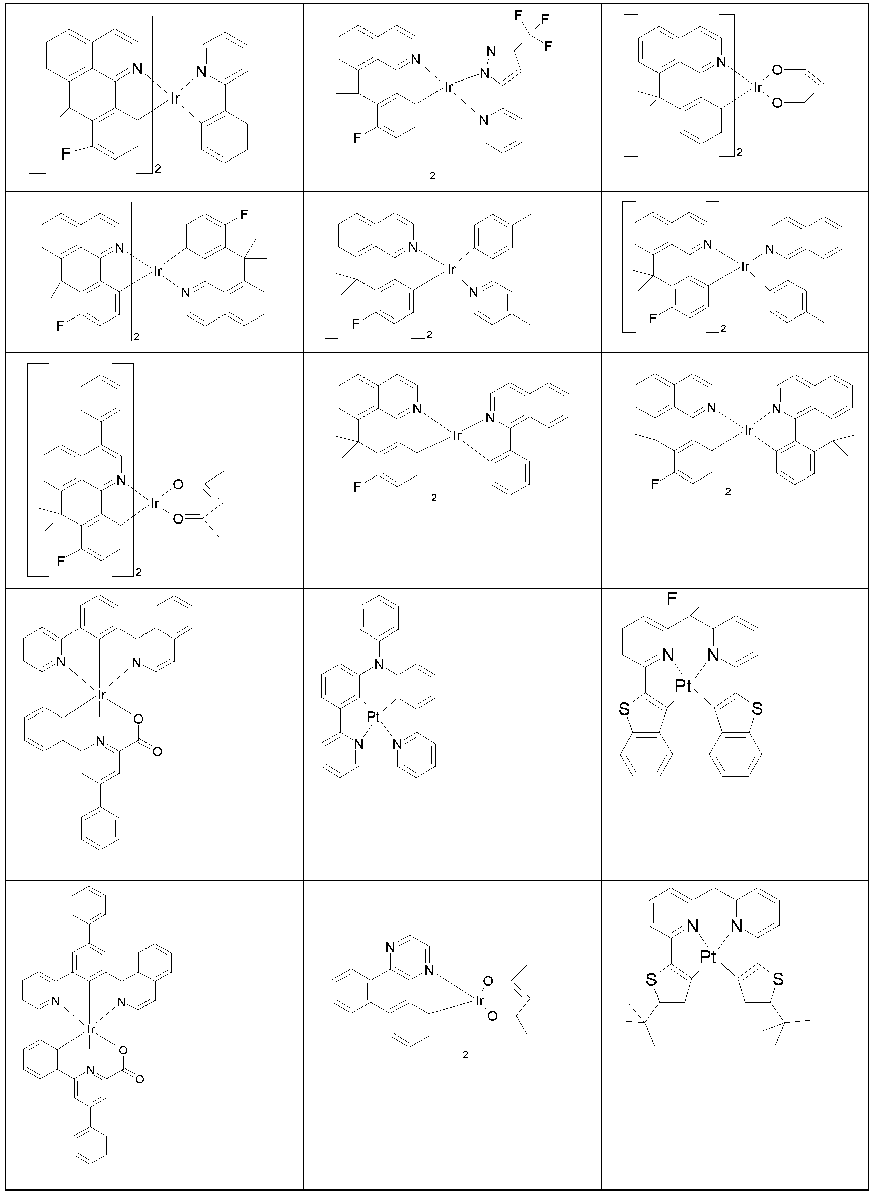

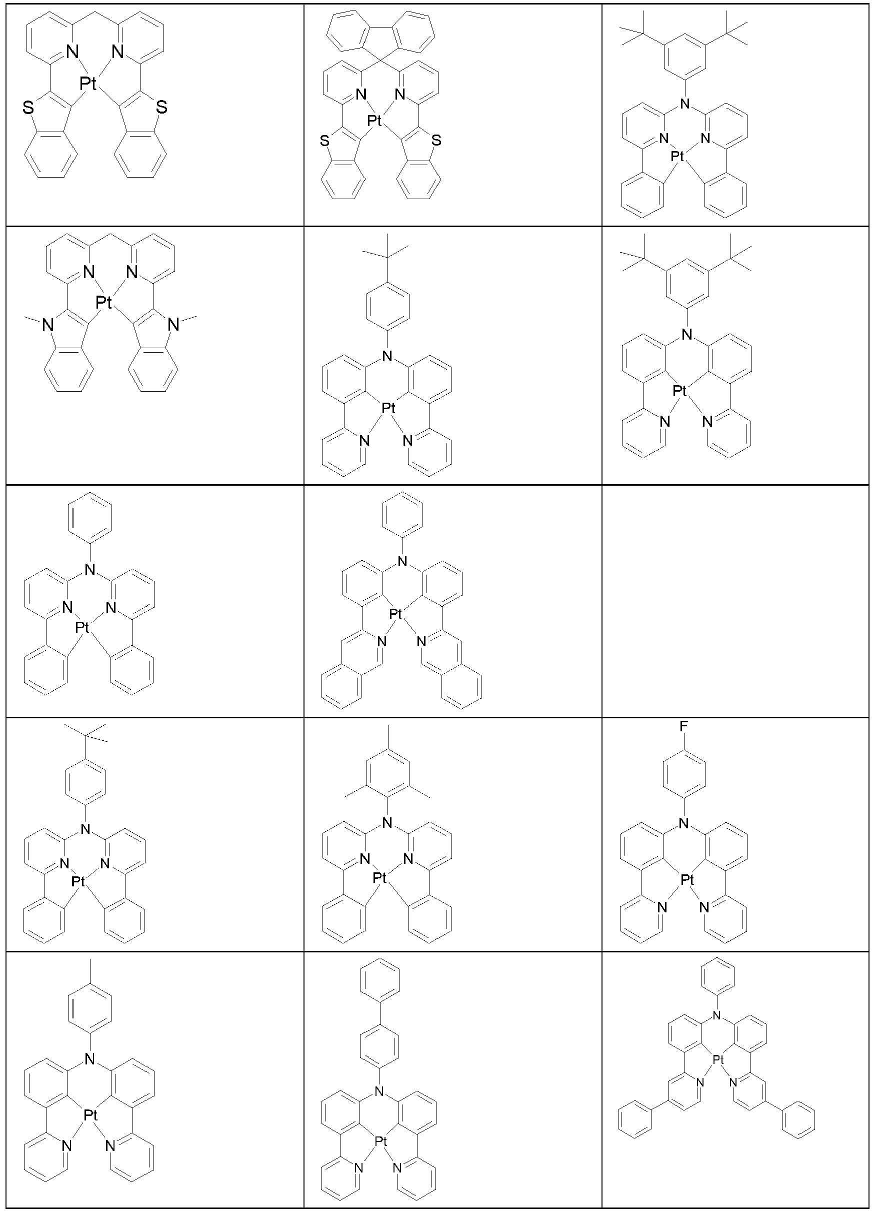

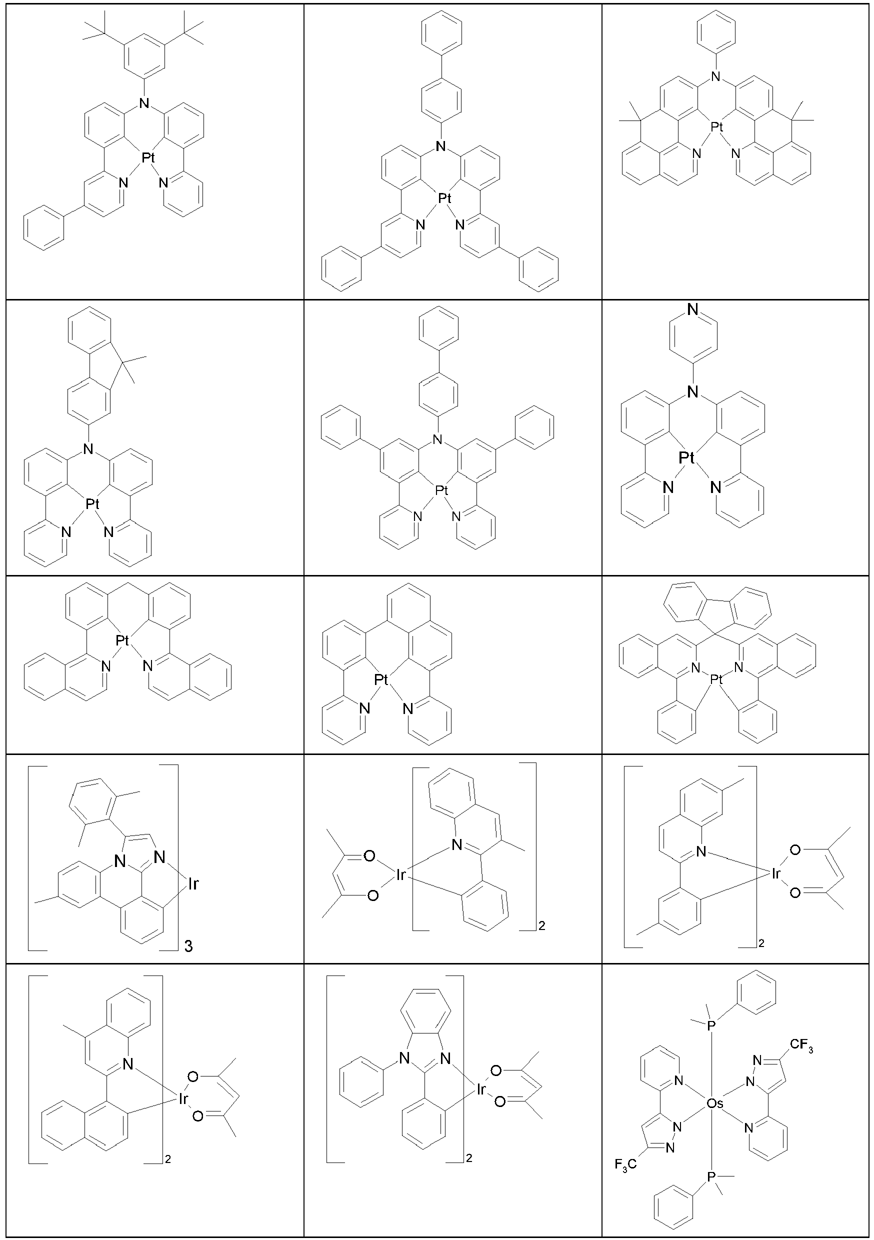

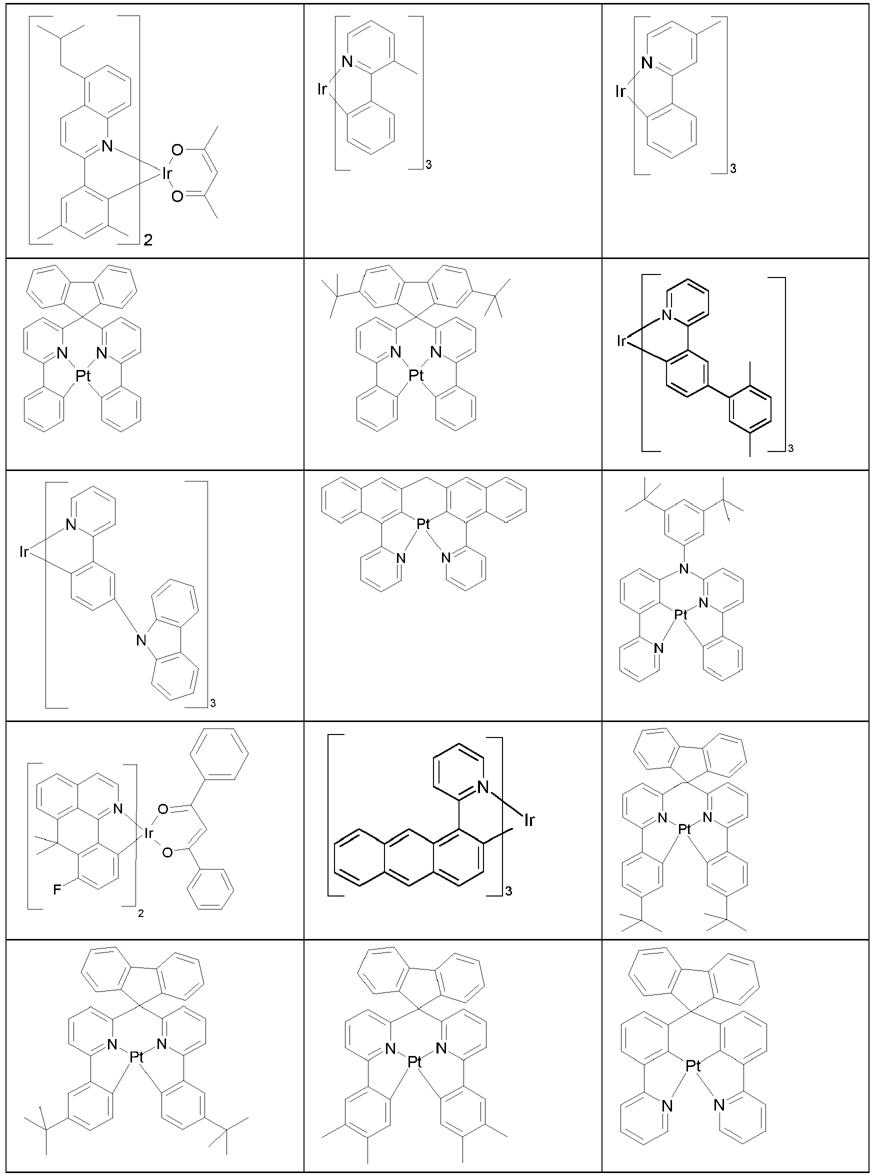

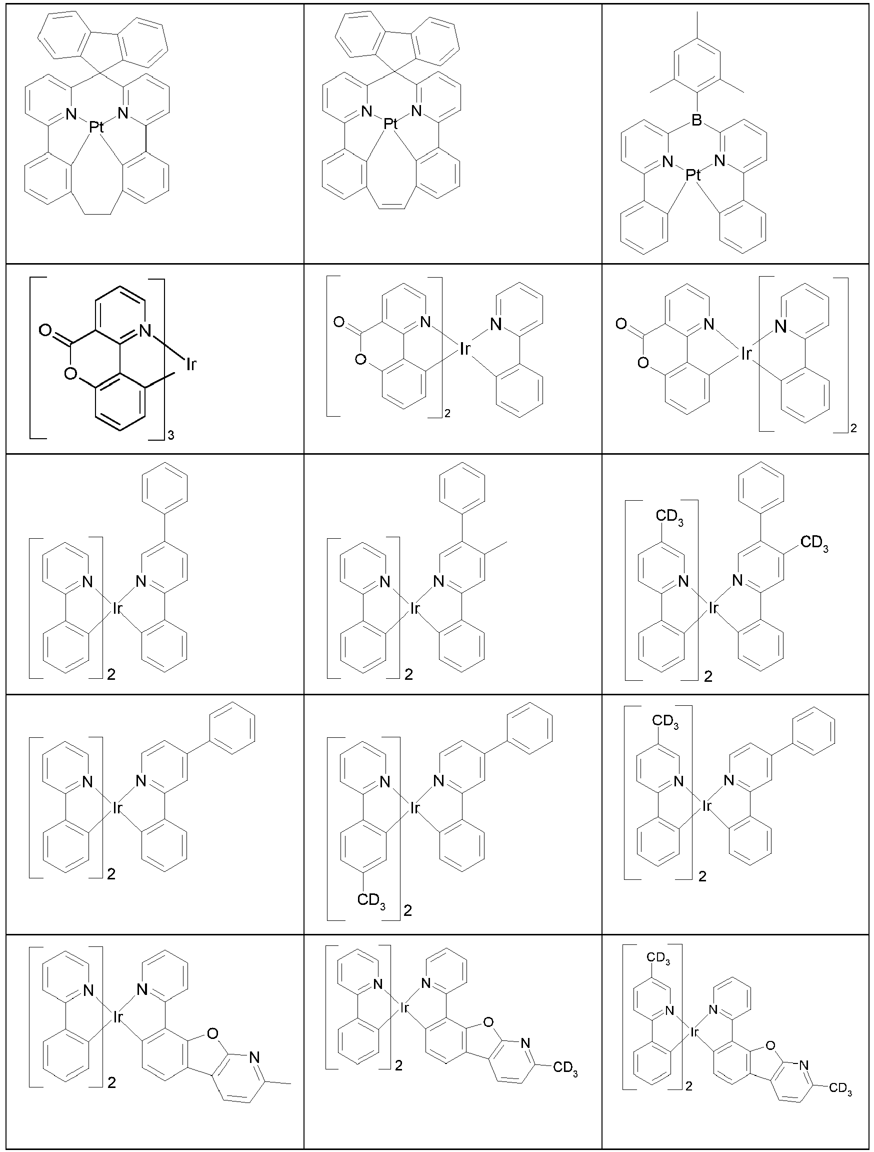

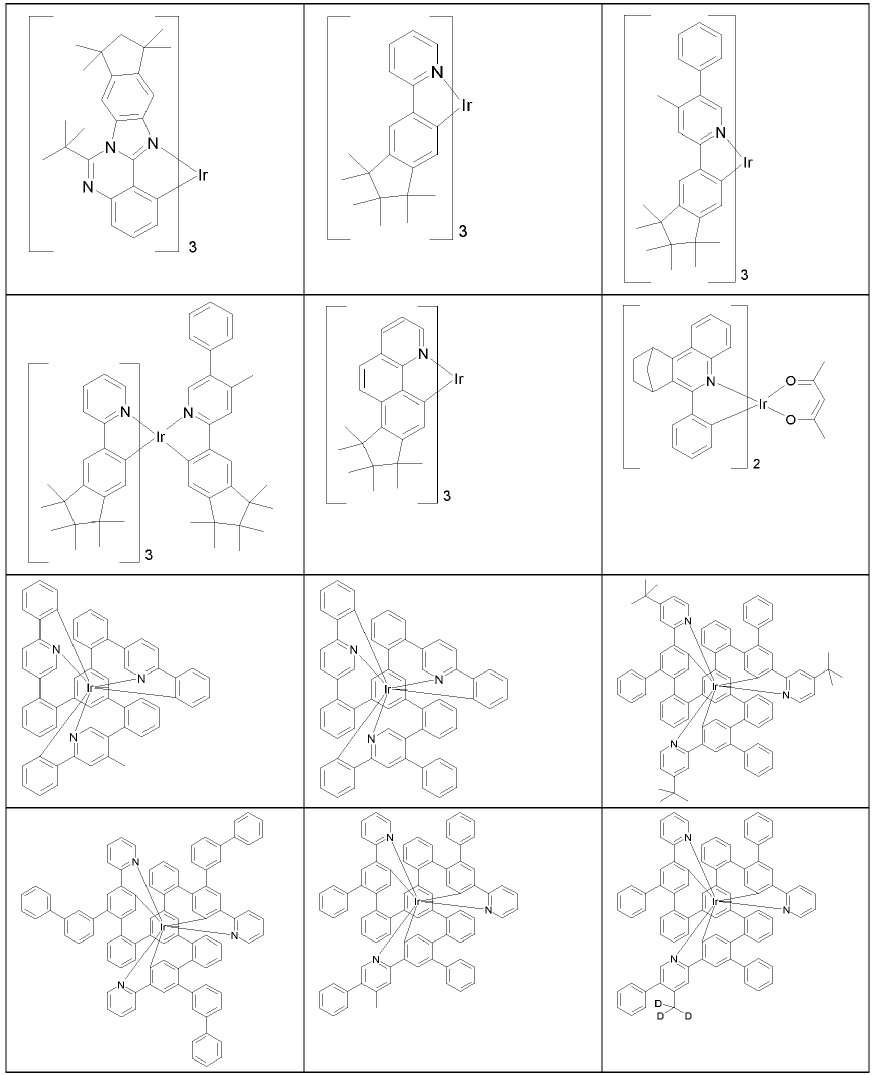

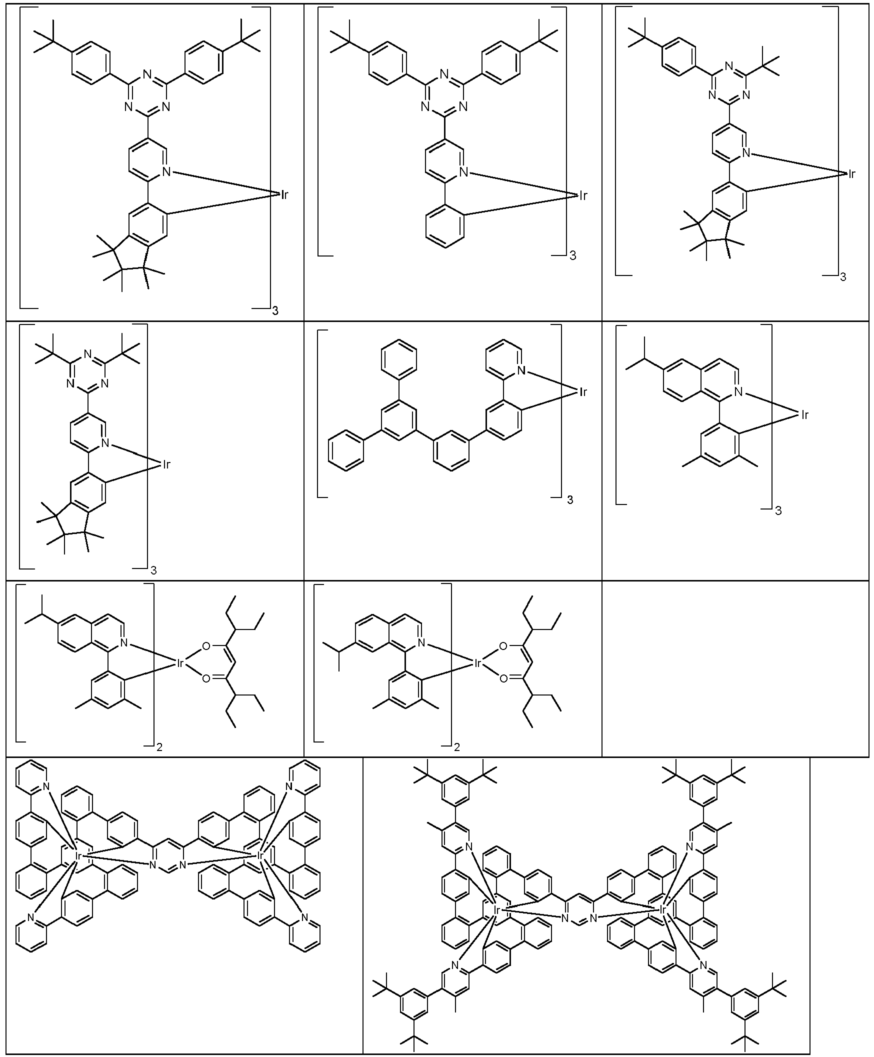

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar aufgedampft, wobei der Druck auch noch geringer sein kann, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spin-Coating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine Verbindung gemäß Formel (1) und einen phosphoreszierenden Dotanden aus Lösung aufzubringen und darauf eine Lochblockierschicht und/oder eine Elektronentransportschicht im Vakuum aufzudampfen. Ebenso kann die emittierende Schicht enthaltend eine Verbindung gemäß Formel (1) und einen phosphoreszierenden Dotanden im Vakuum aufgedampft werden und eine oder mehrere andere Schichten können aus Lösung aufgebracht werden.

Diese Verfahren sind dem Fachmann generell bekannt und können von ihm ohne Probleme auf organische Elektrolumineszenzvorrichtungen enthaltend Verbindungen gemäß Formel (1) bzw. den oben aufgeführten bevorzugten Ausführungsformen angewandt werden.

Die erfindungsgemäßen Verbindungen weisen bei Verwendung in organischen Elektrolumineszenzvorrichtungen die folgenden überraschenden Vorteile gegenüber dem Stand der Technik auf:
1. Die erfindungsgemäßen Verbindungen weisen eine hohe thermische Stabilität und eine geringe Kristallisationstendenz auf. Insbesondere weisen sie eine geringere Kristallisationstendenz auf als entsprechende Fluorenderivate, die keine Gruppe Ar am Fluorengrundkörper enthalten. Dies ermöglicht es, die Schicht nach der Herstellung aus Lösung bei einer höheren Temperatur auszuheizen, was einen wesentlichen technischen Vorteil darstellt. Durch eine höhere Ausheiztemperatur kann eine bessere Deviceperformance erreicht werden, wobei sich dies insbesondere in einer verbesserten Lebensdauer zeigt. Bei Kristallisation der Verbindungen wird generell eine schlechtere Effizienz beobachtet.
2. Die erfindungsgemäßen Verbindungen weisen eine hohe Löslichkeit in gängigen organischen Lösemitteln, beispielsweise Toluol, und sehr gute Filmbildungseigenschaften auf und eignen sich daher besonders gut für die Verarbeitung aus Lösung. Insbesondere zeigen sie eine bessere Löslichkeit im Vergleich zu entsprechenden Fluorenderivaten, die keine Gruppe Ar am Fluorengrundkörper enthalten.
3. Lösungen enthaltend die erfindungsgemäßen Verbindungen weisen eine hervorragende Lösungs-Langzeitstabilität auf. Insbesondere weisen die Lösungen eine verbesserte Langzeitstabilität auf gegenüber Lösungen, welche Fluorenderivate ohne eine Gruppe Ar enthalten.
4. Die mit den erfindungsgemäßen Verbindungen hergestellten OLEDs weisen generell eine hohe Lebensdauer auf. Insbesondere weisen die mit den erfindungsgemäßen Verbindungen hergestellten OLEDs eine bessere Lebensdauer auf als OLEDs, die ein vergleichbares Fluorenderivat in der emittierenden Schicht enthalten, bei dem jedoch die Gruppe Ar in der 2-Position statt in der 4-Position am Fluorengrundkörper gebunden ist.

Die Erfindung wird durch die nachfolgenden Beispiele genauer beschrieben, ohne sie dadurch einschränken zu wollen. Der Fachmann kann, ohne erfinderisch tätig zu werden, weitere erfindungsgemäße Verbindungen herstellen und diese in organischen Elektrolumineszenzvorrichtungen verwenden.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegebenen Nummern sind die entsprechenden CAS-Nummern.

Folgende Bausteine können zur Synthese der erfindungsgemäßen Materialien verwendet werden:

### Boronsäuren

| | | |
|---|---|---|
| 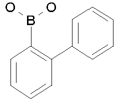 | 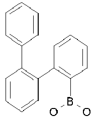 | 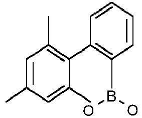 |
| CAS-1817781-37-5 BB-1 | CAS-1310405-29-8 BB-2 | CAS-1265312-48-8 BB-3 |
| 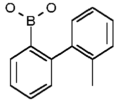 | 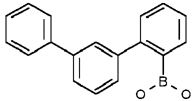 | 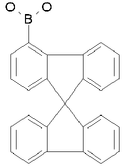 |
| CAS-1228458-44-3 BB-4 | CAS-1133796-50-5 BB-5 | CAS-1421789-05-0 BB-6 |
| 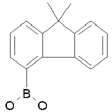 | 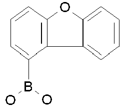 | |
| CAS-1246022-50-3 BB-7 | CAS-162607-19-4 BB-8 | |

### Ortho-Brom-Methylester

| | | |
|---|---|---|
| 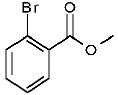 | 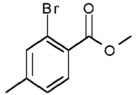 | 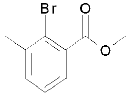 |
| CAS-610-94-6 BB-9 | CAS-87808-49-9 BB-10 | CAS-131001-86-0 BB-11 |
| 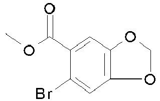 | 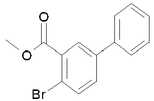 | |
| CAS-61441-09-6 BB-12 | CAS-727408-92-6 BB-13 | |

### Synthesen der erfindungsgemäßen Materialien

### Beispiel 1: Kupplung von ortho-Brombenzoat und [1,1'-biphenyl]-2-boronsäure

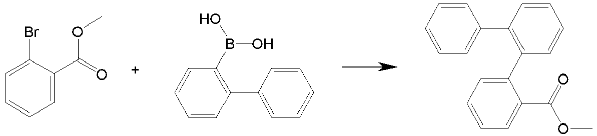

In einen ausgeheizten 1L-4-Halskolben mit KPG-Rührer, Rückflusskühler, Thermometer und Schutzgasanschluss werden 30 g (139,5 mmol) 2-Brombenzoat (BB-9), 33,2 g (167,4 mmol, 1,2 eq.) Biphenyl-2-boronsäure (BB-1), 32,5 g (306,9 mmol, 2,2 eq.) Natriumcarbonat und 1,83 g (6,98 mmol, 0,05 eq.) Triphenylphosphin in 450 ml THF und 150 ml Wasser gelöst und entgast. Zu der Reaktionsmischung werden 0,78 g (3,49 mmol, 0,025 eq.) Palladium(II)acetat zugegeben und die Reaktionsmischung 24 h unter Rückfluss gerührt. Nach Abkühlen auf Raumtemperatur werden 300 ml Ethylacetat zugegeben, die organische Phase abgetrennt und im Vakuum das Lösemittel entfernt. Der Rückstand wird in Ethylacetat aufgenommen, über Kieselgel filtriert und im Vakuum getrocknet. Es werden 33,5 g (116,2 mmol, 83% Ausbeute) des farblosen Feststoffes BB-50 erhalten werden.

Analog dieser Vorschrift können die angegebenen Boronsäuren mit den Ortho-Brombenzoesäure-methylestern mit ähnlichen Ausbeuten gekoppelt werden:

| | | |
|---|---|---|
| 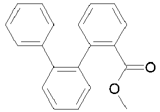 | 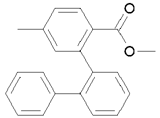 | 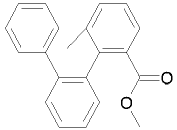 |
| BB-50 Edukte: BB-1 / BB-9 | BB-51 Edukte: BB-1 / BB-10 | BB-52 Edukte: BB-1 / BB-11 |
| 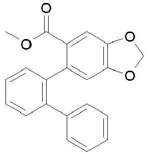 | 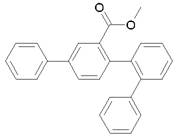 | 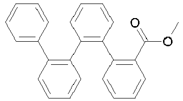 |
| BB-53 Edukte: BB-1 / BB-12 | BB-54 Edukte: BB-1 / BB-13 | BB-55 Edukte: BB-2 / BB-9 |
| 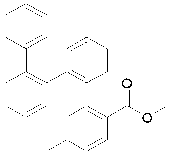 | 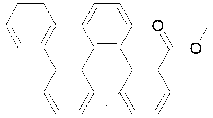 | 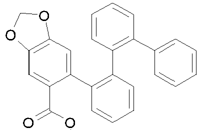 |
| BB-56 Edukte: BB-2 / BB-1 0 | BB-57 Edukte: BB-2 / BB-11 | BB-58 Edukte: BB-2 / BB-12 |
| 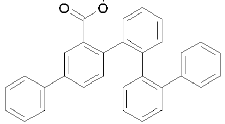 | 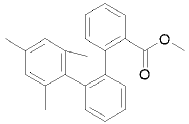 | 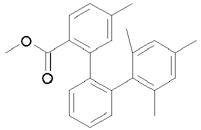 |
| BB-59 Edukte: BB-2 / BB-13 | BB-60 Edukte: BB-3 / BB-9 | BB-61 Edukte: BB-3 / BB-1 0 |
| 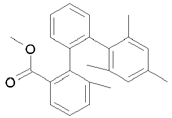 | 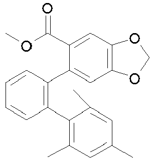 | 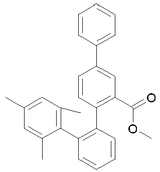 |
| BB-62 Edukte: BB-3 / BB-103 | BB-63 Edukte: BB-3 / BB-12 | BB-64 Edukte: BB-3 / BB-13 |
| 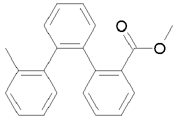 | 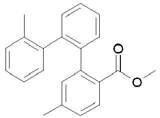 | 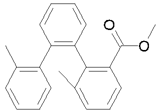 |
| BB-65 Edukte: BB-4 / BB-9 | BB-66 Edukte: BB-4 / BB-10 | BB-67 Edukte: BB-4 / BB-11 |
| 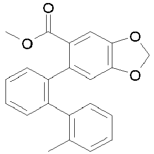 | 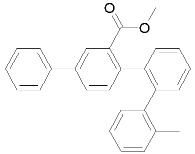 | 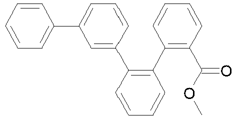 |
| BB-68 Edukte: BB-4 / BB-12 | BB-69 Edukte: BB-4 / BB-13 | BB-70 Edukte: BB-5 / BB-9 |
| 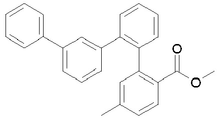 | 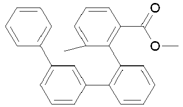 | 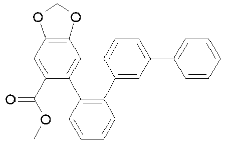 |
| BB-71 Edukte: BB-5 / BB-1 0 | BB-72 Edukte: BB-5 / BB-11 | BB-73 Edukte: BB-5 / BB-12 |
| 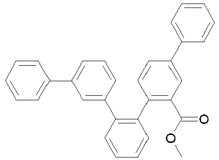 | 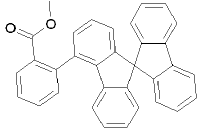 | 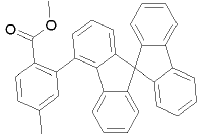 |
| BB-74 Edukte: BB-5 / BB-13 | BB-75 Edukte: BB-6 / BB-9 | BB-76 Edukte: BB-6 / BB-1 0 |
| 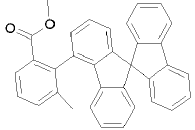 | 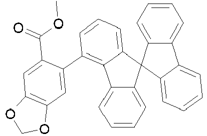 | 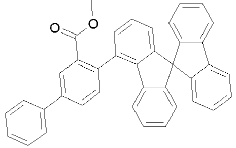 |
| BB-77 Edukte: BB-6 / BB-11 | BB-78 Edukte: BB-6 / BB-12 | BB-79 Edukte: BB-6 / BB-13 |
| 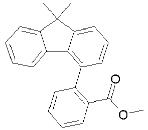 | 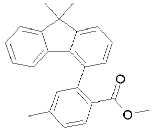 | 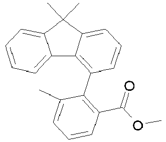 |
| BB-80 Edukte: BB-7 / BB-9 | BB-81 Edukte: BB-7 / BB-1 0 | BB-82 Edukte: BB-7 / BB-11 |
| 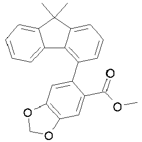 | 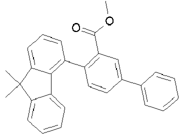 | 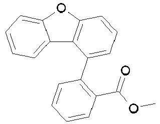 |
| BB-83 Edukte: BB-7 / BB-12 | BB-84 Edukte: BB-7 / BB-13 | BB-85 Edukte: BB-8 / BB-9 |
| 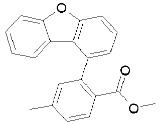 | 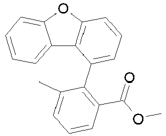 | 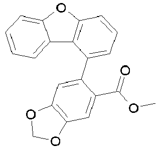 |
| BB-86 Edukte: BB-8 / BB-1 0 | BB-87 Edukte: BB-8 / BB-103 | BB-88 Edukte: BB-8 / BB-12 |
| 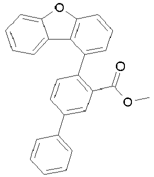 | | |
| BB-89 Edukte: BB-8 / BB-13 | | |

### Beispiel 2: Überführung der Methylesther in 9,9'-Diarylfluorene

### Bausteine:

| | |
|---|---|
| 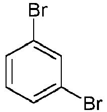 | 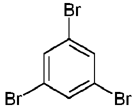 |
| CAS: 108-36-1 BB-90 | CAS: 626-39-1 BB-91 |

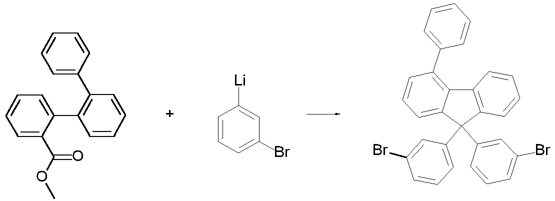

In einen ausgeheizten 1L-4-Halskolben mit KPG-Rührer, Rückflusskühler, Thermometer und Schutzgasanschluss werden 27,65 ml (228,9 mmol) 1,3-Dibrombenzol BB-90 (CAS: 108-36-1) in 450 ml wasserfreiem 2-Methoxy-2-methylpropan gelöst, mit Argon inertisiert und auf -70 °C abgekühlt. Langsam werden 143,7 ml (228,9 mmol, 1 eq.) einer 15 %igen n-Butyllithium-Lösung in n-Hexan so zugetropft, dass die Innentemperatur -65 °C nicht übersteigt. Die Lösung wird für weitere 90 Minuten bei -70 °C gerührt. Dabei entsteht eine weiße Suspension. 30 g (132,9 mmol) [1,1'-2',1"]Terphenyl-2-carbonsäuremethylester (BB-50) werden in 18,3 ml wasserfreiem 2-Methoxy-2-methylpropan gelöst, mit Argon inertisiert und langsam zu der weißen Suspension getropft, so dass die Innentemperatur nicht über -65 °C ansteigt. Die Mischung wird über Nacht langsam auf Raumtemperatur aufgetaut. Es werden zügig 200 ml Wasser zudosiert und für 60 Minuten gerührt. Die wässrige Phase wird abgetrennt und die organische Phase von soviel Lösemittel befreit, dass diese trüb wird. Es werden 300 ml Heptan zugegeben, so dass ein Feststoff aus der Lösung ausfällt. Der Feststoff wird abgesaugt, mit Heptan gewaschen und getrocknet.

Der getrocknete Feststoff wird in einen 4L-4-Halskolben mit Rückflusskühler, KPG-Rührer, Thermometer und Schutzgasanschluss überführt und mit 950 ml 100%iger Essigsäure und 22 ml einer 25%igen Salzsäure versetzt. Die Mischung wird 72 h unter Rückfluss erhitzt, so dass eine braune Lösung entsteht. Nach Abkühlen auf Raumtemperatur werden 1000 ml Wasser zugegeben, so dass die Lösung trüb wird. Das Produkt wird mit Toluol extrahiert. Es werden 30,5 g (55,24 mmol, 53% Ausbeute) des farblosen Feststoffes BB-100 erhalten.

Mit ähnlichen Ausbeuten und gleichen Reaktionsbedingungen können die folgenden Zwischenstufen hergestellt werden:

| | | |
|---|---|---|
| 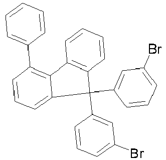 | 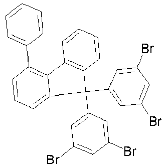 | 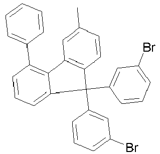 |
| BB-100 Edukte: BB-50+BB-90 | BB-101 Edukte: BB-50+BB-91 | BB-102 Edukte: BB-51+BB-90 |
| 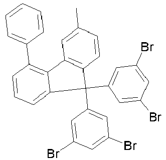 | 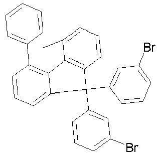 | 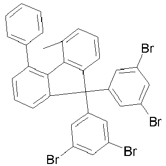 |
| BB-103 Edukte: BB-51+BB-91 | BB-104 Edukte: BB-52+BB-90 | BB-105 Edukte: BB-52+BB-91 |
| 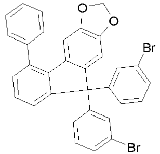 | 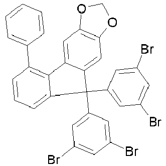 | 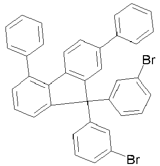 |
| BB-106 Edukte: BB-53+BB-90 | BB-107 Edukte: BB-53+BB-91 | BB-108 Edukte: BB-54+BB-90 |
| 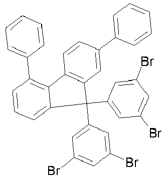 | 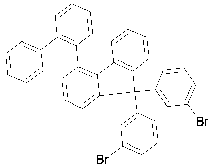 | 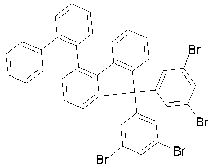 |
| BB-109 Edukte: BB-54+BB-91 | BB-110 Edukte: BB-55+BB-90 | BB-111 Edukte: BB-55+BB-91 |
| 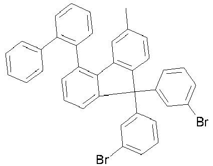 | 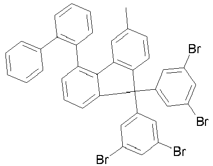 | 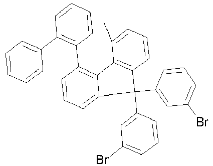 |
| BB-112 Edukte: BB-56+BB-90 | BB-113 Edukte: BB-56+BB-91 | BB-114 Edukte: BB-57+BB-90 |
| 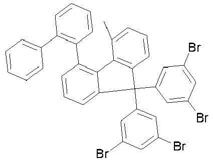 | 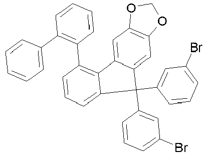 | 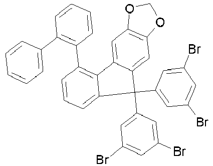 |
| BB-115 Edukte: BB-57+BB-91 | BB-116 Edukte: BB-58+BB-90 | BB-117 Edukte: BB-58+BB-91 |
| 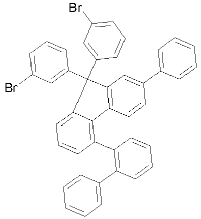 | 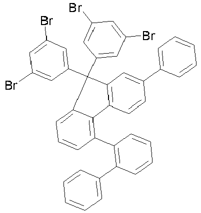 | 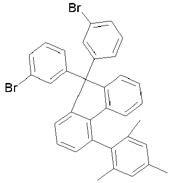 |
| BB-118 Edukte: BB-59+BB-90 | BB-119 Edukte: BB-59+BB-91 | BB-120 Edukte: BB-60+BB-90 |
| 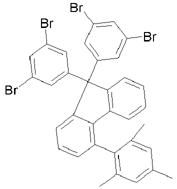 | 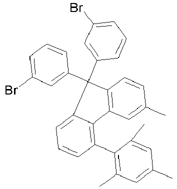 | 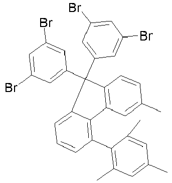 |
| BB-121 Edukte: BB-60+BB-91 | BB-122 Edukte: BB-61+BB-90 | BB-123 Edukte: BB-61+BB-91 |
| 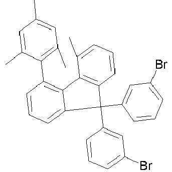 | 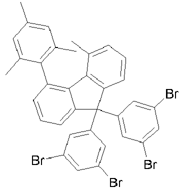 | 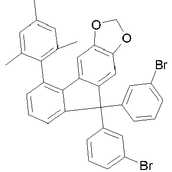 |
| BB-124 Edukte: BB-62+BB-90 | BB-125 Edukte: BB-62+BB-91 | BB-126 Edukte: BB-63+BB-90 |
| 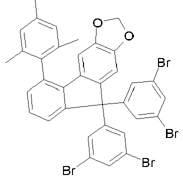 | 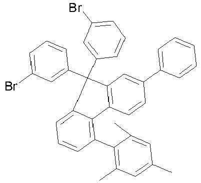 | 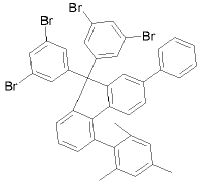 |
| BB-127 Edukte: BB-63+BB-91 | BB-128 Edukte: BB-64+BB-90 | BB-129 Edukte: BB-64+BB-91 |
| 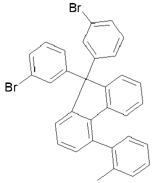 | 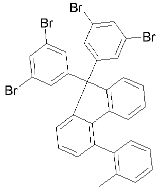 | 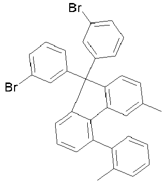 |
| BB-130 Edukte: BB-65+BB-90 | BB-131 Edukte: BB-65+BB-91 | BB-132 Edukte: BB-66+BB-90 |
| 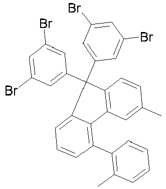 | 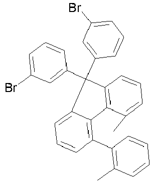 | 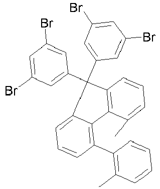 |
| BB-133 Edukte: BB-66+BB-91 | BB-134 Edukte: BB-67+BB-90 | BB-135 Edukte: BB-67+BB-91 |
| 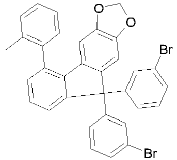 | 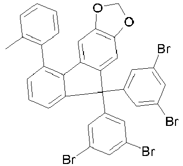 | 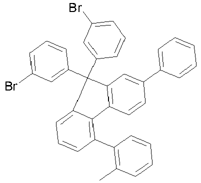 |
| BB-135 Edukte: BB-68+BB-90 | BB-136 Edukte: BB-68+BB-91 | BB-137 Edukte: BB-69+BB-90 |
| 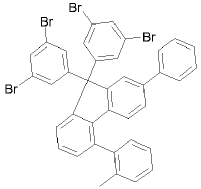 | 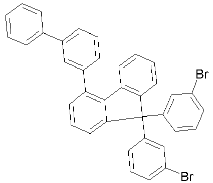 | 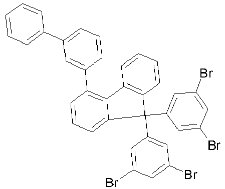 |
| BB-138 Edukte: BB-69+BB-91 | BB-139 Edukte: BB-70+BB-90 | BB-140 Edukte: BB-70+BB-91 |
| 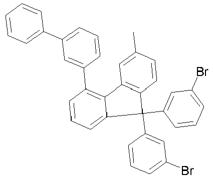 | 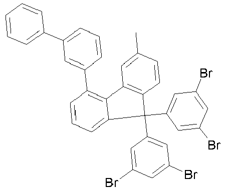 | 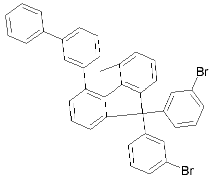 |
| BB-141 Edukte: BB-71+BB-90 | BB-142 Edukte: BB-71+BB-91 | BB-143 Edukte: BB-72+BB-90 |
| 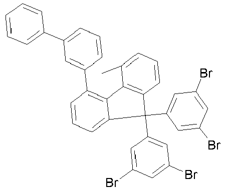 | 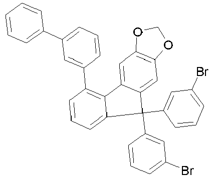 | 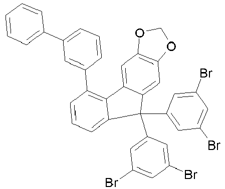 |
| BB-144 Edukte: BB-72+BB-91 | BB-145 Edukte: BB-73+BB-90 | BB-146 Edukte: BB-73+BB-91 |
| 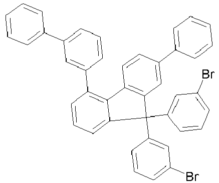 | 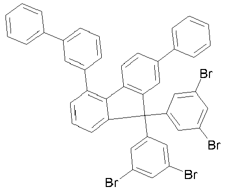 | 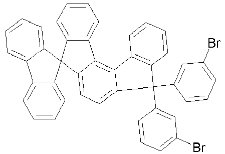 |
| BB-147 Edukte: BB-74+BB-90 | BB-148 Edukte: BB-74+BB-91 | BB-149 Edukte: BB-75+BB-90 |
| 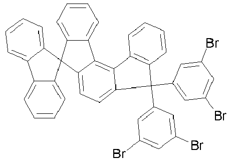 | 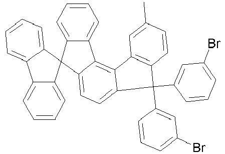 | 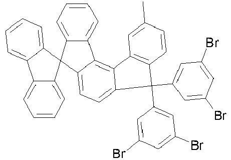 |
| BB-150 Edukte: BB-75+BB-91 | BB-151 Edukte: BB-76+BB-90 | BB-152 Edukte: BB-76+BB-91 |
| 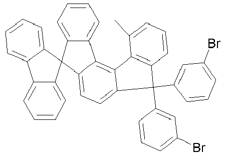 | 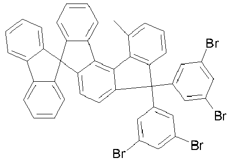 | 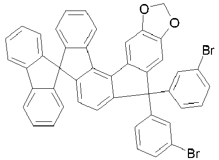 |
| BB-153 Edukte: BB-77+BB-90 | BB-154 Edukte: BB-77+BB-91 | BB-155 Edukte: BB-78+BB-90 |
| 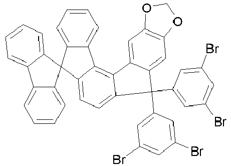 | 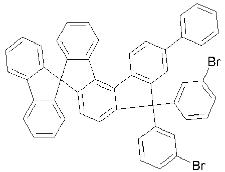 | 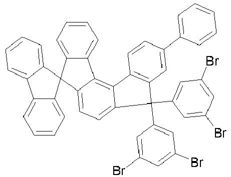 |
| BB-156 Edukte: BB-78+BB-91 | BB-157 Edukte: BB-79+BB-90 | BB-158 Edukte: BB-79+BB-91 |
| 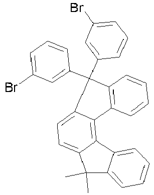 | 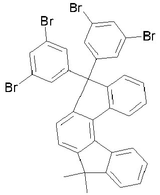 | 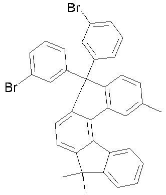 |
| BB-159 Edukte: BB-80+BB-90 | BB-160 Edukte: BB-80+BB-91 | BB-161 Edukte: BB-81+BB-90 |
| 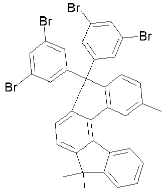 | 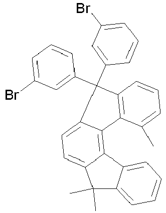 | 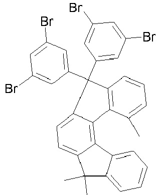 |
| BB-162 Edukte: BB-81+BB-91 | BB-163 Edukte: BB-82+BB-90 | BB-164 Edukte: BB-82+BB-91 |
| 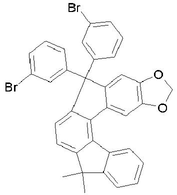 | 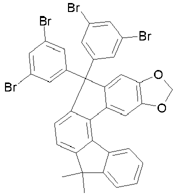 | 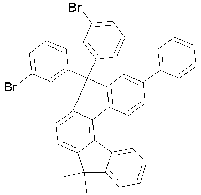 |
| BB-165 Edukte: BB-83+BB-90 | BB-166 Edukte: BB-83+BB-91 | BB-167 Edukte: BB-84+BB-90 |
| 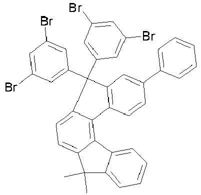 | 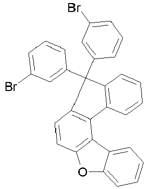 | 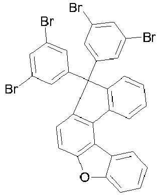 |
| BB-168 Edukte: BB-84+BB-91 | BB-169 Edukte: BB-85+BB-90 | BB-170 Edukte: BB-85+BB-91 |
| 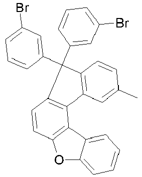 | 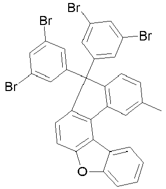 | 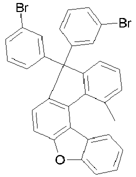 |
| BB-171 Edukte: BB-86+BB-90 | BB-172 Edukte: BB-86+BB-91 | BB-173 Edukte: BB-87+BB-90 |
| 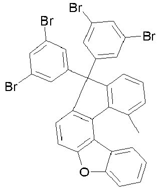 | 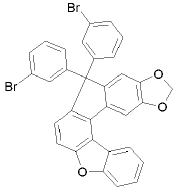 | 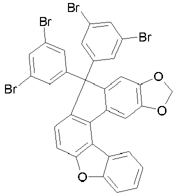 |
| BB-174 Edukte: BB-87+BB-91 | BB-175 Edukte: BB-88+BB-90 | BB-176 Edukte: BB-88+BB-91 |
| 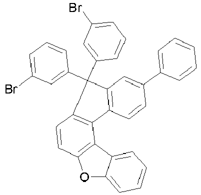 | 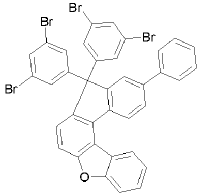 | |
| BB-177 Edukte: BB-89+BB-90 | BB-178 Edukte: BB-89+BB-91 | |

### Beispiel 3: Synthese der erfindungsgemäßen Materialien

Unter anderem können die folgenden Bausteine zur Synthese der erfindungsgemäßen Materialien benutzt werden:

| | | |
|---|---|---|
| 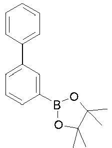 | 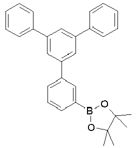 | 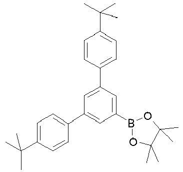 |
| CAS-912844-88-3 BB-179 | CAS-1257248-43-3 BB-180 | CAS-848437-85-4 BB-181 |
| 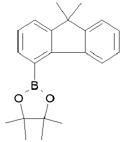 | | |
| CAS-1365692-79-0 BB-182 | | |

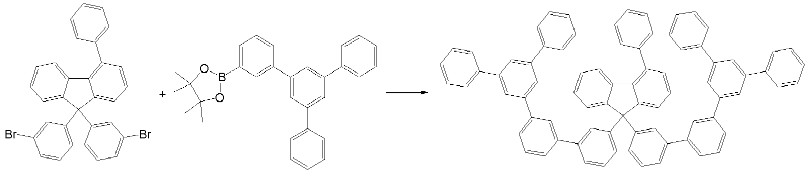

In einem 2L-Mehrhalskolben mit Rückflusskühler, Argonanschluss und KPG-Rührer werden 15 g (27,16 mmol) BB-100, 24,07 g (55,68 mmol, 2,05 eq.) BB-180, 3,14 g (2,72 mmol, 0,1 eq.) Tetrakis(triphenylphosphin)-palladium(0) (CAS: 14221-01-3) eingewogen und inertisiert. Es werden 400 ml Tetrahydrofuran und 85 ml einer 20%igen Tetraethylammoniumhydroxid-Lösung in Wasser zugegeben und erneut inertisiert. Die Reaktionsmischung wird für 24 h unter Rückfluss erhitzt, abgekühlt und mit Wasser versetzt. Die Phasen werden getrennt, die organische Phase mit Wasser extrahiert und über Natriumsulfat getrocknet. Das Lösemittel wird im Vakuum entfernt, der Rückstand in Toluol aufgenommen und über Kieselgel filtriert. Das Lösemittel wird im Vakuum entfernt und der Rückstand im Trockenschrank getrocknet. Der Feststoff wird mehrfach aus Ethylacetat umkristallisiert. Es werden 3,1 g eines farblosen Feststoffes M-0002 (11 % Ausbeute, 3,09 mmol) erhalten.

Weitere erfindungsgemäße Materialien können durch gleiche Reaktionsbedingungen in ähnlichen Ausbeuten erhalten werden:

| | | |
|---|---|---|
| 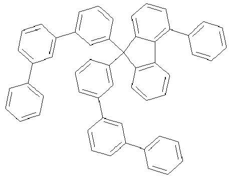 | 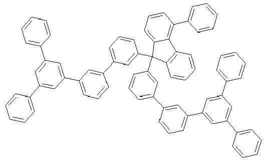 | 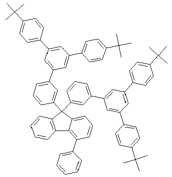 |
| BB-100+BB-179 M-0001 | BB-100+BB-180 M-0002 | BB-100+BB-181 M-0003 |
| 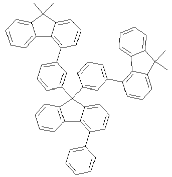 | 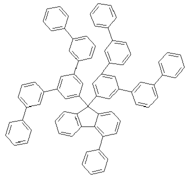 | 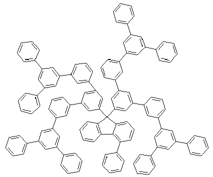 |
| BB-100+BB-182 M-0004 | BB-101+BB-179 M-0005 | BB-101+BB-180 M-00006 |
| 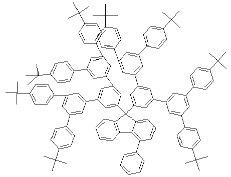 | 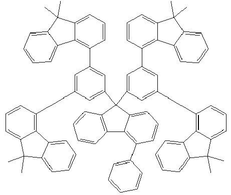 | 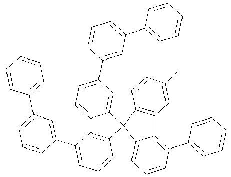 |
| BB-101+BB-181 M-0007 | BB-101+BB-182 M-0008 | BB-102+BB-179 M-0009 |
| 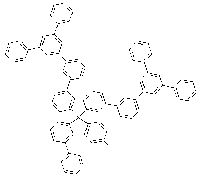 | 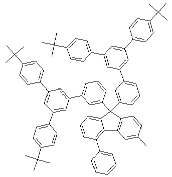 | 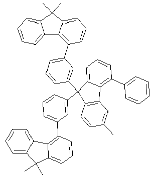 |
| BB-102+BB-180 M-0010 | BB-102+BB-181 M-0011 | BB-102+BB-182 M-0012 |
| 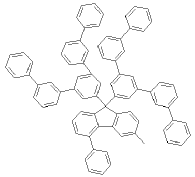 | 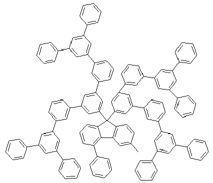 | 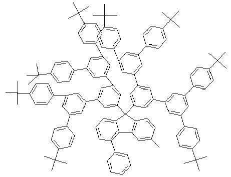 |
| BB-103+BB-179 M-0013 | BB-103+BB-180 M-0014 | BB-103+BB-181 M-0015 |
| 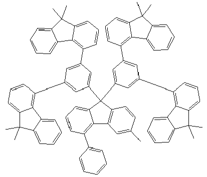 | 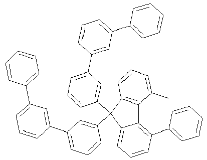 | 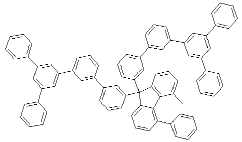 |
| BB-103+BB-182 M-0016 | BB-104+BB-179 M-0017 | BB-104+BB-180 M-0018 |
| 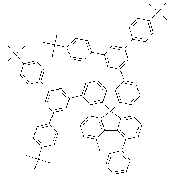 | 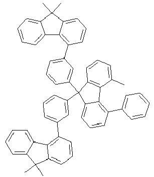 | 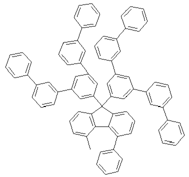 |
| BB-104+BB-181 M-0019 | BB-104+BB-182 M-0020 | BB-105+BB-179 M-0021 |
| 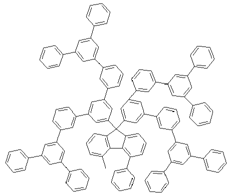 | 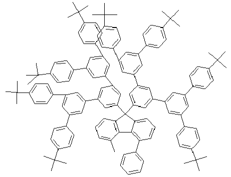 | 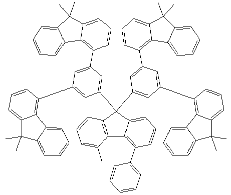 |
| BB-105+BB-180 M-0022 | BB-105+BB-181 M-0023 | BB-105+BB-182 M-0024 |
| 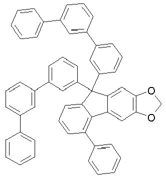 | 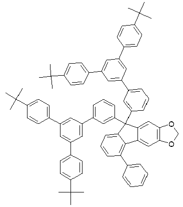 | 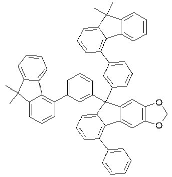 |
| BB-106+BB-179 M-0025 | BB-106+BB-181 M-0026 | BB-106+BB-182 M-0027 |
| 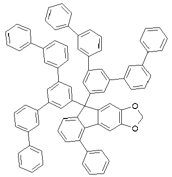 | 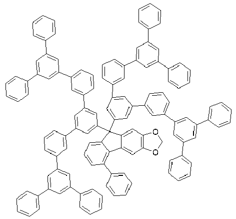 | 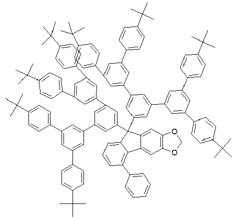 |
| BB-107+BB-179 M-0028 | BB-107+BB-180 M-0029 | BB-107+BB-181 M-0030 |
| 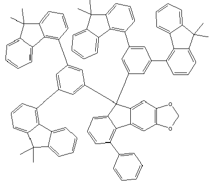 | 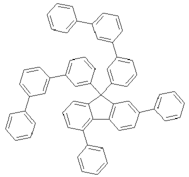 | 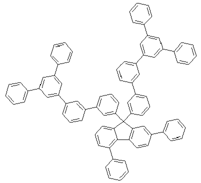 |
| BB-107+BB-182 M-0031 | BB-108+BB-179 M-0032 | BB-108+BB-180 M-0033 |
| 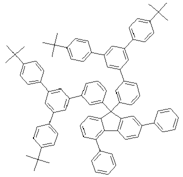 | 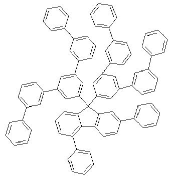 | 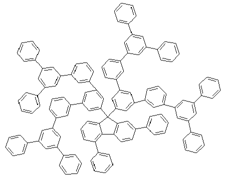 |
| BB-108+BB-181 M-0034 | BB-109+BB-179 M-0035 | BB-109+BB-180 M-0036 |
| 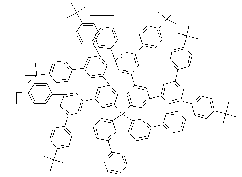 | 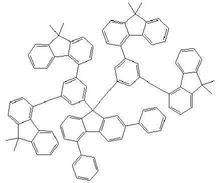 | 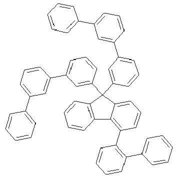 |
| BB-109+BB-181 M-0037 | BB-109+BB-182 M-0038 | BB-110+BB-179 M-0039 |
| 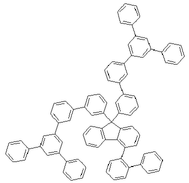 | 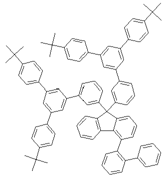 | 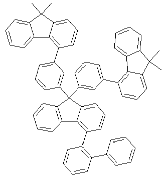 |
| BB-110+BB-180 M-0040 | BB-110+BB-181 M-0041 | BB-110+BB-182 M-0042 |
| 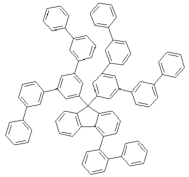 | 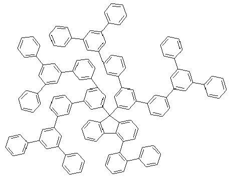 | 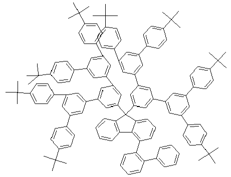 |
| BB-111+BB-179 M-0043 | BB-111+BB-180 M-0044 | BB-111+BB-181 M-0045 |
| 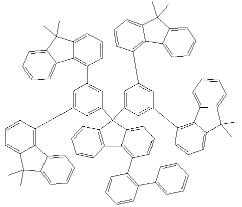 | 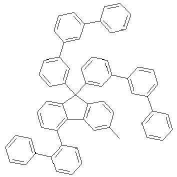 | 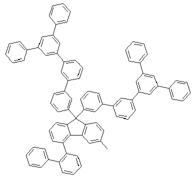 |
| BB-111+BB-182 M-0046 | BB-112+BB-179 M-0047 | BB-112+BB-180 M-0048 |
| 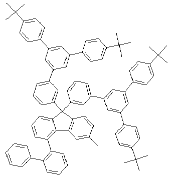 | 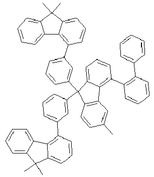 | 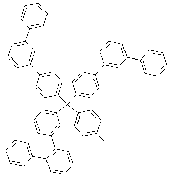 |
| BB-112+BB-181 M-0049 | BB-112+BB-182 M-0050 | BB-2038+BB-179 M-0051 |
| 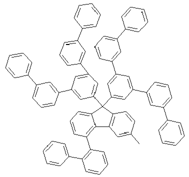 | 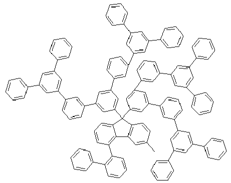 | 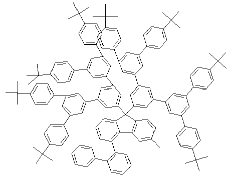 |
| BB-113+BB-179 M-0052 | BB-113+BB-180 M-0053 | BB-113+BB-181 M-0054 |
| 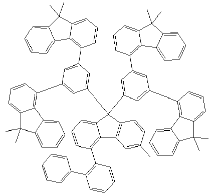 | 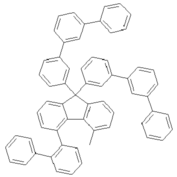 | 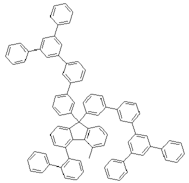 |
| BB-113+BB-182 M-0055 | BB-114+BB-179 M-0056 | BB-114+BB-180 M-0057 |
| 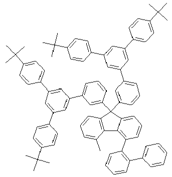 | 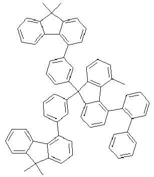 | 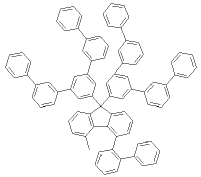 |
| BB-114+BB-181 M-0058 | BB-114+BB-182 M-0059 | BB-115+BB-179 M-0060 |
| 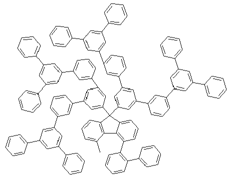 | 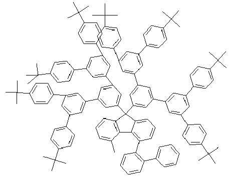 | 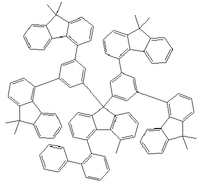 |
| BB-115+BB-180 M-0061 | BB-115+BB-181 M-0062 | BB-115+BB-182 M-0063 |
| 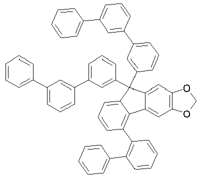 | 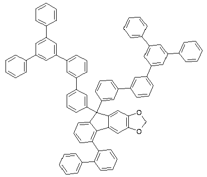 | 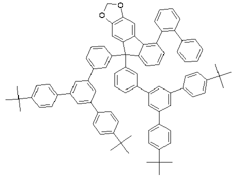 |
| BB-116+BB-179 M-0064 | BB-116+BB-180 M-0065 | BB-116+BB-181 M-0066 |
| 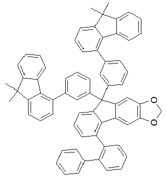 | 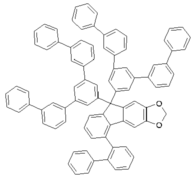 | 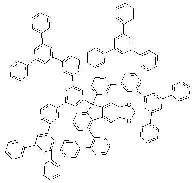 |
| BB-116+BB-182 M-0067 | BB-117+BB-179 M-0068 | BB-117+BB-180 M-0069 |
| 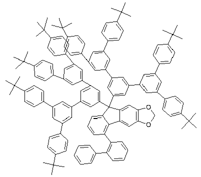 | 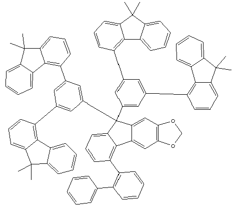 | 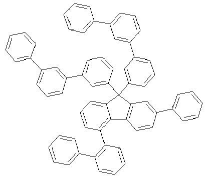 |
| BB-117+BB-181 M-0070 | BB-117+BB-182 M-0071 | BB-118+BB-179 M-0072 |
| 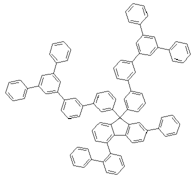 | 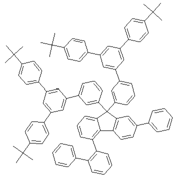 | 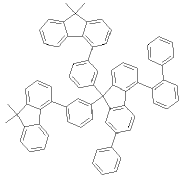 |
| BB-118+BB-180 M-0073 | BB-118+BB-181 M-0074 | BB-118+BB-182 M-0075 |
| 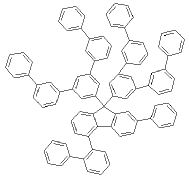 | 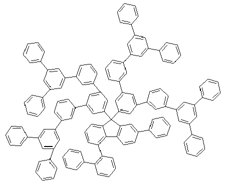 | 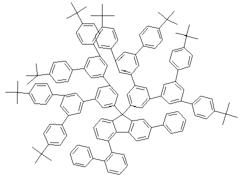 |
| BB-119+BB-179 M-0076 | BB-119+BB-180 M-0077 | BB-119+BB-181 M-0078 |
| 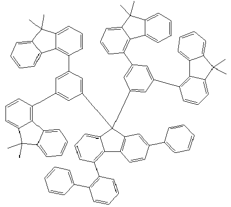 | 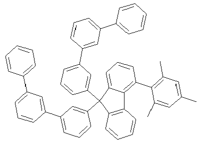 | 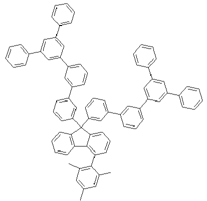 |
| BB-119+BB-182 M-0079 | BB-120+BB-179 M-0080 | BB-120+BB-180 M-0081 |
| 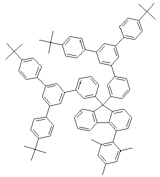 | 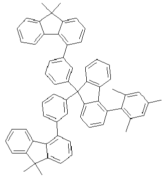 | 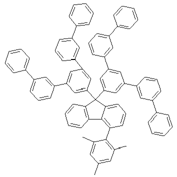 |
| BB-120+BB-181 M-0082 | BB-120+BB-182 M-0083 | BB-121+BB-179 M-0084 |
| 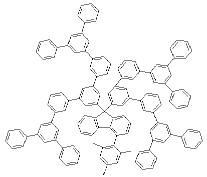 | 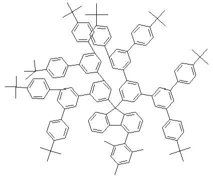 | 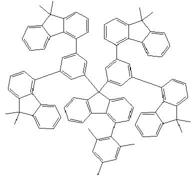 |
| BB-121+BB-180 M-0085 | BB-121+BB-181 M-0086 | BB-121+BB-182 M-0087 |
| 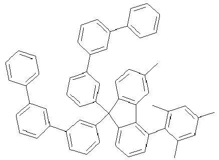 | 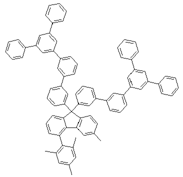 | 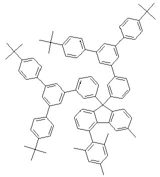 |
| BB-122+BB-179 M-0088 | BB-122+BB-180 M-0089 | BB-122+BB-181 M-0090 |
| 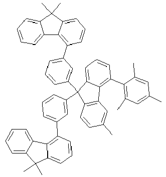 | | 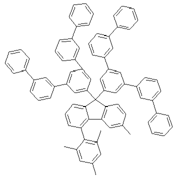 |
| BB-122+BB-182 M-0091 | | BB-123+BB-179 M-0092 |
| 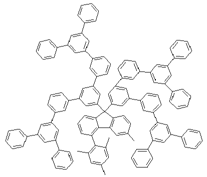 | 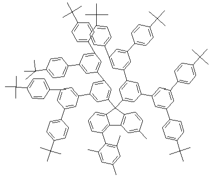 | 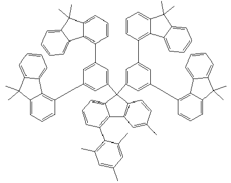 |
| BB-123+BB-180 M-0093 | BB-123+BB-181 M-0094 | BB-123+BB-182 M-0095 |
| 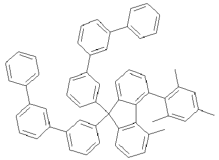 | 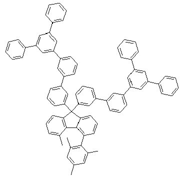 | 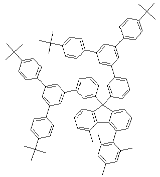 |
| BB-124+BB-179 M-0096 | BB-124+BB-180 M-0097 | BB-124+BB-181 M-0098 |
| 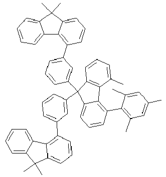 | 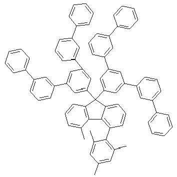 | 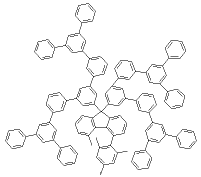 |
| BB-124+BB-182 M-0099 | BB-125+BB-179 M-0100 | BB-125+BB-180 M-0101 |
| 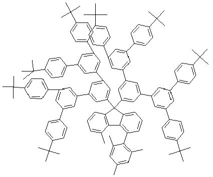 | 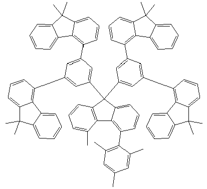 | 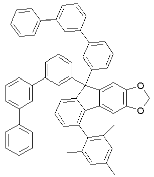 |
| BB-125+BB-181 M-0102 | BB-125+BB-182 M-0103 | BB-126+BB-179 M-0104 |
| 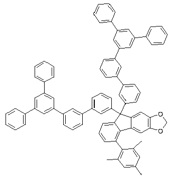 | 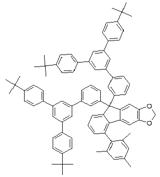 | 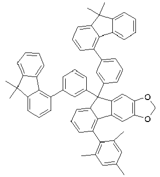 |
| BB-126+BB-180 M-0105 | BB-126+BB-181 M-0106 | BB-126+BB-182 M-0107 |
| 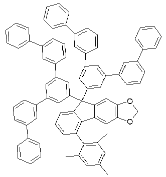 | 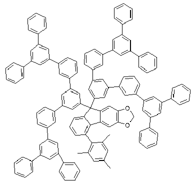 | 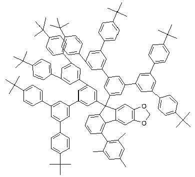 |
| BB-127+BB-179 M-0108 | BB-127+BB-180 M-0109 | BB-127+BB-181 M-0110 |
| 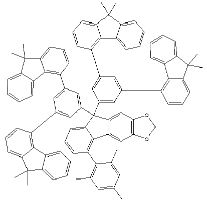 | 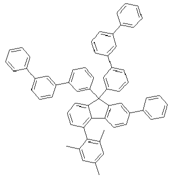 | 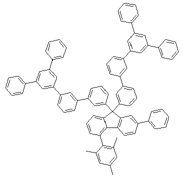 |
| BB-127+BB-182 M-0111 | BB-128+BB-179 M-0112 | BB-128+BB-180 M-0113 |
| 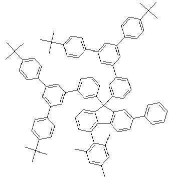 | 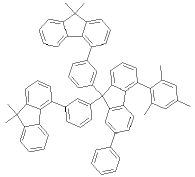 | 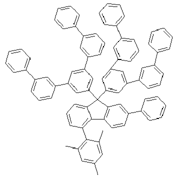 |
| BB-128+BB-181 M-0114 | BB-128+BB-182 M-0115 | BB-129+BB-179 M-0116 |
| 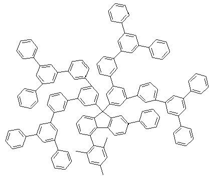 | 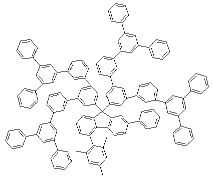 | 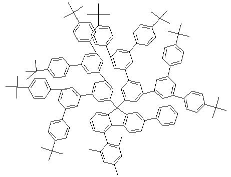 |
| BB-129+BB-180 M-0117 | BB-129+BB-180 M-0118 | BB-129+BB-181 M-0119 |
| 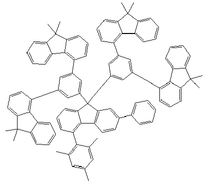 | 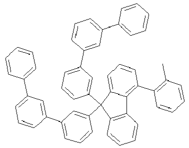 | 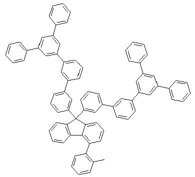 |
| BB-129+BB-182 M-0120 | BB-130+BB-179 M-0121 | BB-130+BB-180 M-0122 |
| 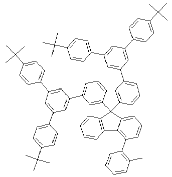 | 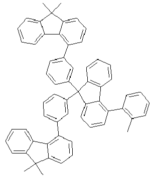 | 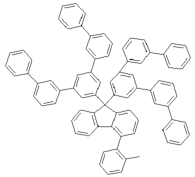 |
| BB-130+BB-181 M-0123 | BB-130+BB-182 M-0124 | BB-131+BB-179 M-0125 |
| 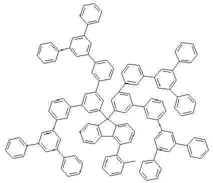 | 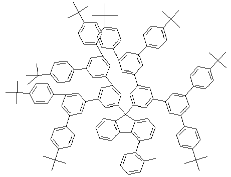 | 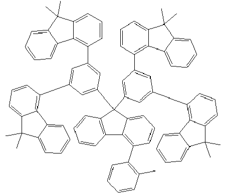 |
| BB-131+BB-180 M-0126 | BB-131+BB-181 M-0127 | BB-131+BB-182 M-0128 |
| 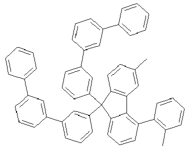 | 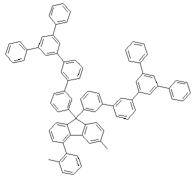 | 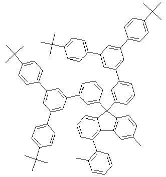 |
| BB-132+BB-179 M-0129 | BB-132+BB-180 M-0130 | BB-132+BB-181 M-0131 |
| 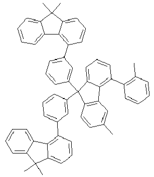 | 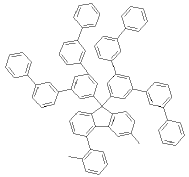 | 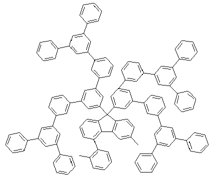 |
| BB-132+BB-182 M-0132 | BB-133+BB-179 M-0133 | BB-133+BB-180 M-0134 |
| 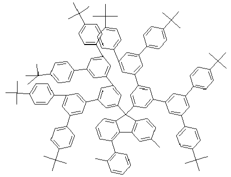 | 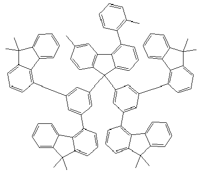 | 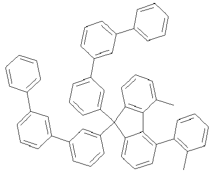 |
| BB-133+BB-181 M-0135 | BB-133+BB-182 M-0136 | BB-134+BB-179 M-0137 |
| 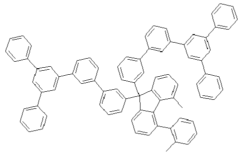 | 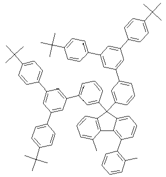 | 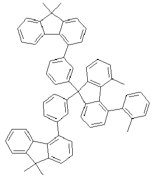 |
| BB-134+BB-180 M-0138 | BB-134+BB-181 M-0139 | BB-134+BB-182 M-0140 |
| 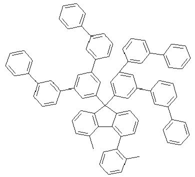 | 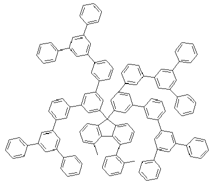 | 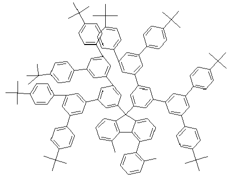 |
| BB-135+BB-179 M-0141 | BB-135+BB-180 M-0142 | BB-135+BB-181 M-0143 |
| 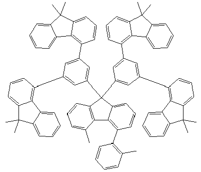 | 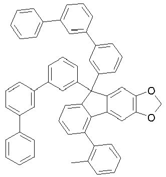 | 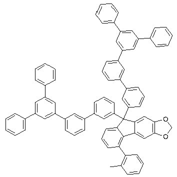 |
| BB-135+BB-182 M-0144 | BB-135+BB-179 M-0145 | BB-135+BB-180 M-0146 |
| 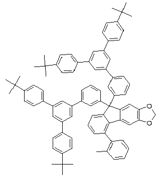 | 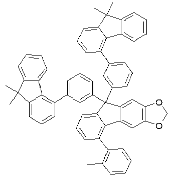 | 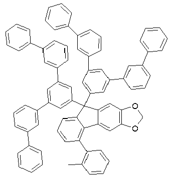 |
| BB-135+BB-181 M-0147 | BB-135+BB-182 M-0148 | BB-136+BB-179 M-0149 |
| 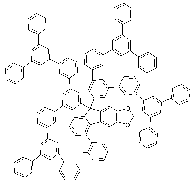 | 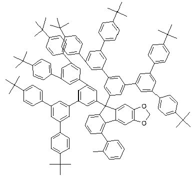 | 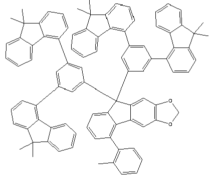 |
| BB-136+BB-180 M-0150 | BB-136+BB-181 M-0151 | BB-136+BB-182 M-0152 |
| 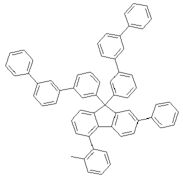 | 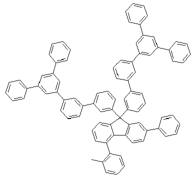 | 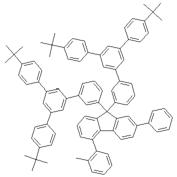 |
| BB-137+BB-179 M-0153 | BB-137+BB-180 M-0154 | BB-137+BB-181 M-0155 |
| 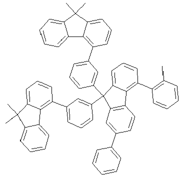 | 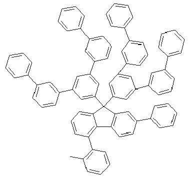 | 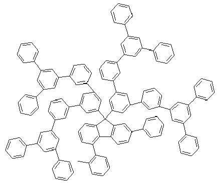 |
| BB-137+BB-182 M-0156 | BB-138+BB-179 M-0157 | BB-138+BB-180 M-0158 |
| 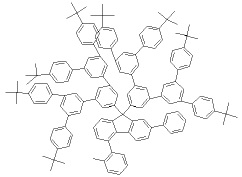 | 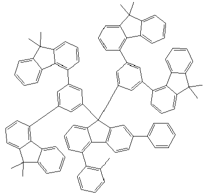 | 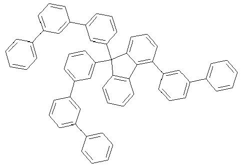 |
| BB-138+BB-181 M-0159 | BB-138+BB-182 M-0160 | BB-139+BB-179 M-0161 |
| 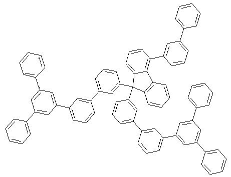 | 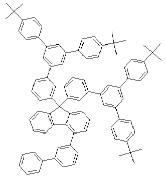 | 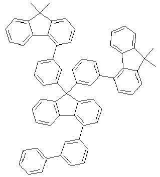 |
| BB-139+BB-180 M-0162 | BB-139+BB-181 M-0163 | BB-139+BB-182 M-0164 |
| 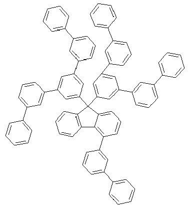 | 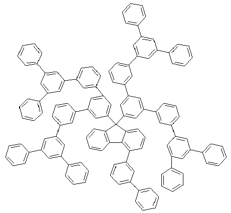 | 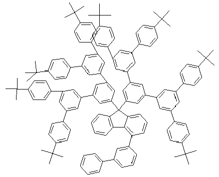 |
| BB-140+BB-179 M-0165 | BB-140+BB-180 M-0166 | BB-140+BB-181 M-0167 |
| 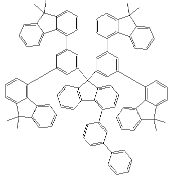 | 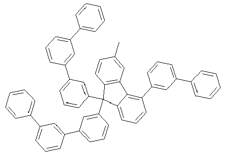 | 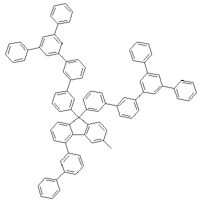 |
| BB-140+BB-182 M-0168 | BB-141+BB-179 M-0169 | BB-141+BB-180 M-0170 |
| 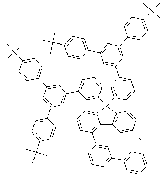 | 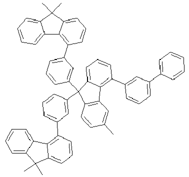 | 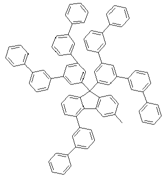 |
| BB-141+BB-181 M-0171 | BB-141+BB-182 M-0172 | BB-142+BB-179 M-0173 |
| 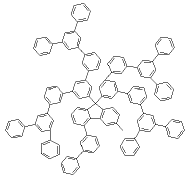 | 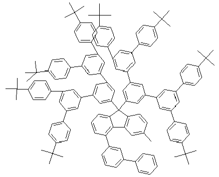 | 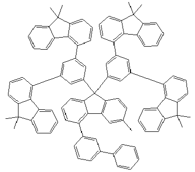 |
| BB-142+BB-180 M-0174 | BB-142+BB-181 M-0175 | BB-142+BB-182 M-0176 |
| 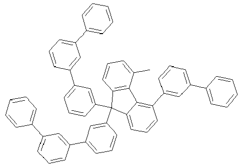 | 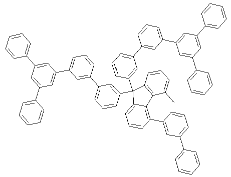 | 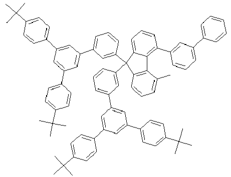 |
| BB-143+BB-179 M-0177 | BB-143+BB-180 M-0178 | BB-143+BB-181 M-0179 |
| 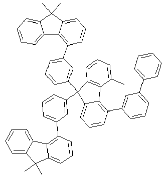 | 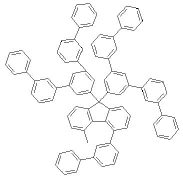 | 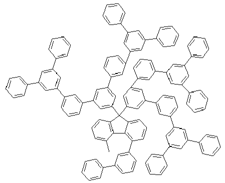 |
| BB-143+BB-182 M-0180 | BB-144+BB-179 M-0181 | BB-144+BB-180 M-0182 |
| 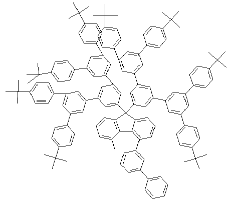 | 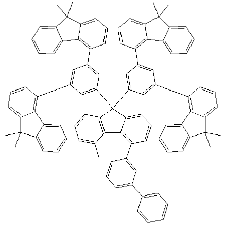 | 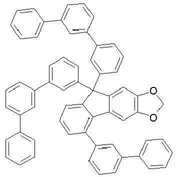 |
| BB-144+BB-181 M-0183 | BB-144+BB-182 M-0184 | BB-145+BB-179 M-0185 |
| 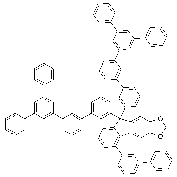 | 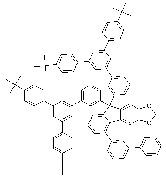 | 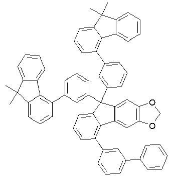 |
| BB-145+BB-180 M-0186 | BB-145+BB-181 M-0187 | BB-145+BB-182 M-0188 |
| 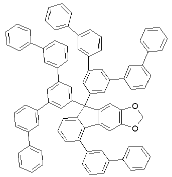 | 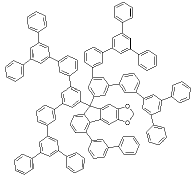 | 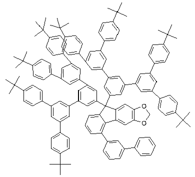 |
| BB-146+BB-179 M-0189 | BB-146+BB-180 M-0190 | BB-146+BB-181 M-0191 |
| 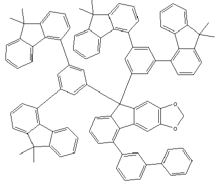 | 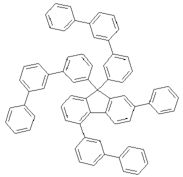 | 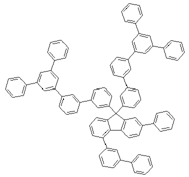 |
| BB-146+BB-182 M-0192 | BB-147+BB-179 M-0193 | BB-147+BB-180 M-0194 |
| 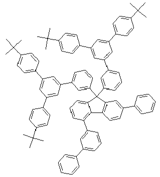 | 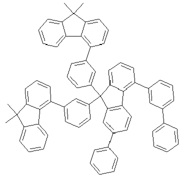 | 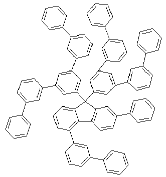 |
| BB-147+BB-181 M-0195 | BB-147+BB-182 M-0196 | BB-148+BB-179 M-0197 |
| 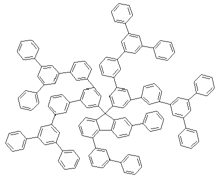 | 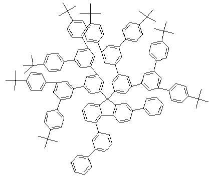 | 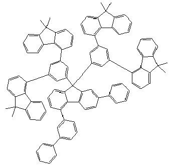 |
| BB-148+BB-180 M-0198 | BB-148+BB-181 M-0199 | BB-148+BB-182 M-0200 |
| 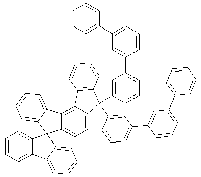 | 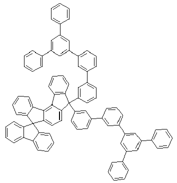 | 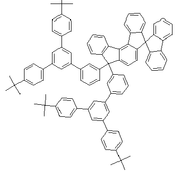 |
| BB-149+BB-179 M-0201 | BB-149+BB-180 M-0202 | BB-149+BB-181 M-0203 |
| 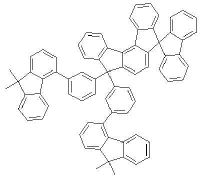 | 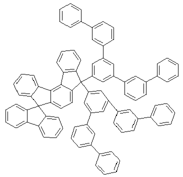 | 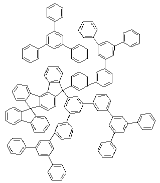 |
| BB-149+BB-182 M-0204 | BB-150+BB-179 M-0205 | BB-150+BB-180 M-0206 |
| 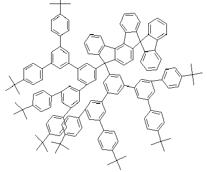 | 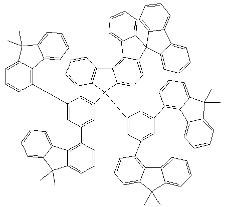 | 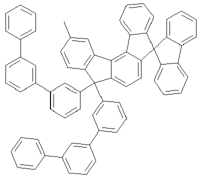 |
| BB-150+BB-181 M-0207 | BB-150+BB-182 M-0208 | BB-151+BB-179 M-0209 |
| 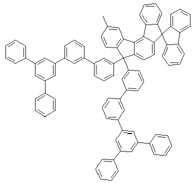 | 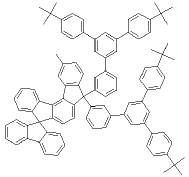 | 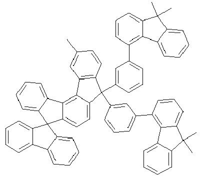 |
| BB-151+BB-180 M-0210 | BB-151+BB-181 M-0211 | BB-151+BB-182 M-0212 |
| 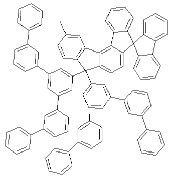 | 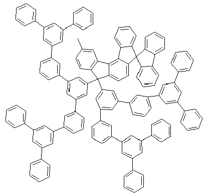 | 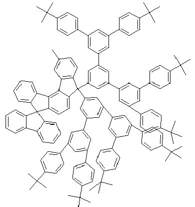 |
| BB-152+BB-179 M-0213 | BB-152+BB-180 M-0214 | BB-152+BB-181 M-0215 |
| 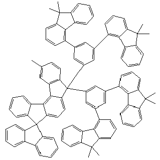 | 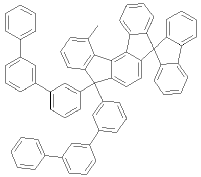 | 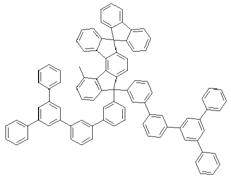 |
| BB-152+BB-182 M-0216 | BB-153+BB-179 M-0217 | BB-153+BB-180 M-0218 |
| 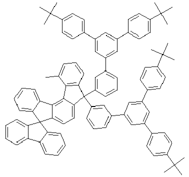 | 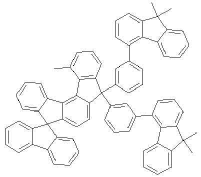 | 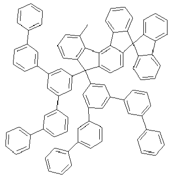 |
| BB-153+BB-181 M-0219 | BB-153+BB-182 M-0220 | BB-154+BB-179 M-0221 |
| 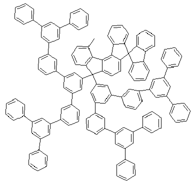 | 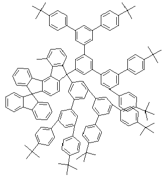 | 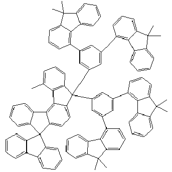 |
| BB-154+BB-180 M-0222 | BB-154+BB-181 M-0223 | BB-154+BB-182 M-0224 |
| 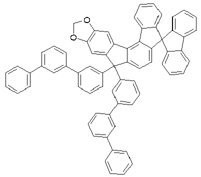 | 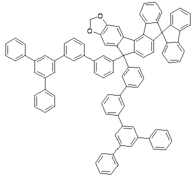 | 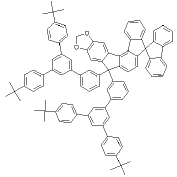 |
| BB-155+BB-179 M-0225 | BB-155+BB-180 M-0226 | BB-155+BB-181 M-0227 |
| 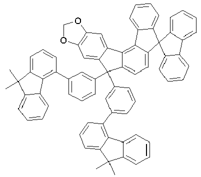 | 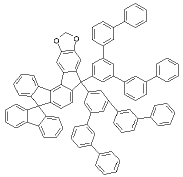 | 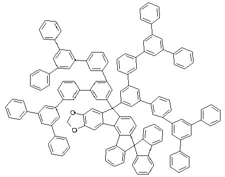 |
| BB-155+BB-182 M-0228 | BB-156+BB-179 M-0229 | BB-156+BB-180 M-0230 |
| 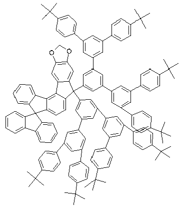 | 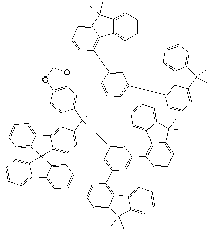 | 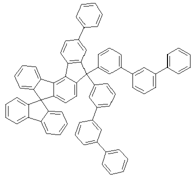 |
| BB-156+BB-181 M-0231 | BB-156+BB-182 M-0232 | BB-157+BB-179 M-0233 |
| 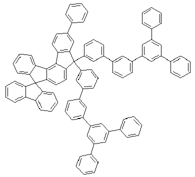 | 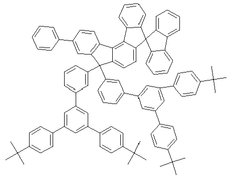 | 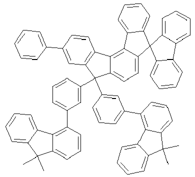 |
| BB-157+BB-180 M-0234 | BB-157+BB-181 M-0235 | BB-157+BB-182 M-0236 |
| 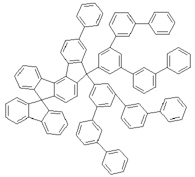 | 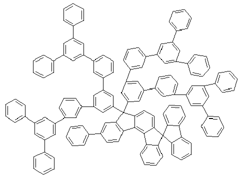 | 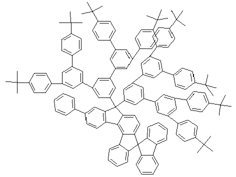 |
| BB-158+BB-179 M-0237 | BB-158+BB-180 M-0238 | BB-158+BB-181 M-0239 |
| 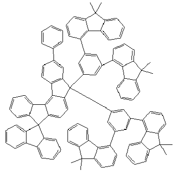 | 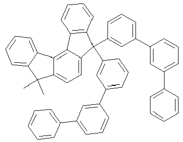 | 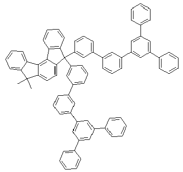 |
| BB-158+BB-182 M-0240 | BB-159+BB-179 M-0241 | BB-159+BB-180 M-0242 |
| 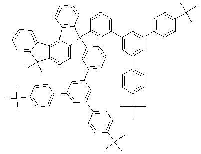 | 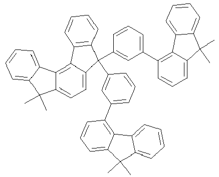 | 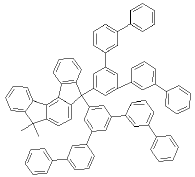 |
| BB-159+BB-181 M-0243 | BB-159+BB-182 M-0244 | BB-160+BB-179 M-0245 |
| 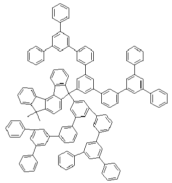 | 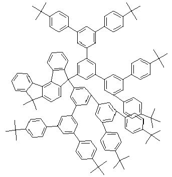 | 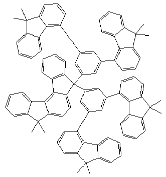 |
| BB-160+BB-180 M-0246 | BB-160+BB-181 M-0247 | BB-160+BB-182 M-0248 |
| 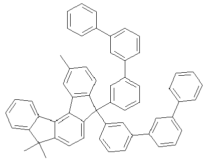 | 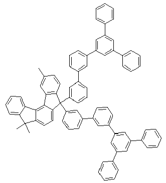 | 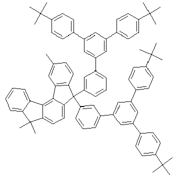 |
| BB-161+BB-179 M-0249 | BB-161+BB-180 M-0250 | BB-161+BB-181 M-0251 |
| 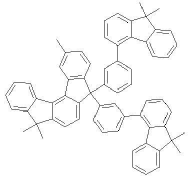 | 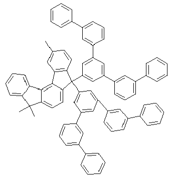 | 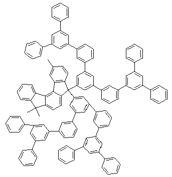 |
| BB-161+BB-182 M-0252 | BB-162+BB-179 M-0253 | BB-162+BB-180 M-0254 |
| 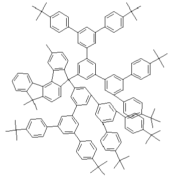 | 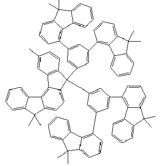 | 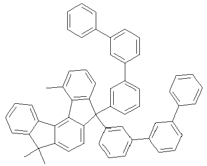 |
| BB-162+BB-181 M-0255 | BB-162+BB-182 M-0256 | BB-163+BB-179 M-0257 |
| 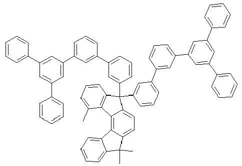 | 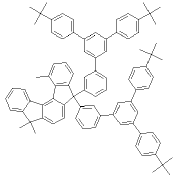 | 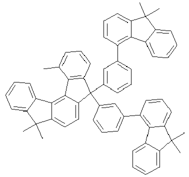 |
| BB-163+BB-180 M-0258 | BB-163+BB-181 M-0259 | BB-163+BB-182 M-0260 |
| | 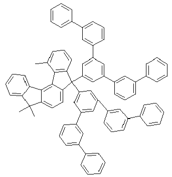 | 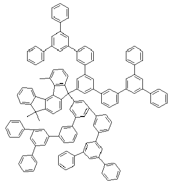 |
| | BB-164+BB-179 M-0261 | BB-164+BB-180 M-0262 |
| 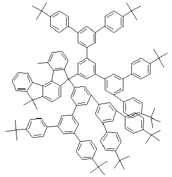 | 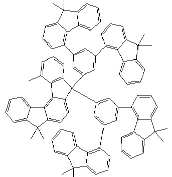 | 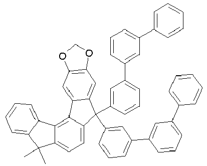 |
| BB-164+BB-181 M-0263 | BB-164+BB-182 M-0264 | BB-165+BB-179 M-0265 |
| 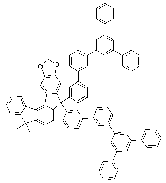 | 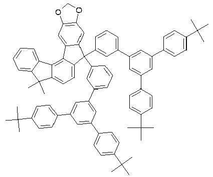 | 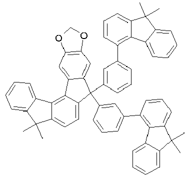 |
| BB-165+BB-180 M-0266 | BB-165+BB-181 M-0267 | BB-165+BB-182 M-0268 |
| 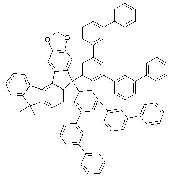 | 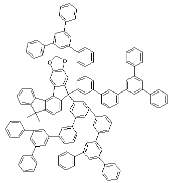 | 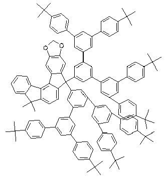 |
| BB-166+BB-179 M-0269 | BB-166+BB-180 M-0270 | BB-166+BB-181 M-0271 |
| 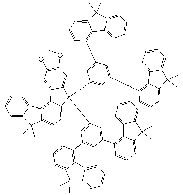 | 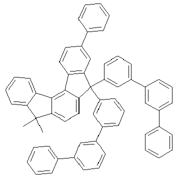 | 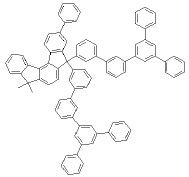 |
| BB-166+BB-182 M-0272 | BB-167+BB-179 M-0273 | BB-167+BB-180 M-0274 |
| 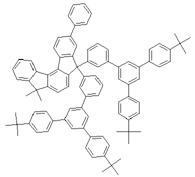 | 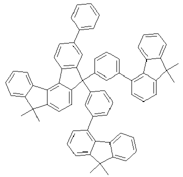 | 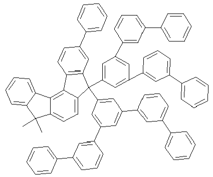 |
| BB-167+BB-181 M-0275 | BB-167+BB-182 M-0276 | BB-168+BB-179 M-0277 |
| 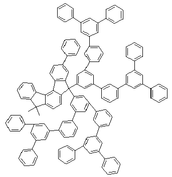 | 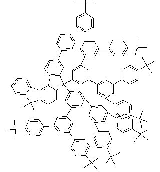 | 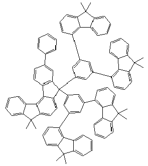 |
| BB-168+BB-180 M-0278 | BB-168+BB-181 M-0279 | BB-168+BB-182 M-0280 |
| 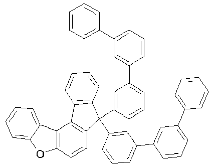 | 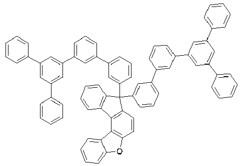 | 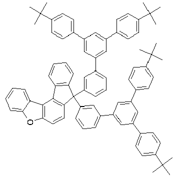 |
| BB-169+BB-179 M-0281 | BB-169+BB-180 M-0282 | BB-169+BB-181 M-0283 |
| 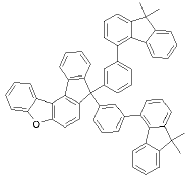 | 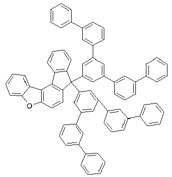 | 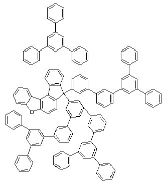 |
| BB-169+BB-182 M-0284 | BB-170+BB-179 M-0285 | BB-170+BB-180 M-0286 |
| 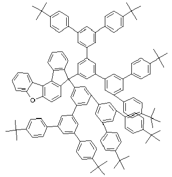 | 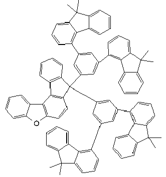 | 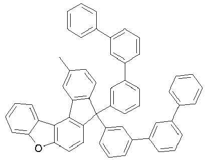 |
| BB-170+BB-181 M-0287 | BB-170+BB-182 M-0288 | BB-171+BB-179 M-0289 |
| 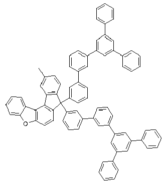 | 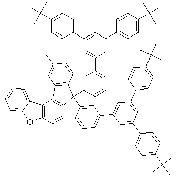 | 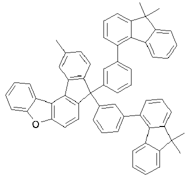 |
| BB-171+BB-180 M-0290 | BB-171+BB-181 M-0291 | BB-171+BB-182 M-0292 |
| 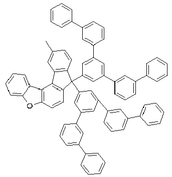 | 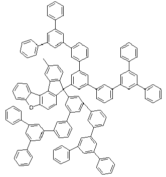 | 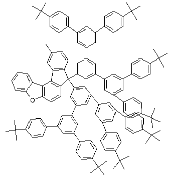 |
| BB-172+BB-179 M-0293 | BB-172+BB-180 M-0294 | BB-172+BB-181 M-0295 |
| 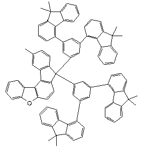 | 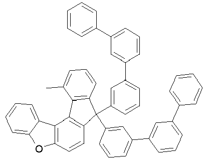 | 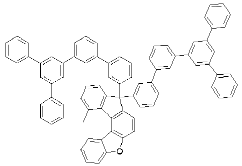 |
| BB-172+BB-182 M-0296 | BB-173+BB-179 M-0297 | BB-173+BB-180 M-0298 |
| 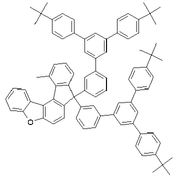 | 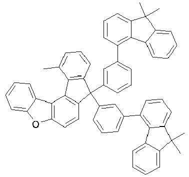 | 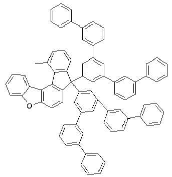 |
| BB-173+BB-181 M-0299 | BB-173+BB-182 M-0300 | BB-174+BB-179 M-0301 |
| 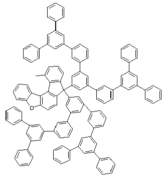 | 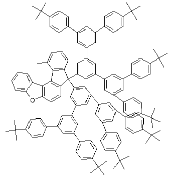 | 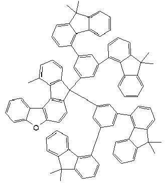 |
| BB-174+BB-180 M-0302 | BB-174+BB-181 M-0303 | BB-174+BB-182 M-0304 |
| 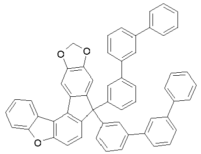 | 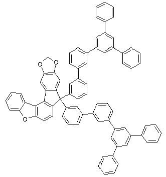 | 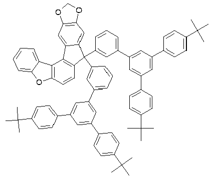 |
| BB-175+BB-179 M-0305 | BB-175+BB-180 M-0306 | BB-175+BB-181 M-0307 |
| 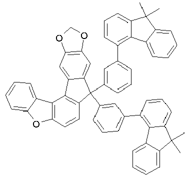 | 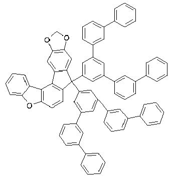 | 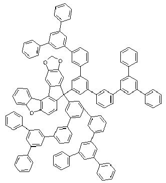 |
| BB-175+BB-182 M-0308 | BB-176+BB-179 M-0309 | BB-176+BB-180 M-0310 |
| 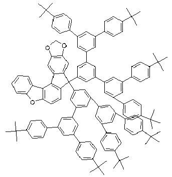 | 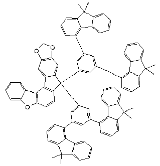 | 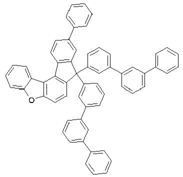 |
| BB-176+BB-181 M-0311 | BB-176+BB-182 M-0312 | BB-177+BB-179 M-0313 |
| 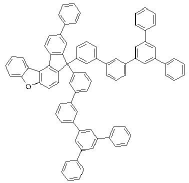 | 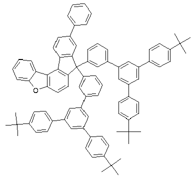 | 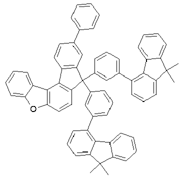 |
| BB-177+BB-180 M-0314 | BB-177+BB-181 M-0315 | BB-177+BB-182 M-0316 |
| 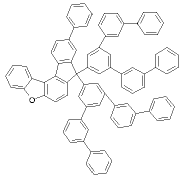 | 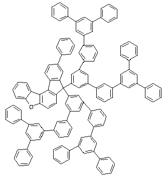 | 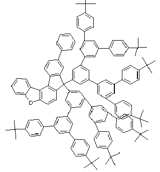 |
| BB-178+BB-179 M-0317 | BB-178+BB-180 M-0318 | BB-178+BB-181 M-0319 |
| 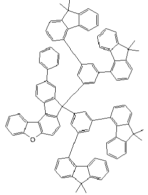 | | |
| BB-178+BB-182 M-0320 | | |

### Device-Beispiele

### Beispiel 1: Prozessierbarkeit

Die erfindungsgemäßen Materialien können unter anderem aus Lösung verarbeitet werden. Dabei zeichnen sie sich durch eine erhöhte Löslichkeit gegenüber symmetrischen Vergleichsverbindungen aus, wie in Tabelle 1 gezeigt. Weiterhin zeichnen sich erfindungsgemäße Materialien dadurch aus, dass ihre Tintenstabilität erhöht ist, wie durch die Langzeitlöslichkeitsdaten in Tabelle 1 hervorgeht.

Die erfindungsgemäße Verbindung M-0005 ist direkt vergleichbar mit der Verbindung B-1 und unterscheidet sich lediglich in der Anwesenheit bzw. Abwesenheit der Gruppe Ar am Fluoren. Man erkennt, dass die Löslichkeit von M-0005 in Cyclohexylbenzol signifikant besser ist als die Löslichkeit von B-1. Weiterhin ist die Langzeitstabilität der Lösung von M-0005 in Mesitylen wesentlich besser als von B-1 in Mesitylen.

Die erfindungsgemäßen Verbindungen M-0002, M-0097 und M-0226 sind direkt vergleichbar mit der Verbindung B-2 und unterscheiden sich lediglich oder im Wesentlichen durch die Anwesenheit bzw. Abwesenheit der Gruppe Ar am Fluoren. Man erkennt, dass die Löslichkeit der erfindungsgemäßen Verbindungen M-0097 und M-0226 in Toluol und Mesitylen signifikant besser ist als die Löslichkeit von B-2. Weiterhin ist die Langzeitstabilität der Lösungen von M-0097 und M-0226 wesentlich besser als von B-2.

**Tabelle 1: Löslichkeit**

| Bsp. | Material | Löslichkeit nach 24 Stunden [g/l] | | | | Löslichkeit nach 7 Tagen [g/l] | | | |
|---|---|---|---|---|---|---|---|---|---|
| | | Cyclohexylbenzol | Tetralin | Toluol | Mesitylen | Cyclohexylbenzol | Tetralin | Toluol | Mesitylen |
| V1.1 | B-1 | <20 | >50 | >50 | >50 | <20 | >50 | >50 | <40 |
| V1.2 | B-2 | >50 | >50 | <1 | <1 | <1 | <20 | <1 | <1 |
| V1.3 | B-3 | >50 | <50 | >50 | <50 | >50 | <50 | >50 | <40 |
| E1.1 | M-0005 | >50 | >50 | >50 | >50 | >50 | <50 | >50 | >50 |
| E1.2 | M-0002 | <1 | >50 | >50 | >50 | <1 | >50 | >50 | 50 |
| E1.3 | M-0008 | <50 | <30 | >50 | >50 | <50 | <30 | >50 | >50 |
| E1.4 | M-0026 | >50 | >50 | >50 | >50 | >50 | >50 | >50 | >50 |
| E1.5 | M-0033 | >50 | >50 | >50 | >50 | >50 | >50 | >50 | >50 |
| E1.6 | M-0043 | <50 | >50 | <50 | <50 | <50 | >50 | <50 | <50 |
| E1.7 | M-0086 | >50 | >50 | >50 | >50 | >50 | >50 | >50 | >50 |
| E1.8 | M-0097 | >50 | >50 | <20 | <20 | <25 | <25 | <20 | <20 |
| E1.9 | M-0162 | <50 | >50 | >50 | >50 | <50 | >50 | >50 | >50 |
| E1.10 | M-0172 | <40 | <40 | <30 | <30 | <40 | <40 | <30 | <30 |
| E1.11 | M-0203 | <40 | <40 | <20 | <20 | <40 | <40 | <20 | <20 |
| E1.12 | M-0226 | <30 | <30 | <20 | <20 | <25 | <25 | <20 | <20 |
| E1.13 | M-0244 | <50 | <50 | <20 | <20 | <40 | <40 | <20 | <20 |
| E1.14 | M-0281 | <50 | <50 | <50 | <50 | <50 | <50 | <50 | <50 |
| E1.15 | M-0315 | <50 | >50 | <50 | <50 | <50 | >50 | <50 | <50 |

### Methode zur Bestimmung der Löslichkeit via HPTLC

**Probenvorbereitung:** Das Material wird zweimal zu je 25 mg eingewogen (W1, W2), mit 0,5 mL Lösungsmittel versetzt, für 60 Minuten bei 60 °C und danach für 24 h bei 25 °C und 600 u/min geschüttelt. Wenn die Lösung klar ist, wird mehr Feststoff hinzugefügt, bis eine gesättigte Lösung entsteht. Nach dem Abkühlen auf Raumtemperatur werden die Proben durch einen Spritzenfilter (0,2 µm) filtriert. Die Lösungen werden in Glasfläschchen 7 Tage bei Raumtemperatur gelagert, und die Löslichkeit wird per HPTLC an Tag 1 und 7 bestimmt. Dazu werden die filtrierten Lösungen verdünnt.
**Kalibrierung:** Erstellung einer Standard-Lösung: ca. 6 mg Substanz werden in 20 mL 2-Methyl-THF aufgelöst. Wenn es nicht möglich ist, das Material unter diesen Bedingungen zu lösen, kann die Konzentration verringert werden. Für die Kalibrierung wird die Stammlösung verdünnt (Normierung).
**Chromatographische Bedingungen (HPTLC):** Platte: HPTLC RP 18
   F254, 10x20 cm Laufmittel: Methanol/2-Methyl-THF 70/30 (V/V)
   Migrations-Abstand: 5 cm
   Erkennung: 366 nm
   Anwendungsvolumen: Standard: 1, 3, 6, 10, 15, 20, 25, 30 µL
**HPTLC-Messung:** Die HPTLC-Platte wird von einem TLC-Scanner gescannt, Peaks integriert und eine Kalibrierfunktion bestimmt.

Darüber hinaus zeichnen erfindungsgemäße Materialien sich, wie in Tabelle 2 gezeigt, dadurch aus, dass Filme, die diese Materialien enthalten, bei höheren Temperaturen ausgeheizt werden können, ohne dass sie trüb werden und bei Verwendung in OLED-Bauteilen zu erniedrigter Effizienz führen. Die Herstellung der entsprechenden OLED-Bauteile wird im Folgenden beschrieben. Die EML-Mischungen und Aufbauten der untersuchten OLED-Bauteile sind in denTabellen 5 und 6 dargestellt.

Beispielsweise ist die Verbindung M-0002 direkt vergleichbar mit der Verbindung B-2 und unterscheidet sich nur in der Anwesenheit der Gruppe Ar am Fluoren. Man erkennt, dass man die Schicht enthaltend M-0002 ohne Trübung und mit einem signifikant geringeren Effizienzverlust bei höherer Temperatur ausheizen kann.

**Tabelle 2: Ausheizen bei erhöhter Temperatur**

| Bsp. | Material | Ref.- Temp. | erhöhte Temp. | Filmzustand nach Ausheizen bei erhöhter Temp. | Effizienzverlust nach Ausheizen bei erhöhter Temperatur |
|---|---|---|---|---|---|
| V2.1 | B-2 | 160 | 180 | leicht trüb | ≥ 10% |
| V2.2 | B-1 | 160 | 180 | trüb | ≥ 70% |
| E2.1 | M-0002 | 160 | 180 | klar | ≤ 5% |
| V2.3 | B-1 | 160 | 170 | leicht trüb | ≥ 10% |
| E2.2 | M-0005 | 160 | 170 | klar | ≤ 5% |
| V2.4 | B-4 | 150 | 180 | leicht trüb | ≥ 10% |
| V2.5 | B-1 | 150 | 180 | trüb | ≥ 70% |
| E2.3 | M-0002 | 150 | 180 | klar | ≤ 5% |
| E2.4 | M-0005 | 150 | 170 | klar | ≤ 5% |
| E2.5 | M-0172 | 150 | 170 | klar | ≤ 5% |
| E2.6 | M-0008 | 150 | 180 | klar | ≤ 5% |
| E2.7 | M-0026 | 150 | 180 | klar | ≤ 5% |
| E2.8 | M-0033 | 150 | 180 | klar | ≤ 5% |
| E2.9 | M-0043 | 150 | 180 | klar | ≤ 5% |
| E2.10 | M-0086 | 150 | 180 | klar | ≤ 5% |
| E2.11 | M-0097 | 150 | 180 | klar | ≤ 5% |
| E2.12 | M-0162 | 150 | 180 | klar | ≤ 5% |
| E2.13 | M-0203 | 150 | 180 | klar | ≤ 5% |
| E2.14 | M-0226 | 150 | 180 | klar | ≤ 5% |
| E2.15 | M-0315 | 150 | 180 | klar | ≤ 5% |

### Beispiel 2: OLED-Bauteile

Die erfindungsgemäßen Matrixmaterialien können unter anderem aus Lösung verarbeitet werden. Die Herstellung vollständig lösungsbasierter OLEDs ist in der Literatur bereits vielfach beschrieben, z. B. in WO 2004/037887 mittels Spin-Coating. Die Herstellung vakuumbasierter OLEDs ist ebenfalls vielfach vorbeschrieben, u.a. in WO 2004/058911. In den im Folgenden diskutierten Beispielen werden lösungsbasiert und vakuumbasiert aufgebrachte Schichten innerhalb einer OLED kombiniert, so dass die Prozessierung bis einschließlich zur Emissionsschicht aus Lösung und in den darauf folgenden Schichten (Lochblockierschicht und Elektronentransportschicht) aus dem Vakuum erfolgt. Die vorbeschriebenen allgemeinen Verfahren werden dafür wie folgt auf die hier beschriebenen Gegebenheiten (Schichtdickenvariation, Materialien) angepasst und kombiniert. Der generelle Aufbau ist wie folgt: Substrat / ITO (50 nm) / Lochinjektionsschicht (HIL) / Lochtransportschicht (HTL) / Emissionsschicht (EML) / ggf. Lochblockierschicht (HBL) / Elektronentransportschicht (ETL) / ggf. Elektroneninjektionsschicht / Kathode (Aluminium, 100 nm). Als Substrat dienen Glasplättchen, die mit strukturiertem ITO (Indium Zinn Oxid) der Dicke 50 nm beschichtet sind. Zur besseren Prozessierung werden diese mit PEDOT:PSS beschichtet (Poly(3,4-ethylendioxy-2,5-thiophen):Polystyrolsulfonat, bezogen von Heraeus Precious Metals GmbH & Co. KG, Deutschland). PEDOT:PSS wird an Luft aus Wasser aufgeschleudert und nachfolgend an Luft bei 180 °C für 10 Minuten ausgeheizt, um Restwasser zu entfernen. Auf diese beschichteten Glasplättchen werden die Lochtransportschicht sowie die Emissionsschicht aufgebracht. Die verwendete Lochtransportschicht ist vernetzbar. Es wird ein Polymer gemäß den nachfolgend abgebildeten Strukturen verwendet, das gemäß WO 2010/097155 bzw. WO 2013/156130 synthetisiert werden kann:

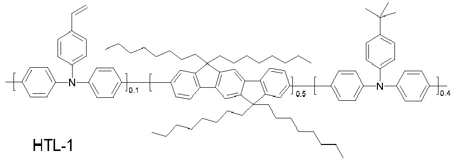

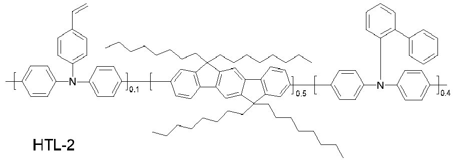

Das Lochtransport-Polymer wird in Toluol gelöst. Der typische Feststoffgehalt solcher Lösungen liegt bei ca. 5 g/l, wenn, wie hier, die für eine Device typische Schichtdicke von 20-60 nm mittels Spincoating erzielt werden soll. Die Schichten werden in einer Inertgasatmosphäre, im vorliegenden Fall Argon, aufgeschleudert und 60 Minuten bei 180 °C ausgeheizt.

Die Emissionsschicht setzt sich aus mindestens einem Matrixmaterial (Hostmaterial, Wirtsmaterial) und einem emittierenden Dotierstoff (Dotand, Emitter) zusammen. Weiterhin können Mischungen aus mehreren Matrixmaterialien sowie Co-Dotanden verwendet werden. Eine Angabe wie TMM-A (92%) : Dotand (8%) bedeutet hierbei, dass das Material TMM-A in einem Gewichtsanteil von 92% und Dotand in einem Gewichtsanteil von 8% in der Emissionsschicht vorliegt. Die Mischung für die Emissionsschicht wird in Toluol oder ggf. Chlorbenzol gelöst. Der typische Feststoffgehalt solcher Lösungen liegt bei ca. 17 g/l, wenn, wie hier, die für eine Device typische Schichtdicke von 60 nm mittels Spincoating erzielt werden soll. Die Schichten werden in einer Inertgasatmosphäre, im vorliegenden Fall Argon, aufgeschleudert und 10 Minuten ausgeheizt. Die im vorliegenden Fall verwendeten Materialien sind in Tabelle 3 gezeigt.

**Tabelle 3: Verwendete EML-Materialien**

| | | |
|---|---|---|
| 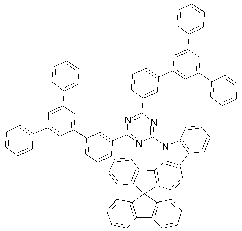 | 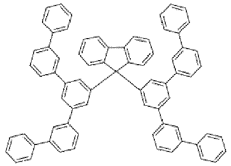 | 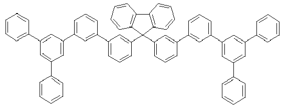 |
| A-1 | B-1 | B-2 |
| s. WO 2014/094963 | [1246496-85-4] | [2047361-36-2] |
| 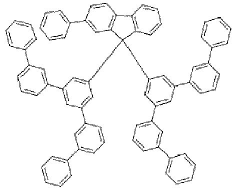 | 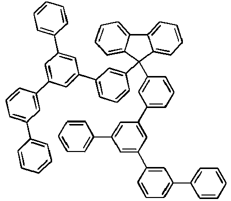 | |
| B-3 | B-4 | |
| s. WO 2016/184540 | [2231029-69-7] | |
| 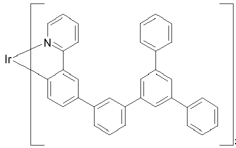 | 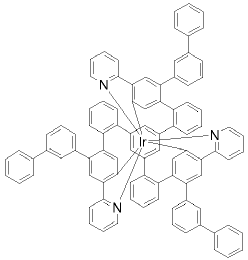 | |
| C-1 | C-2 | |
| [1269508-30-6] | s. WO 2016/124304 | |
| 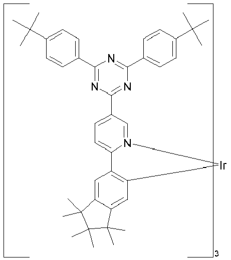 | 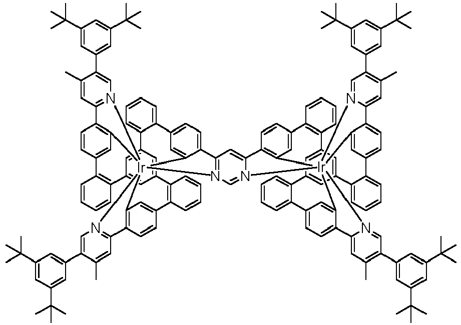 | |
| D-1 | D-2 | |
| [1870013-87-8 | [2202715-38-4] | |

Die Materialien für die Lochblockierschicht, Elektronentransportschicht und Elektroneninjektionsschicht werden in einer Vakuumkammer thermisch aufgedampft. Dabei kann z. B. die Elektronentransportschicht aus mehr als einem Material bestehen, die einander durch Co-Verdampfung in einem bestimmten Volumenanteil beigemischt werden. Eine Angabe wie ETM1:ETM2 (50%:50%) bedeutet hierbei, dass die Materialien ETM1 und ETM2 in einem Volumenanteil von je 50% in der Schicht vorliegen. Die im vorliegenden Fall verwendeten Materialien sind in Tabelle 4 gezeigt.

**Tabelle 4: Verwendete HBL- und ETL-Materialien**

| | | |
|---|---|---|
| 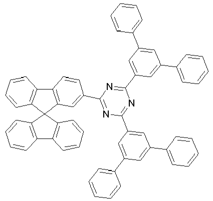 | 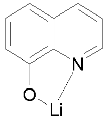 | |
| ETM1 | ETM2 | |
| [1233200-52-6] | [25387-93-3] | |

Die Kathode wird durch die thermische Verdampfung einer 100 nm dicken Aluminiumschicht gebildet. Die OLEDs werden standardmäßig charakterisiert. Hierfür werden die Elektrolumineszenzspektren, Strom-Spannungs-Leuchtdichte-Kennlinien (IUL-Kennlinien) unter Annahme einer Lambert'schen Abstrahlcharakteristik, sowie die (Betriebs-)Lebensdauer bestimmt. Aus den IUL-Kennlinien werden Kennzahlen bestimmt, wie z. B. die externe Quanteneffizienz bei einer bestimmten Helligkeit. LT97 @ 60 mA/cm² ist die Lebensdauer, bis die OLED bei konstanter Stromdichte von 60 mA/cm² auf 97% der Anfangsleuchtdichte abgefallen ist.

Die EML-Mischungen und Aufbauten der untersuchten OLED-Bauteile sind in den Tabelle 5 und 6 dargestellt. Die zugehörigen Ergebnisse finden sich in Tabelle 7.

**Tabelle 5: EML-Mischungen der untersuchten OLED-Bauteile**

| Bsp. | Matrix A | | Co-Matrix B | | Co-Dotand C | | Dotand D | |
|---|---|---|---|---|---|---|---|---|
| | Material | % | Material | % | Material | % | Material | % |
| V2.1 | A-1 | 20 | B-2 | 60 | C-1 | 20 | --- | --- |
| V2.2 | A-1 | 20 | B-1 | 60 | C-1 | 20 | --- | --- |
| E2.1 | A-1 | 20 | M-0002 | 60 | C-1 | 20 | --- | --- |
| V2.3 | A-1 | 25 | B-1 | 53 | C-2 | 22 | --- | --- |
| E2.2 | A-1 | 25 | M-0005 | 53 | C-2 | 22 | --- | --- |
| V2.4 | A-1 | 30 | B-4 | 34 | C-1 | 30 | D-1 | 6 |
| V2.5 | A-1 | 30 | B-1 | 34 | C-1 | 30 | D-1 | 6 |
| E2.3 | A-1 | 30 | M-0002 | 34 | C-1 | 30 | D-1 | 6 |
| E2.4 | A-1 | 30 | M-0005 | 34 | C-1 | 30 | D-2 | 6 |
| E2.5 | A-1 | 30 | M-0172 | 34 | C-1 | 30 | D-1 | 6 |
| E2.6 | A-1 | 30 | M-0008 | 34 | C-1 | 30 | D-1 | 6 |
| E2.7 | A-1 | 30 | M-0026 | 34 | C-1 | 30 | D-1 | 6 |
| E2.8 | A-1 | 30 | M-0033 | 34 | C-1 | 30 | D-2 | 6 |
| E2.9 | A-1 | 30 | M-0043 | 34 | C-1 | 30 | D-2 | 6 |
| E2.10 | A-1 | 30 | M-0086 | 34 | C-1 | 30 | D-2 | 6 |
| E2.11 | A-1 | 30 | M-0097 | 34 | C-1 | 30 | D-1 | 6 |
| E2.12 | A-1 | 30 | M-0162 | 34 | C-1 | 30 | D-1 | 6 |
| E2.13 | A-1 | 30 | M-0203 | 34 | C-1 | 30 | D-1 | 6 |
| E2.14 | A-1 | 30 | M-0226 | 34 | C-1 | 30 | D-1 | 6 |
| E2.15 | A-1 | 30 | M-0315 | 34 | C-1 | 30 | D-1 | 6 |
| V3.1 | A-1 | 40 | B-3 | 37 | C-2 | 15 | D-2 | 8 |
| E3.1 | A-1 | 40 | M-0005 | 37 | C-2 | 15 | D-2 | 8 |
| V3.2 | A-1 | 40 | B-3 | 37 | C-2 | 15 | D-2 | 8 |
| E3.2 | A-1 | 40 | M-0005 | 37 | C-2 | 15 | D-2 | 8 |
| V3.3 | A-1 | 30 | B-1 | 34 | C-1 | 30 | D-1 | 6 |
| V3.4 | A-1 | 30 | B-2 | 34 | C-1 | 30 | D-1 | 6 |
| E3.3 | A-1 | 30 | M-0002 | 34 | C-1 | 30 | D-1 | 6 |
| V3.5 | A-1 | 20 | B-4 | 60 | C-1 | 20 | --- | --- |
| E3.4 | A-1 | 20 | M-0002 | 60 | C-1 | 20 | --- | --- |
| V3.6 | A-1 | 20 | B-4 | 60 | C-1 | 20 | --- | --- |
| E3.5 | A-1 | 20 | M-0002 | 60 | C-1 | 20 | --- | --- |

**Tabelle 6: Aufbau der untersuchten OLED-Bauteile**

| Bsp. | HIL (Dicke) | HTL (Dicke) | EML-Dicke | EML-Ausheiztemperatur | HBL (Dicke) | ETL (Dicke) | EIL (Dicke) |
|---|---|---|---|---|---|---|---|
| V2.1 | PEDOT (20nm) | HTL-1 (20nm) | 60nm | s. Tab. 2 | ETM-1 (10nm) | ETM-1 : ETM-2 (50:50) (40nm) | --- |
| V2.2 | PEDOT (20nm) | HTL-1 (20nm) | 60nm | s. Tab. 2 | ETM-1 (10nm) | ETM-1 : ETM-2 (50:50) (40nm) | --- |
| E2.1 | PEDOT (20nm) | HTL-2 (20nm) | 60nm | s. Tab. 2 | ETM-1 (10nm) | ETM-1 : ETM-2 (50:50) (40nm) | |
| V2.3 | PEDOT (20nm) | HTL-2 (20nm) | 60nm | s. Tab. 2 | ETM-1 (10nm) | ETM-1 : ETM-2 (50:50) (40nm) | --- |
| E2.2 | PEDOT (20nm) | HTL-2 (20nm) | 60nm | s. Tab. 2 | ETM-1 (10nm) | ETM-1 : ETM-2 (50:50) (40nm) | --- |
| V2.4 | PEDOT (60nm) | HTL-2 (20nm) | 60nm | s. Tab. 2 | ETM-1 (10nm) | ETM-1 : ETM-2 (50:50) (40nm) | --- |
| V2.5 | PEDOT (60nm) | HTL-2 (20nm) | 60nm | s. Tab. 2 | ETM-1 (10nm) | ETM-1 : ETM-2 (50:50) (40nm) | --- |
| E2.3 | PEDOT (60nm) | HTL-2 (20nm) | 60nm | s. Tab. 2 | ETM-1 (10nm) | ETM-1 : ETM-2 (50:50) (40nm) | --- |
| E2.4 | PEDOT (60nm) | HTL-2 (20nm) | 60nm | s. Tab. 2 | ETM-1 (10nm) | ETM-1 : ETM-2 (50:50) (40nm) | --- |
| E2.5 | PEDOT (60nm) | HTL-2 (20nm) | 60nm | s. Tab. 2 | ETM-1 (10nm) | ETM-1 : ETM-2 (50:50) (40nm) | --- |
| E2.6 | PEDOT (60nm) | HTL-2 (20nm) | 60nm | s. Tab. 2 | ETM-1 (10nm) | ETM-1 : ETM-2 (50:50) (40nm) | --- |
| E2.7 | PEDOT (60nm) | HTL-2 (20nm) | 60nm | s. Tab. 2 | ETM-1 (10nm) | ETM-1 : ETM-2 (50:50) (40nm) | --- |
| E2.8 | PEDOT (60nm) | HTL-2 (20nm) | 60nm | s. Tab. 2 | ETM-1 (10nm) | ETM-1 : ETM-2 (50:50) (40nm) | --- |
| E2.9 | PEDOT (60nm) | HTL-2 (20nm) | 60nm | s. Tab. 2 | ETM-1 (10nm) | ETM-1 : ETM-2 (50:50) (40nm) | --- |
| E2.10 | PEDOT (60nm) | HTL-2 (20nm) | 60nm | s. Tab. 2 | ETM-1 (10nm) | ETM-1 : ETM-2 (50:50) (40nm) | --- |
| E2.11 | PEDOT (60nm) | HTL-2 (20nm) | 60nm | s. Tab. 2 | ETM-1 (10nm) | ETM-1 : ETM-2 (50:50) (40nm) | --- |
| E2.12 | PEDOT (60nm) | HTL-2 (20nm) | 60nm | s. Tab. 2 | ETM-1 (10nm) | ETM-1 : ETM-2 (50:50) (40nm) | --- |
| E2.13 | PEDOT (60nm) | HTL-2 (20nm) | 60nm | s. Tab. 2 | ETM-1 (10nm) | ETM-1 : ETM-2 (50:50) (40nm) | --- |
| E2.14 | PEDOT (60nm) | HTL-2 (20nm) | 60nm | s. Tab. 2 | ETM-1 (10nm) | ETM-1 : ETM-2 (50:50) (40nm) | --- |
| E2.15 | PEDOT (60nm) | HTL-2 (20nm) | 60nm | s. Tab. 2 | ETM-1 (10nm) | ETM-1 : ETM-2 (50:50) (40nm) | --- |
| V3.1 | PEDOT (80nm) | HTL-2 (20nm) | 90nm | 150 | --- | ETM-1 (20nm) | ETM-2 (3nm) |
| E3.1 | PEDOT (80nm) | HTL-2 (20nm) | 90nm | 150 | --- | ETM-1 (20nm) | ETM-2 (3nm) |
| V3.2 | PEDOT (80nm) | HTL-2 (20nm) | 90nm | 160 | --- | ETM-1 (20nm) | ETM-2 (3nm) |
| E3.2 | PEDOT (80nm) | HTL-2 (20nm) | 90nm | 160 | --- | ETM-1 (20nm) | ETM-2 (3nm) |
| V3.3 | PEDOT (60nm) | HTL-2 (20nm) | 60nm | 150 | ETM-1 (10nm) | ETM-1 : ETM-2 (50:50) (40nm) | --- |
| V3.4 | PEDOT (60nm) | HTL-2 (20nm) | 60nm | 150 | ETM-1 (10nm) | ETM-1 : ETM-2 (50:50) (40nm) | --- |
| E3.3 | PEDOT (60nm) | HTL-2 (20nm) | 60nm | 170 | ETM-1 (10nm) | ETM-1 : ETM-2 (50:50) (40nm) | --- |
| V3.5 | PEDOT (20nm) | HTL-2 (20nm) | 60nm | 160 | ETM-1 (10nm) | ETM-1 : ETM-2 (50:50) (40nm) | --- |
| E3.4 | PEDOT (20nm) | HTL-2 (20nm) | 60nm | 160 | ETM-1 (10nm) | ETM-1 : ETM-2 (50:50) (40nm) | --- |
| V3.6 | PEDOT (20nm) | HTL-2 (20nm) | 60nm | 150 | ETM-1 (10nm) | ETM-1 : ETM-2 (50:50) (40nm) | --- |
| E3.5 | PEDOT (20nm) | HTL-2 (20nm) | 60nm | 150 | ETM-1 (10nm) | ETM-1 : ETM-2 (50:50) (40nm) | --- |

**Tabelle 7: Ergebnisse lösungsprozessierter OLEDs**

| Bsp. | EQE [%] @1000cd/m² | LT97 @x mA/cm² | |
|---|---|---|---|
| V3.1 | 18,4 | 27 | 60 |
| E3.1 | 18,6 | 36 | 60 |
| V3.2 | 18,9 | 31 | 60 |
| E3.2 | 19,0 | 46 | 60 |
| V3.3 | 16,5 | 81 | 60 |
| V3.4 | 16,4 | 86 | 60 |
| E3.3 | 16,4 | 131 | 60 |
| V3.5 | 19,4 | 7 | 40 |
| E3.4 | 20,3 | 16 | 40 |
| V3.6 | 21,0 | 8 | 40 |
| E3.5 | 21,0 | 12 | 40 |

Wie aus den oben gezeigten OLED-Bauteildaten zu ersehen ist, führen die erfindungsgemäßen Materialien zu guten Leistungsdaten in Bezug auf Lebensdauer und Effizienz. Sie bieten hier eine Verbesserung im Vergleich zu bereits bekannten Vergleichsmaterialien. Zusätzlich zeigt sich, dass das größere Prozessfenster in Bezug auf Ausheiztemperaturen nicht nur praktische Vorteile in der industriellen Fertigung von Bauteilen hat, sondern auch das Erreichen hoher Bauteillebensdauern erleichtert.

Beispielsweise ist die Verbindung M-0005 direkt vergleichbar mit der Verbindung B-3 und unterscheidet sich nur in der Position der Gruppe Ar am Fluoren. Man erkennt, dass das erfindungsgemäße Substitutionsmuster sich positiv auf die Bauteillebensdauer auswirkt.

Ebenso ist die Verbindung M-0002 direkt vergleichbar mit den Verbindungen B-2 und B-4, von denen sie sich in der Anwesenheit der Gruppe Ar am Fluoren unterscheidet. Mit B-2 ist sie ansonsten identisch; von der Verbindung B-4 unterscheidet sie sich ansonsten nur in der Verknüpfung der Phenylringe in 9-Position des Fluorens. In beiden Fällen führt M-0002 zu verbesserten Bauteillebensdauern, im Beispiel E3.4 im Vergleich zu V3.5 darüber hinaus auch zu einer Verbesserung der Effizienz.

Auch im Vergleich zu der aus dem Stand der Technik bekannten Vergleichsverbindung B-1 führt M-0002 zu einer Verbesserung der Bauteillebensdauer.

## Patentansprüche

1. Verbindung der Formel (1), 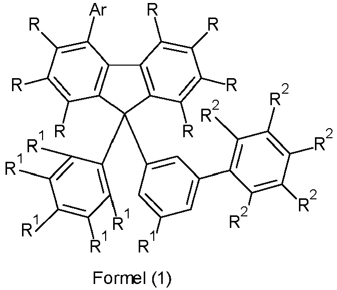 wobei für die verwendeten Symbole gilt:
Ar ist ein aromatisches Ringsystem mit 6 bis 30 aromatischen Ringatomen, welches mit einem oder mehreren Resten R³ substituiert sein kann, oder eine Dibenzofuran- oder Dibenzothiophengruppe, welche jeweils mit einem oder mehreren Resten R³ substituiert sein kann, oder eine Kombination aus einem aromatischen Ringsystem mit 6 bis 18 aromatischen Ringatomen und einer Dibenzofuran- oder Dibenzothiophengruppe, wobei diese Gruppen jeweils durch einen oder mehrere Reste R³ substituiert sein können; dabei kann Ar mit dem benachbarten Substituenten R ein Ringsystem bilden;
R ist bei jedem Auftreten gleich oder verschieden H, D, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenylgruppe mit 2 bis 20 C-Atomen, wobei die Alkyl-, Alkoxy- bzw. Alkenylgruppe jeweils mit einem oder mehreren Resten R³ substituiert sein kann, wobei ein oder mehrere H-Atome durch D ersetzt sein können, oder ein aromatisches Ringsystem mit 6 bis 30 aromatischen Ringatomen, das durch einen oder mehrere Reste R³ substituiert sein kann, oder eine Dibenzofuran- oder Dibenzothiophengruppe, die jeweils durch einen oder mehrere Reste R³ substituiert sein kann; dabei können zwei oder mehrere benachbarte Substituenten R auch miteinander ein mono- oder polycyclisches, aliphatisches Ringsystem bilden; weiterhin kann R mit einer benachbarten Gruppe Ar ein Ringsystem bilden;
R¹, R² ist bei jedem Auftreten gleich oder verschieden H, D, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenylgruppe mit 2 bis 20 C-Atomen, wobei die Alkyl-, Alkoxy- bzw. Alkenylgruppe jeweils mit einem oder mehreren Resten R³ substituiert sein kann, wobei ein oder mehrere H-Atome durch D ersetzt sein können, oder ein aromatisches Ringsystem mit 6 bis 30 aromatischen Ringatomen, das durch einen oder mehrere Reste R³ substituiert sein kann, oder eine Dibenzofuran- oder Dibenzothiophengruppe, die jeweils durch einen oder mehrere Reste R³ substiuiert sein kann; dabei können mehrere benachbarte Substituenten R¹ miteinander ein Ringsystem bilden; weiterhin können mehrere benachbarte Substituenten R² miteinander ein Ringsystem bilden;
R³ ist bei jedem Auftreten gleich oder verschieden H, D, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die Alkyl- bzw. Alkoxygruppe jeweils mit einem oder mehreren Resten R⁴ substituiert sein kann, wobei ein oder mehrere H-Atome durch D ersetzt sein können, oder ein aromatisches Ringsystem mit 6 bis 30 aromatischen Ringatomen, das durch einen oder mehrere Reste R⁴ substituiert sein kann, oder eine Dibenzofuran- oder Dibenzothiophengruppe, die jeweils durch einen oder mehrere Reste R⁴ substituiert sein kann; dabei können zwei oder mehrere benachbarte Substituenten R³ auch miteinander ein Ringsystem bilden;
R⁴ ist bei jedem Auftreten gleich oder verschieden H, D oder ein aliphatischer und/oder aromatischer Kohlenwasserstoffrest mit 1 bis 20 C-Atomen.

2. Verbindung nach Anspruch 1, ausgewählt aus den Verbindungen der Formeln (2a), (2b) und (2c), 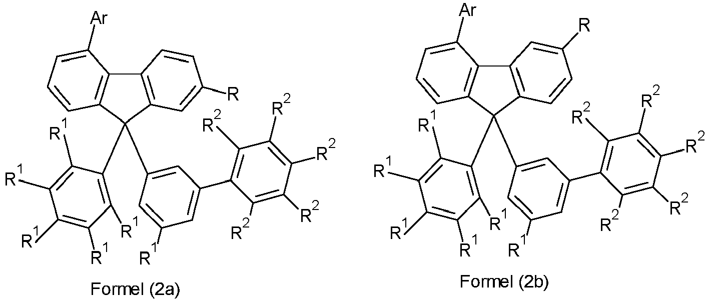 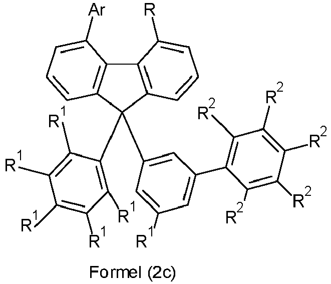 wobei die verwendeten Symbole die in Anspruch 1 genannten Bedeutungen aufweisen.

3. Verbindung nach Anspruch 1 oder 2, ausgewählt aus den Verbindungen der Formeln (3a) und (3b), 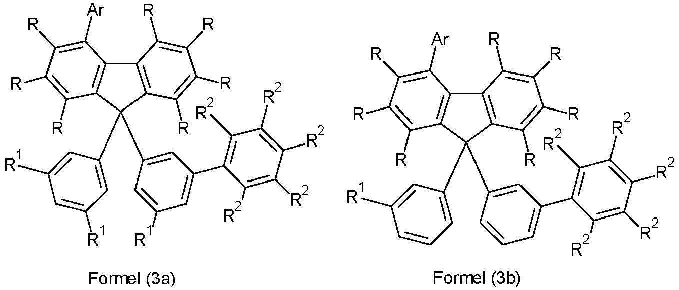 wobei die verwendeten Symbole die in Anspruch 1 genannten Bedeutungen aufweisen.

4. Verbindung nach einem oder mehreren der Ansprüche 1 bis 3, ausgewählt aus den Verbindungen der Formeln (4a) bis (4f), 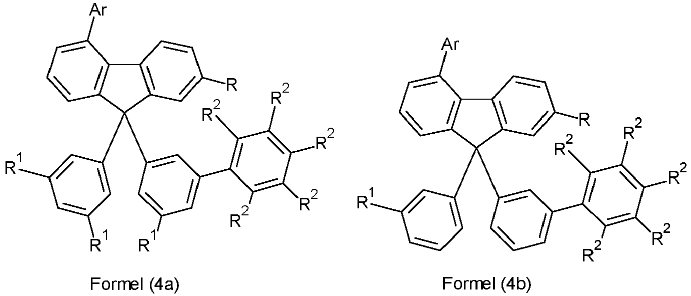 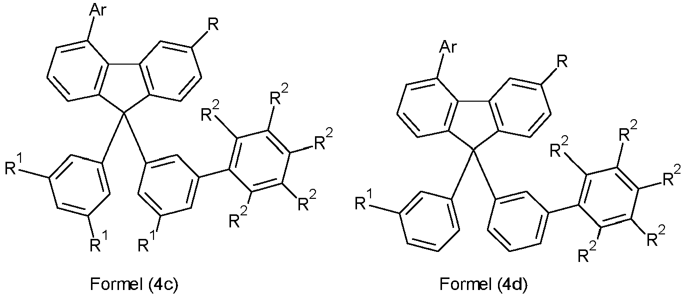 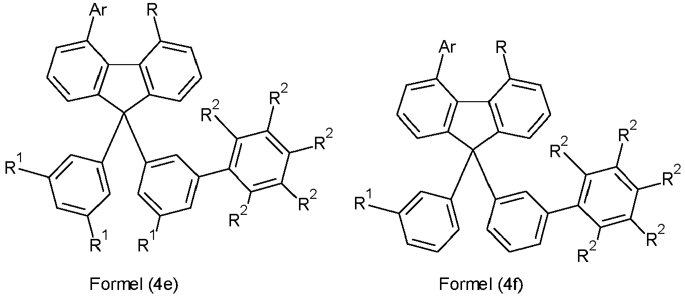 wobei die verwendeten Symbole die in Anspruch 1 genannten Bedeutungen aufweisen.

5. Verbindung nach einem der mehreren der Anpsprüche 1 bis 4, ausgewählt aus den Verbindungen der Formeln (6a) bis (6f), 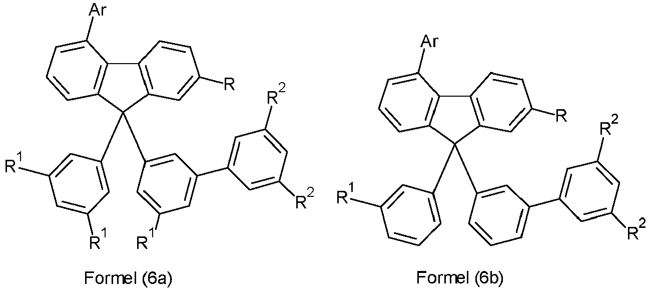 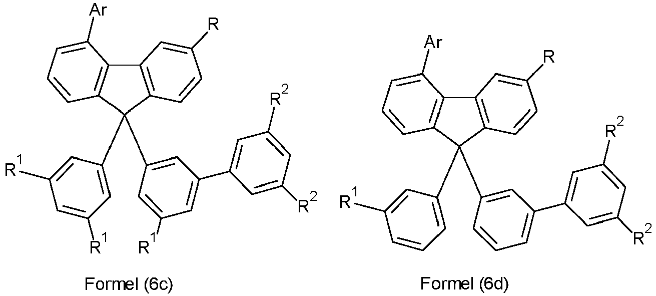 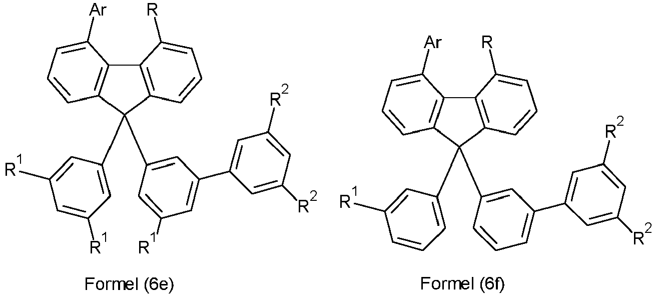 wobei die verwendeten Symbole die in Anspruch 1 genannten Bedeutungen aufweisen.

6. Verbindung nach einem oder mehreren der Ansprüche 1 bis 5, **dadurch gekennzeichnet, dass** Ar ausgewählt ist aus der Gruppe bestehend aus Phenyl, Biphenyl, Terphenyl, Quaterphenyl, Fluoren, Spirobifluoren, Naphthalin, Dibenzofuran, Dibenzothiophen, Phenanthren, Triphenylen oder einer Kombination dieser Gruppen, welche jeweils mit einem oder mehreren Resten R³ substituiert sein können.

7. Verbindung nach einem oder mehreren der Ansprüche 1 bis 6, ausgewählt aus den Verbindungen der Formeln (2d), (6g) bzw. (6h), 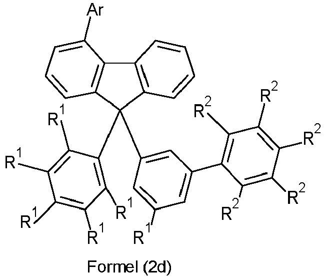 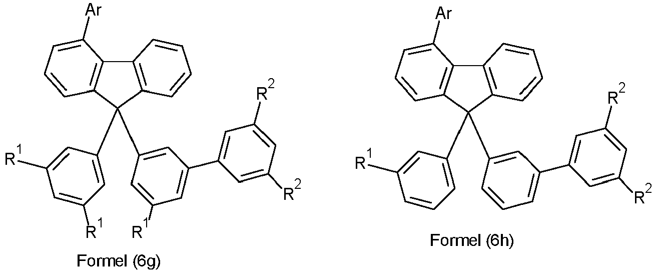 wobei die verwendeten Symbole die in Anspruch 1 genannten Bedeutungen aufweisen.

8. Verbindung nach einem oder mehreren der Ansprüche 1 bis 7, ausgewählt aus den Verbindungen der Formeln (8a), (8b), (9a) und (9b), 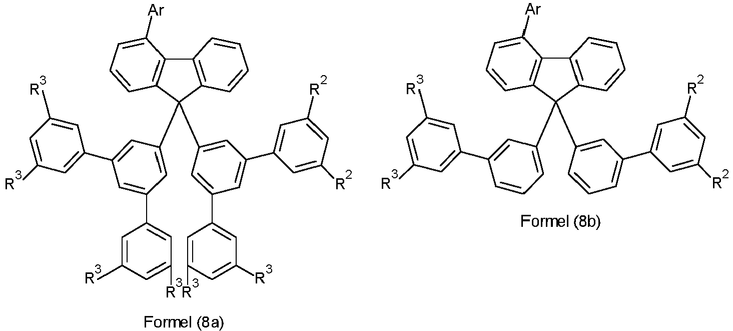 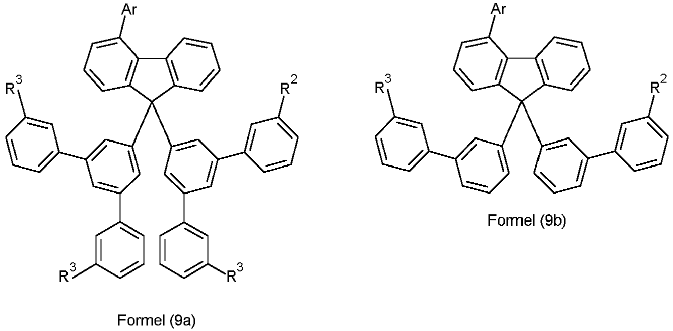 wobei die verwendeten Symbole die in Anspruch 1 genannten Bedeutungen aufweisen.

9. Zusammensetzung enthaltend mindestens eine Verbindung nach einem oder mehreren der Ansprüche 1 bis 8 und wenigstens ein weiteres funktionelles Material, wobei das weitere funktionelle Material bevorzugt ausgewählt ist aus einem phosphoreszierenden Emitter, einem Emitter, der TADF zeigt, und/oder einem Matrixmaterial, welches ausgewählt ist aus der Gruppe bestehend aus Elektronentransportmaterialien, Lochtransportmaterialien und bipolaren Materialien.

10. Formulierung enthaltend mindestens eine Verbindung nach einem oder mehreren der Ansprüche 1 bis 8 oder eine Zusammensetzung nach Anspruch 9 und mindestens ein organisches Lösemittel.

11. Verwendung einer Verbindung nach einem oder mehreren der Ansprüche 1 bis 8 oder eine Zusammensetzung nach Anspruch 9 in einer elektronischen Vorrichtung.

12. Elektronische Vorrichtung, enthaltend mindestens eine Verbindung nach einem oder mehreren der Ansprüche 1 bis 8 oder eine Zusammensetzung nach Anspruch 9.

13. Elektronische Vorrichtung nach Anspruch 12, wobei es sich um eine organische Elektrolumineszenzvorrichtung handelt, **dadurch gekennzeichnet, dass** die emittierende Schicht mindestens eine Verbindung nach einem oder mehreren der Ansprüche 1 bis 8 enthält.

14. Elektronische Vorrichtung nach Anspruch 13, **dadurch gekennzeichnet, dass** die emittierende Schicht mindestens ein Triazin-, Chinazolin- bzw. Pyrimidinderivat gemäß einer der Formeln (10), (11) und (12) enthält, 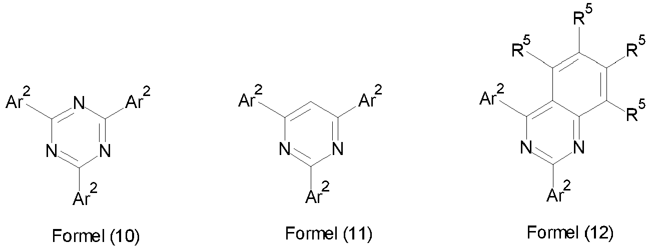 wobei R³ die in Anspruch 1 genannten Bedeutungen aufweist und für die weiteren verwendeten Symbole gilt:
Ar² ist bei jedem Auftreten gleich oder verschieden ein aromatisches oder heteroaromatisches Ringsystem mit 5 bis 40 aromatischen Ringatomen, welches durch einen oder mehrere Reste R⁵ substituiert sein kann;
R⁵ ist bei jedem Auftreten gleich oder verschieden H, D, F, Cl, Br, I, CN, NO₂, N(R³)₂, OR³, SR³, COOR³, C(=O)N(R³)₂, Si(R³)₃, B(OR³)₂, C(=O)R³, P(=O)(R³)₂, S(=O)R³, S(=O)₂R³, OSO₂R³, eine geradkettige Alkyl- oder Alkoxygruppe mit 1 bis 20 C-Atomen oder eine Alkenyl- oder Alkinylgruppe mit 2 bis 20 C-Atomen oder eine verzweigte oder cyclische Alkyl- oder Alkoxygruppe mit 3 bis 20 C-Atomen, wobei die Alkyl-, Alkoxy-, Alkenyl- oder Alkinylgruppe jeweils mit einem oder mehreren Resten R³ substituiert sein kann, wobei eine oder mehrere nicht benachbarte CH₂-Gruppen durch Si(R³)₂, C=O, NR³, O, S oder CONR³ ersetzt sein können, oder ein aromatisches oder heteroaromatisches Ringsystem mit 5 bis 30 aromatischen Ringatomen, das jeweils durch einen oder mehrere Reste R³ substituiert sein kann; dabei können zwei Reste R⁵ auch miteinander ein aliphatisches, heteroaliphatisches, aromatisches oder heteroaromatisches Ringsystem bilden.

## Claims

1. Compound of the formula (1) 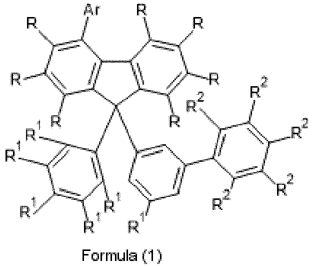 where the symbols used are as follows:
Ar is an aromatic ring system which has 6 to 30 aromatic ring atoms and may be substituted by one or more R³ radicals, or a dibenzofuran or dibenzothiophene group, each of which may be substituted by one or more R³ radicals, or a combination of an aromatic ring system having 6 to 18 aromatic ring atoms and a dibenzofuran or dibenzothiophene group, where these groups may each be substituted by one or more R³ radicals; Ar here may form a ring system together with the adjacent substituent R;
R is the same or different at each instance and is H, D, a straight-chain alkyl or alkoxy group having 1 to 20 carbon atoms or a branched or cyclic alkyl or alkoxy group having 3 to 20 carbon atoms or an alkenyl group having 2 to 20 carbon atoms, where the alkyl, alkoxy or alkenyl group may in each case be substituted by one or more R³ radicals, where one or more hydrogen atoms may be replaced by D, or an aromatic ring system which has 6 to 30 aromatic ring atoms and may be substituted by one or more R³ radicals, or a dibenzofuran or dibenzothiophene group, each of which may be substituted by one or more R³ radicals; it is also possible here for two or more adjacent substituents R together to form a mono- or polycyclic aliphatic ring system; in addition, it is possible for R with an adjacent Ar group to form a ring system;
R¹, R² is the same or different at each instance and is H, D, a straight-chain alkyl or alkoxy group having 1 to 20 carbon atoms or a branched or cyclic alkyl or alkoxy group having 3 to 20 carbon atoms or an alkenyl group having 2 to 20 carbon atoms, where the alkyl, alkoxy or alkenyl group may in each case be substituted by one or more R³ radicals, where one or more hydrogen atoms may be replaced by D, or an aromatic ring system which has 6 to 30 aromatic ring atoms and may be substituted by one or more R³ radicals, or a dibenzofuran or dibenzothiophene group, each of which may be substituted by one or more R³ radicals; it is possible here for multiple adjacent substituents R¹ together to form a ring system; in addition, it is possible for multiple adjacent substituents R² together to form a ring system;
R³ is the same or different at each instance and is H, D, a straight-chain alkyl or alkoxy group having 1 to 20 carbon atoms or a branched or cyclic alkyl or alkoxy group having 3 to 20 carbon atoms, where the alkyl or alkoxy group may in each case be substituted by one or more R⁴ radicals, where one or more hydrogen atoms may be replaced by D, or an aromatic ring system which has 6 to 30 aromatic ring atoms and may be substituted by one or more R⁴ radicals, or a dibenzofuran or dibenzothiophene group, each of which may be substituted by one or more R⁴ radicals; it is also possible here for two or more adjacent substituents R³ together to form a ring system;
R⁴ is the same or different at each instance and is H, D or an aliphatic and/or aromatic hydrocarbyl radical having 1 to 20 carbon atoms.

2. Compound according to Claim 1, selected from the compounds of the formulae (2a), (2b) and (2c) 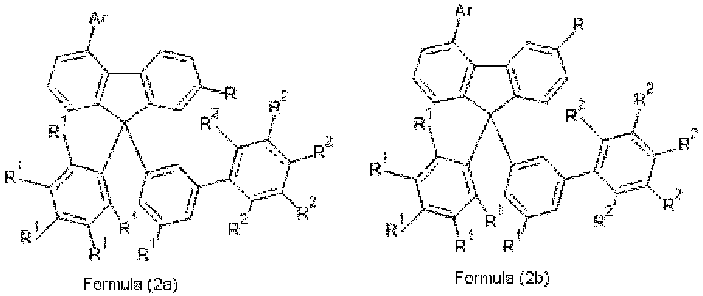 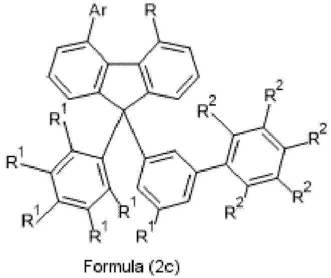 where the symbols used have the definitions given in Claim 1.

3. Compound according to Claim 1 or 2, selected from the compounds of the formulae (3a) and (3b) 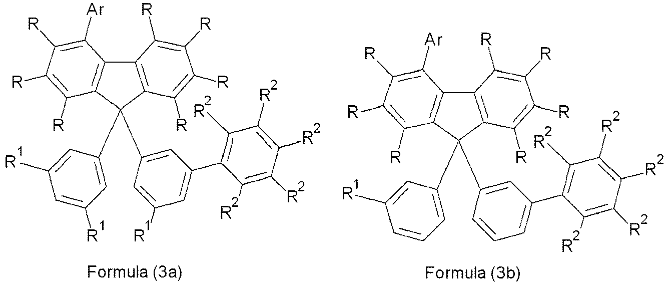 where the symbols used have the definitions given in Claim 1.

4. Compound according to one or more of Claims 1 to 3, selected from the compounds of the formulae (4a) to (4f) 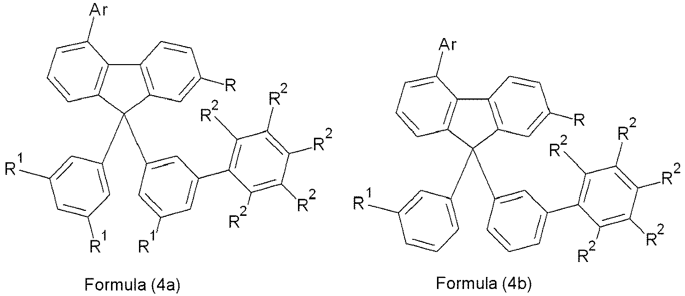 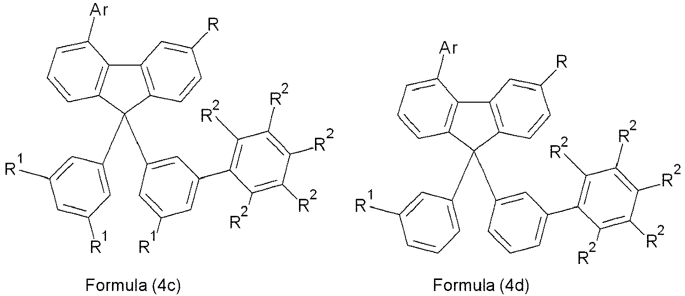 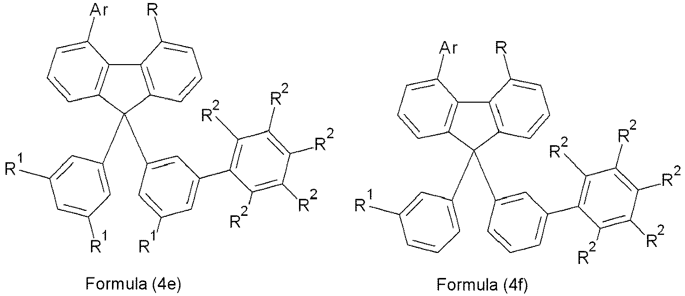 where the symbols used have the definitions given in Claim 1.

5. Compound according to one or more of Claims 1 to 4, selected from the compounds of the formulae (6a) to (6f) 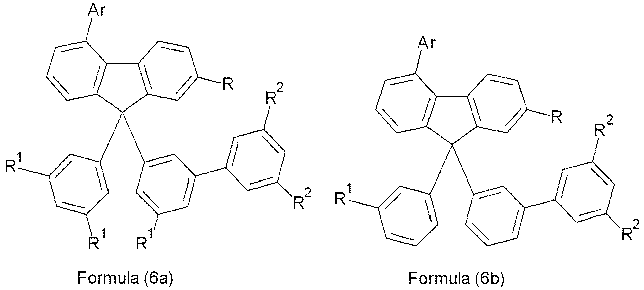 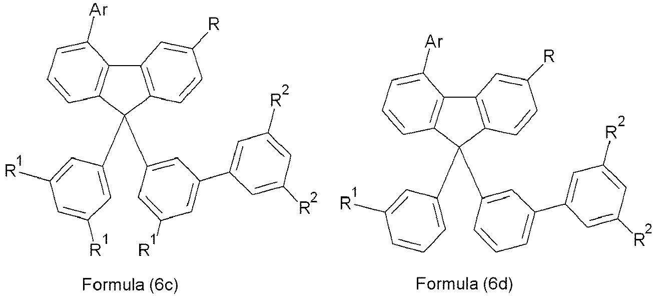 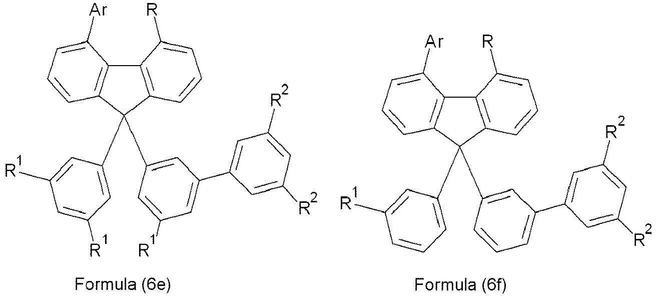 where the symbols used have the definitions given in Claim 1.

6. Compound according to one or more of Claims 1 to 5, **characterized in that** Ar is selected from the group consisting of phenyl, biphenyl, terphenyl, quaterphenyl, fluorene, spirobifluorene, naphthalene, dibenzofuran, dibenzothiophene, phenanthrene, triphenylene or a combination of these groups, each of which may be substituted by one or more R³ radicals.

7. Compound according to one or more of Claims 1 to 6, selected from the compounds of the formulae (2d), (6g) and (6h) 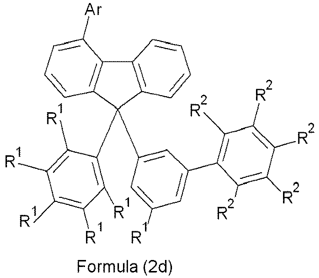 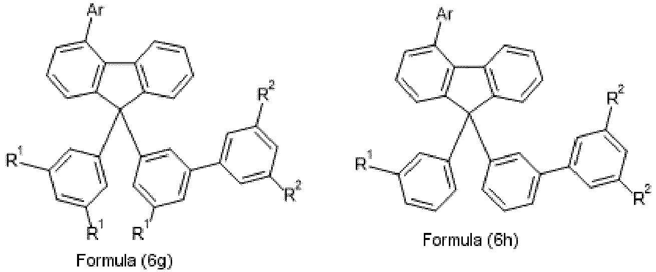 where the symbols used have the definitions given in Claim 1.

8. Compound according to one or more of Claims 1 to 7, selected from the compounds of the formulae (8a), (8b), (9a) and (9b) 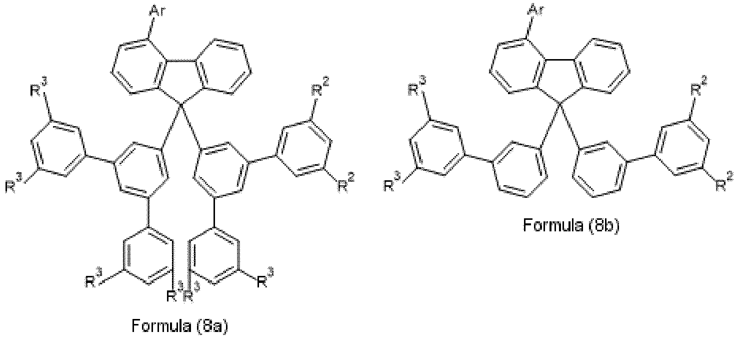 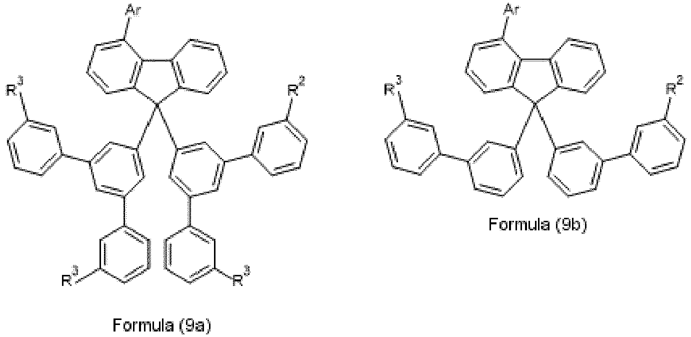 where the symbols used have the definitions given in Claim 1.

9. Composition comprising at least one compound according to one or more of Claims 1 to 8 and at least one further functional material, wherein the further functional material is preferably selected from a phosphorescent emitter, an emitter that exhibits TADF, and/or a matrix material selected from the group consisting of electron transport materials, hole transport materials and bipolar materials.

10. Formulation comprising at least one compound according to one or more of Claims 1 to 8 or a composition according to Claim 9 and at least one organic solvent.

11. Use of a compound according to one or more of Claims 1 to 8 or a composition according to Claim 9 in an electronic device.

12. Electronic device comprising at least one compound according to one or more of Claims 1 to 8 or a composition according to Claim 9.

13. Electronic device according to Claim 12 which is an organic electroluminescent device, **characterized in that** the emitting layer comprises at least one compound according to one or more of Claims 1 to 8.

14. Electronic device according to Claim 13, **characterized in that** the emitting layer contains at least one triazine, quinazoline or pyrimidine derivative of one of the formulae (10), (11) and (12) 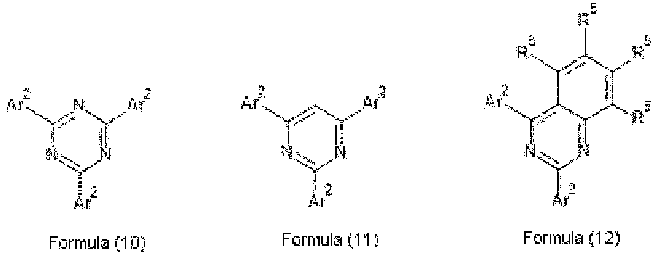 where R³ has the definitions given in Claim 1 and the other symbols used are as follows:
Ar² is the same or different at each instance and is an aromatic or heteroaromatic ring system which has 5 to 40 aromatic ring atoms and may be substituted by one or more R⁵ radicals;
R⁵ is the same or different at each instance and is H, D, F, Cl, Br, I, CN, NO₂, N(R³)₂, OR³, SR³, COOR³, C(=O)N(R³)₂, Si(R³)³, B(OR³)², C(=O)R³, P(=O) (R³)₂, S(=O)R³, S(=O)₂R³, OSO₂R³, a straight-chain alkyl or alkoxy group having 1 to 20 carbon atoms or an alkenyl or alkynyl group having 2 to 20 carbon atoms or a branched or cyclic alkyl or alkoxy group having 3 to 20 carbon atoms, where the alkyl, alkoxy, alkenyl or alkynyl group may in each case be substituted by one or more R³ radicals, where one or more nonadjacent CH₂ groups may be replaced by Si(R³)₂, C=O, NR³, O, S or CONR³, or an aromatic or heteroaromatic ring system which has 5 to 30 aromatic ring atoms and may be substituted in each case by one or more R³ radicals; it is also possible here for two R⁵ radicals together to form an aliphatic, heteroaliphatic, aromatic or heteroaromatic ring system.

## Revendications

1. Composé de formule (1), 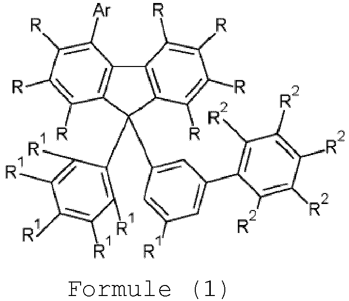 dans laquelle les symboles utilisés ont les significations suivantes :
Ar est un système cyclique aromatique ayant de 6 à 30 atomes formant le cycle aromatique, qui peut être substitué par un ou plusieurs radicaux R³, ou un groupe dibenzofurane ou dibenzothiophène qui peut dans chaque cas être substitué par un ou plusieurs radicaux R³, ou une combinaison d'un système cyclique aromatique ayant de 6 à 18 atomes formant le cycle aromatique et d'un groupe dibenzofurane ou dibenzothiophène, ces groupes pouvant être substitués chacun par un ou plusieurs radicaux R³ ; en outre Ar peut former un système cyclique avec le substituant R voisin ;
R est, le même ou différent à chaque occurrence, H, D, un groupe alkyle ou alcoxy à chaîne droite ayant de 1 à 20 atomes de carbone ou un groupe alkyle ou alcoxy ramifié ou cyclique ayant de 3 à 20 atomes de carbone ou un groupe alcényle ayant de 2 à 20 atomes de carbone, le groupe alkyle, alcoxy ou alcényle pouvant dans chaque cas être substitué par un ou plusieurs radicaux R³, un ou plusieurs atomes d'hydrogène pouvant être remplacés par D, ou un système cyclique aromatique ayant de 6 à 30 atomes formant le cycle aromatique, qui peut être substitué par un ou plusieurs radicaux R³, ou un groupe dibenzofurane ou dibenzothiophène, qui peut dans chaque cas être substitué par un ou plusieurs radicaux R³ ; de plus deux ou plus de deux substituants R voisins peuvent également former l'un avec l'autre/les uns avec les autres un système cyclique mono- ou polycyclique, aliphatique, en outre R peut former un système cyclique avec un groupe Ar voisin ;
R¹, R², identiques ou différents à chaque occurrence, représentent H, D, un groupe alkyle ou alcoxy à chaîne droite ayant de 1 à 20 atomes de carbone ou un groupe alkyle ou alcoxy ramifié ou cyclique ayant de 3 à 20 atomes de carbone ou un groupe alcényle ayant de 2 à 20 atomes de carbone, le groupe alkyle, alcoxy ou alcényle pouvant dans chaque cas être substitué par un ou plusieurs radicaux R³, un ou plusieurs atomes d'hydrogène pouvant être remplacés par D, ou un système cyclique aromatique ayant de 6 à 30 atomes formant le cycle aromatique, qui peut être substitué par un ou plusieurs radicaux R³, ou un groupe dibenzofurane ou dibenzothiophène, qui peut dans chaque cas être substitué par un ou plusieurs radicaux R³ ; de plus plusieurs substituants R¹ voisins peuvent former ensemble un système cyclique ; en outre plusieurs substituants R² voisins peuvent former ensemble un système cyclique ;
R³ est, le même ou différent à chaque occurrence, H, D, un groupe alkyle ou alcoxy à chaîne droite ayant de 1 à 20 atomes de carbone ou un groupe alkyle ou alcoxy ramifié ou cyclique ayant de 3 à 20 atomes de carbone, le groupe alkyle ou alcoxy pouvant dans chaque cas être substitué par un ou plusieurs radicaux R⁴, un ou plusieurs atomes d'hydrogène pouvant être remplacés par D, ou un système cyclique aromatique ayant de 6 à 30 atomes formant le cycle aromatique, qui peut être substitué par un ou plusieurs radicaux R⁴, ou un groupe dibenzofurane ou dibenzothiophène, qui peut dans chaque cas être substitué par un ou plusieurs radicaux R⁴ ; en outre deux ou plus de deux substituants R³ voisins peuvent également former l'un avec l'autre/les uns avec les autres un système cyclique ;
R⁴ est, le même ou différent à chaque occurrence, H, D ou un radical hydrocarboné aliphatique et/ou aromatique ayant de 1 à 20 atomes de carbone.

2. Composé selon la revendication 1, choisi parmi les composés de formules (2a), (2b) et (2c), 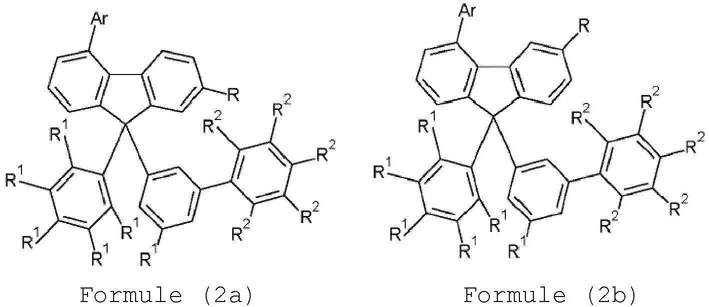 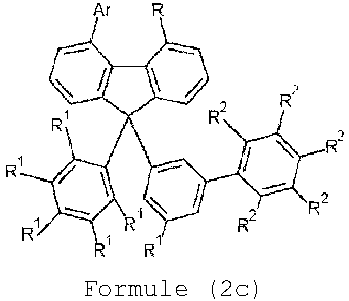 dans lesquelles les symboles utilisés ont les significations indiquées dans la revendication 1.

3. Composé selon la revendication 1 ou 2, choisi parmi les composés de formules (3a) et (3b), 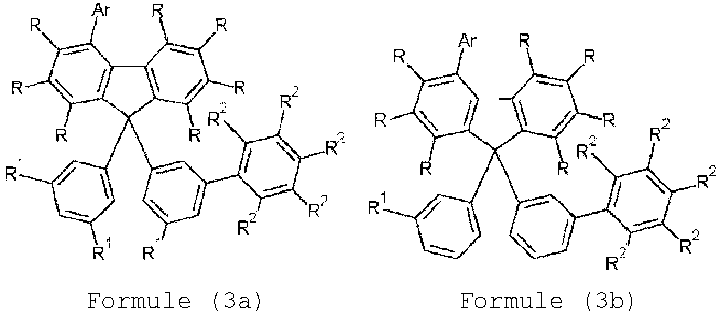 dans lesquelles les symboles utilisés ont les significations indiquées dans la revendication 1.

4. Composé selon une ou plusieurs des revendications 1 à 3, choisi parmi les composés de formules (4a) à (4f), 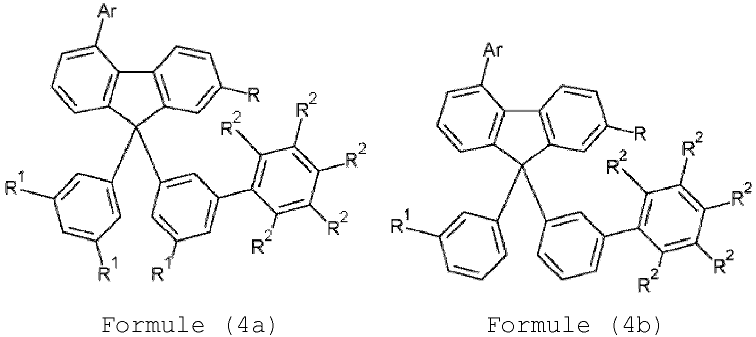 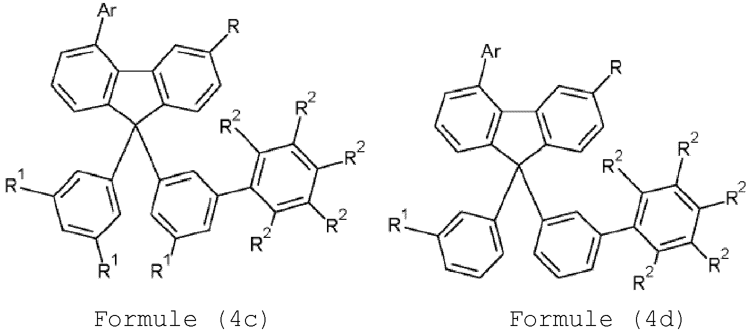 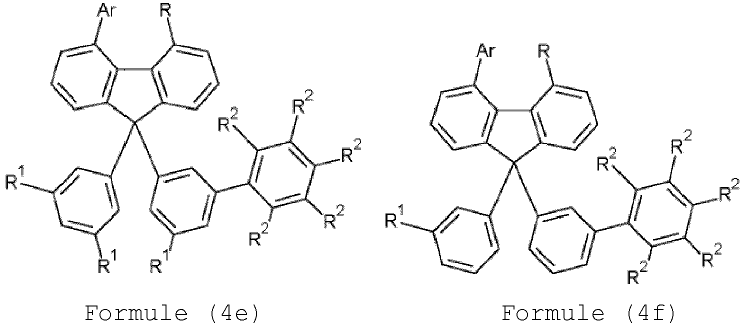 dans lesquelles les symboles utilisés ont les significations indiquées dans la revendication 1.

5. Composé selon une ou plusieurs des revendications 1 à 4, choisi parmi les composés de formules (6a) à (6f), 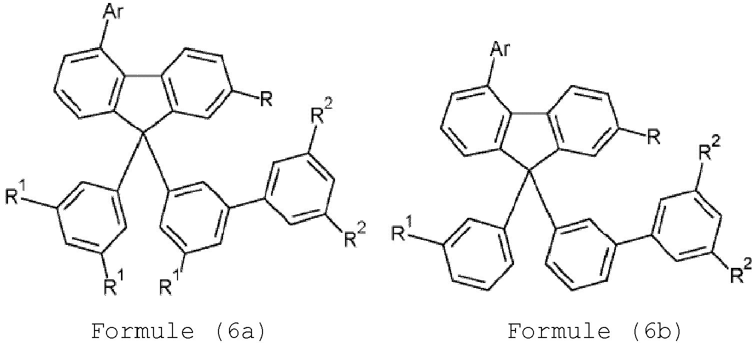 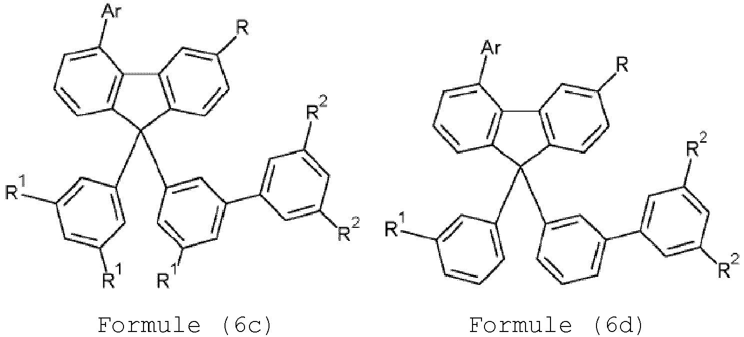 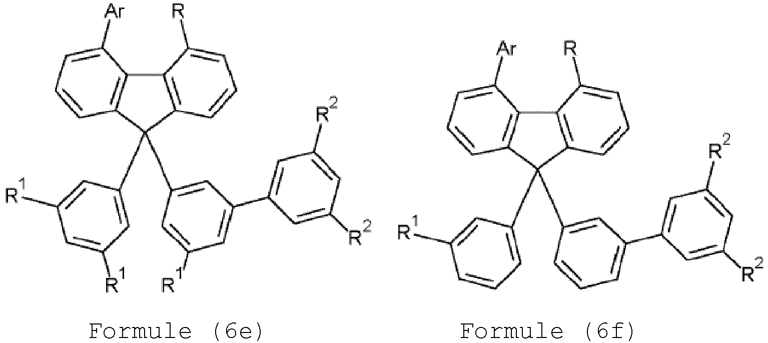 dans lesquelles les symboles utilisés ont les significations indiquées dans la revendication 1.

6. Composé selon une ou plusieurs des revendication 1 à 5, **caractérisé en ce que** Ar est choisi dans le groupe constitué par les groupes phényle, biphényle, terphényle, quaterphényle, fluorène, spirobifluorène, naphtalène, dibenzofurane, dibenzothiophène, phénanthrène, triphénylène ou une combinaison de ces groupes, qui peuvent être substitués chacun par un ou plusieurs radicaux R³.

7. Composé selon une ou plusieurs des revendications 1 à 6, choisi parmi les composés de formules (2d), (6g) ou (6h), 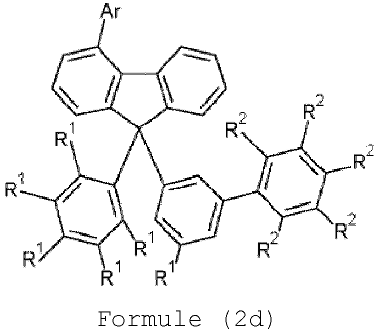 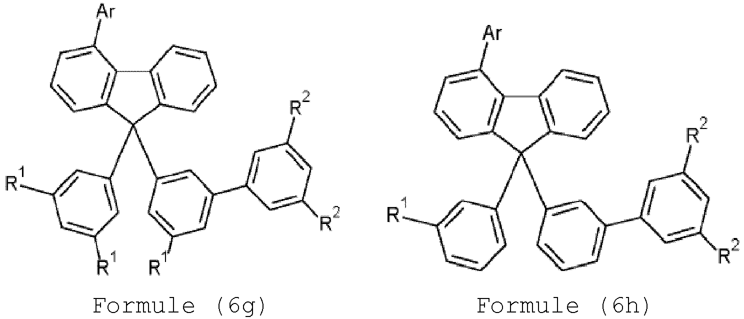 dans lesquelles les symboles utilisés ont les significations indiquées dans la revendication 1.

8. Composé selon une ou plusieurs des revendications 1 à 7, choisi parmi les composés de formules (8a), (8b), (9a) et (9b), 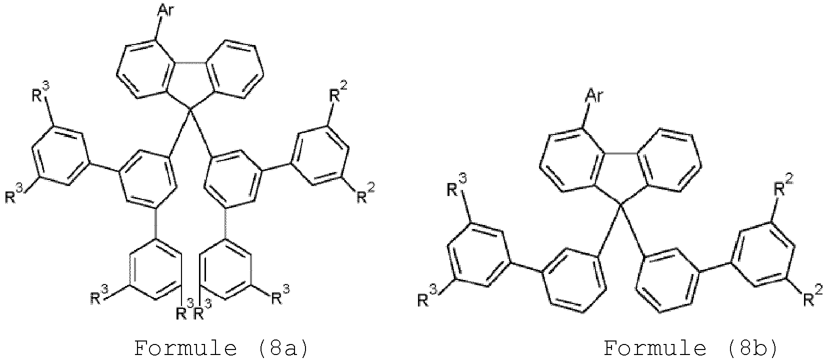 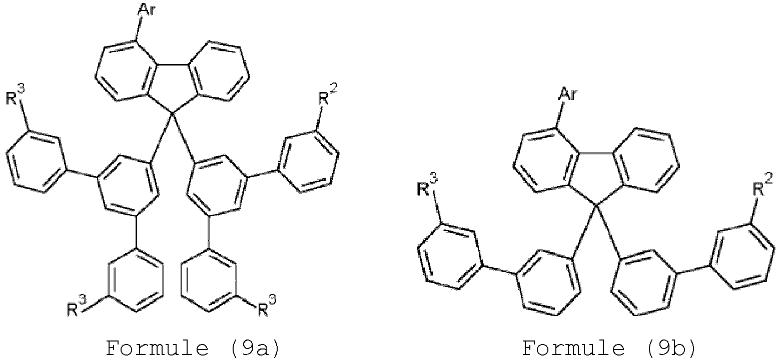 dans lesquelles les symboles utilisés ont les significations indiquées dans la revendication 1.

9. Composition contenant au moins un composé selon une ou plusieurs des revendications 1 à 8 et au moins un autre matériau fonctionnel, l'autre matériau fonctionnel étant choisi de préférence parmi un émetteur phosphorescent, un émetteur qui présente une TADF, et/ou un matériau matrice qui est choisi dans le groupe constitué par les matériaux de transport d'électrons, les matériaux de transport de trous et les matériaux bipolaires.

10. Formulation contenant au moins un composé selon une ou plusieurs des revendications 1 à 8 ou une composition selon la revendication 9 et au moins un solvant organique.

11. Utilisation d'un composé selon une ou plusieurs des revendications 1 à 8 ou d'une composition selon la revendication 9 dans un dispositif électronique.

12. Dispositif électronique, contenant au moins un composé selon une ou plusieurs des revendications 1 à 8 ou une composition selon la revendication 9.

13. Dispositif électronique selon la revendication 12, qui consiste en un dispositif électroluminescent organique, **caractérisé en ce que** la couche émettrice contient au moins un composé selon une ou plusieurs des revendications 1 à 8.

14. Dispositif électronique selon la revendication 13, **caractérisé en ce que** la couche émettrice contient au moins un dérivé de triazine, quinazoline ou pyrimidine conforme à l'une des formules (10), (11) et (12), 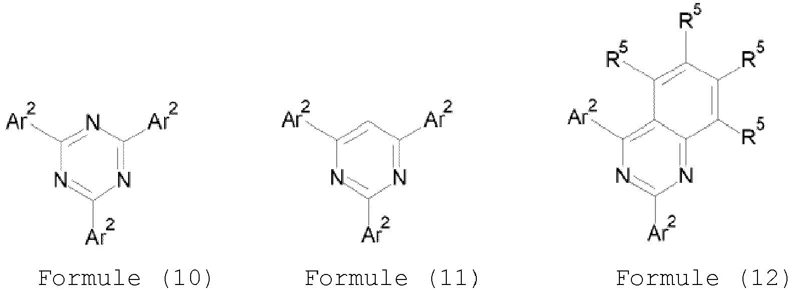 dans lesquelles R³ a les significations indiquées dans la revendication 1 et les autres symboles utilisés ont les significations suivantes :
Ar² est, le même ou différent à chaque occurrence, un système cyclique aromatique ou hétéroaromatique ayant de 5 à 40 atomes de carbone formant le cycle aromatique, qui peut être substitué par un ou plusieurs radicaux R^{5 ;}
R⁵ est, le même ou différent à chaque occurrence, H, D, F, Cl, Br, I, CN, NO₂, N(R³)₂, OR³, SR³, COOR³, C(=O)N(R³)₂, Si(R³)₃, B(OR³)₂, C(=O)R³, P(=O)(R³)₂, S(=O)R³, S(=O)₂R³, OSO₂R³, un groupe alkyle ou alcoxy à chaîne droite ayant de 1 à 20 atomes de carbone ou un groupe alcényle ou alcynyle ayant de 2 à 20 atomes de carbone ou un groupe alkyle ou alcoxy ramifié ou cyclique ayant de 3 à 20 atomes de carbone, le groupe alkyle, alcoxy, alcényle ou alcynyle pouvant dans chaque cas être substitué par un ou plusieurs radicaux R³, un ou plusieurs groupes CH₂ non contigus pouvant être remplacés par Si(R³)₂, C=O, NR³, O, S ou CONR³, ou un système cyclique aromatique ou hétéroaromatique ayant de 5 à 30 atomes formant le cycle aromatique, qui peut dans chaque cas être substitué par un ou plusieurs radicaux R³ ; en outre deux radicaux R⁵ peuvent également former l'un avec l'autre un système cyclique aliphatique, hétéroaliphatique, aromatique ou hétéroaromatique.
